# EUROPÄISCHE PATENTANMELDUNG

(11) **EP 2 264 010 A1**
(43) Veröffentlichungstag der Anmeldung: **22.12.2010**
(21) Anmeldenummer: 09161835.5
(22) Anmeldetag: 03.06.2009
(51) Int. Cl.: C07D 213/74, A01N 43/40

(54) **Hetarylamidine**

(71) Anmelder: Bayer CropScience AG, 40789 Monheim (DE)
(72) Erfinder: Die Erfindernennung liegt noch nicht vor

(57) **Zusammenfassung**

Beschrieben werden spezielle Hetarylamidine, ein Verfahren zu deren Herstellung, die Verwendung der erfindungsgemäßen Hetarylamidine zum Bekämpfen von unerwünschten Mikroorganismen, sowie ein Mittel zu diesem Zweck, umfassend die erfindungsgemäßen Hetarylamidine. Weiterhin betrifft die Erfindung ein Verfahren zum Bekämpfen unerwünschter Mikroorganismen durch Ausbringen der crfindungsgcmäßen Verbindungen auf die Mikroorganismen und/oder in deren Lebensraum.

## Beschreibung

Die vorliegende Erfindung betrifft spezielle Hetarylamidine, ein Verfahren zu deren Herstellung, die Verwendung der erfindungsgemäßen Hetarylamidine zum Bekämpfen von unerwünschten Mikroorganismen, sowie ein Mittel zu diesem Zweck, umfassend die erfindungsgemäßen Hetarylamidine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

WO00/46184 A1 betrifft Amidinylphenylverbindungen und deren Verwendung als Fungizide.

WO 03/093224 A1 betrifft Amidinylphenylverbindungen und deren Verwendung als Fungizide.

WO 2008/101682 A2 betrifft Mikrobiozide, insbesondere Fungizide, auf Basis von Pyridinamidinen, die in ortho-Position zu dem Stickstoffatom des Pyridinringes einem über ein Sauerstoffatom gebundenen Substituenten aufweisen.

Die gemäß den oben genannten Schriften bereits bekannten Wirkstoffe weisen bei ihrer Anwendung Nachteile auf, insbesondere dass sie keine oder aber eine nur unzureichende fungizide Wirkung besitzen oder dass sie nur ein geringes Spektrum an Pilzen bekämpfen.

Daher besteht Bedarf nach weiteren fungiziden Wirkstoffen.

Gelöst wird diese Aufgabe durch spezielle Hetarylamidine.

Die erfindungsgemäßen Hetarylamidine sind durch die folgende allgemeine Struktur (I) gekennzeichnet 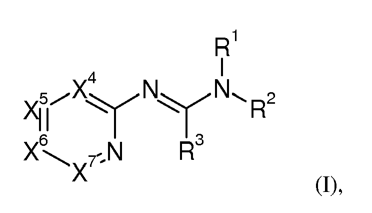 in welcher
- R¹ und R²,: unabhängig voneinander, für gegebenenfalls substituierte Reste ausgewählt aus Alkyl, Alkenyl, Alkinyl, Carbocyclyl oder Heterocyclyl; Wasserstoff; Cyano; Acyl; -OR^{a} oder -SR^{a} stehen, wobei R^{a} für gegebenenfalls substituierte Reste, ausgewählt aus Alkyl, Alkenyl, Alkinyl, Carbocyclyl oder Heterocyclyl steht; oder
- R¹undR²,: zusammen mit den Atomen, an welche sie gebunden sind, einen fünf- bis siebengliedrigen Ring bilden, welcher substituiert sein kann und welcher gegebenenfalls 1 oder 2 Sauerstoffatome aufweist;
- R³: für gegebenenfalls substituierte Reste, ausgewählt aus Alkyl, Alkenyl, Alkinyl, Alkoxycarbonyl, Carbocyclyl oder Heterocyclyl; OH; SH; SO₃H; Cyano; Wasserstoff; -OR^{b} oder -SR^{b} steht, wobei R^{b} für gegebenenfalls substituierte Reste, ausgewählt aus Alkyl, Alkenyl, Alkinyl, Carbocyclyl oder Heterocyclyl steht; oder
- R² und R³,: zusammen mit den Atomen, an welche sie gebunden sind, einen fünf- bis siebengliedrigen Ring bilden, welcher substituiert sein kann und welcher gegebenenfalls 1 oder 2 Sauerstoffatome aufweist;
- X⁴: für C-R⁴ oder N steht;
- X⁵: für C-R⁵ oder N steht;
- X⁶: für C-R⁶ oder N steht;
- X⁷: für C-R⁷ oder N steht;
- R⁴, R⁵, R⁶ und R⁷,: jeweils unabhängig voneinander für Wasserstoff; Halogen; Hydroxy; gegebenenfalls substituierte Reste, ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b} stehen, wobei R^{b} für gegebenenfalls substituierte Reste, ausgewählt aus C₁-C₁₂- Alkyl, C₃-C₁₀-Carbocyclyl oder C₃-C₁₀-Heterocyclyl steht und n für eine ganzzahlige Zahl von 0 bis 2 steht; mit der Maßgabe, dass von den Resten X⁴, X⁵, X⁶ und X⁷ 0 bis 2 Reste gleich Stickstoff sind; sowie die N-Oxide und Salze der Verbindungen der allgemeinen Formel (I).

Erfindungsgemäß wurde gefunden, dass die Verbindungen der allgemeinen Formel (I) sowie deren N-Oxide und Salze fungizide Eigenschaften besitzen und sich im Pflanzenschutz zur Bekämpfung unerwünschter Schädlinge verwenden lassen.

### Allgemeine Definitionen

Für die vorstehende und nachfolgende Beschreibung der erfindungsgemäßen Verbindungen der allgemeinen Formel (I) gelten - soweit nichts anderes angegeben - die nachfolgenden Definitionen:
Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.
Gegebenenfalls substituierte Gruppen können einfach oder mehrfach substituiert sein, wobei bei Mehrfachsubstitutionen die Substituenten gleich oder verschieden sein können.
Alkyl-Gruppen sind im Zusammenhang mit der vorliegenden Erfindung, soweit nicht abweichend definiert, lineare, verzweigte oder ringförmige Kohlenwasserstoff-Gruppen mit im Allgemeinen 1 bis 12 Kohlenstoffatomen, vorzugsweise 1 bis 8 Kohlenstoffatomen, besonders bevorzugt 1 bis 6 Kohlenstoffatomen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR'₂), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁₋₁₂-Alkyl umfasst den größten hierin definierten Bereich für eine AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR'₂), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂₋₁₂-Alkenyl umfasst den größten hierin definierten Bereich für eine AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S, aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR'₂), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂₋₁₂-Alkinyl umfasst den größten hierin definierten Bereich für eine Alkinyl-Gruppe. Im Einzelnen umfasst diese Definition beispielsweise die Bedeutungen Ethinyl (Acetylenyl); Prop-1-inyl und Prop-2-inyl.

Unter Carbocyclylgruppen werden im Rahmen der vorliegenden Erfindung Cycloalkylgruppen und Arylgruppen verstanden.

Unter Cycloalkylgruppen wird im Rahmen der vorliegenden Erfindung C₃₋₈-Cycloalkyl verstanden, wobei C₃₋₈-Cycloalkyl monocyclische, gesättigte Kohlenwasserstoffgruppen mit 3 bis 8 Kohlenstoffringgliedern, wie Cyclopropyl, Cyclobutyl, Cyclopentyl, Cyclohexyl, Cycloheptyl und Cyclooctyl, umfasst.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy-(-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR'₂), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Unter Heterocyclylgruppen werden im Rahmen der vorliegenden Erfindung Carbocyclylgruppen und Arylgruppen verstanden, welche mindestens ein Heteroatom umfassen.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR'₂), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Im Folgenden werden bevorzugte Ausführungsformen der Verbindungen der allgemeinen Formel (I) beschrieben. Die vorstehenden allgemeinen und die im Folgenden dargestellten speziellen Definitionen für die Reste gelten sowohl für die Verbindungen der allgemeinen Formel (I) als auch für etwaige Zwischenverbindungen, welche bei der Synthese der Verbindungen der allgemeinen Formel (I) durchlaufen werden.

In einer **ersten Ausgestaltung** der vorliegenden Erfindung sind Verbindungen der allgemeinen Formel (I) bevorzugt, in welchen die Reste R¹ und R², jeweils unabhängig voneinander, für gegebenenfalls substituierte Reste, ausgewählt aus Alkyl, Alkenyl und Carbocyclyl; und Wasserstoff stehen oder R¹ und R², zusammen mit den Atomen, an welche sie gebunden sind, einen fünf- bis siebengliedrigen Ring bilden, welcher substituiert sein kann und welcher gegebenenfalls 1 oder 2 Sauerstoffatome aufweist.

In dieser ersten Ausgestaltung der vorliegenden Erfindung sind Verbindungen der allgemeinen Formel (I) besonders bevorzugt, in welchen die Reste R¹ und R², jeweils unabhängig voneinander, für einen gegebenenfalls substituierten Alkylrest stehen oder R¹ und R², zusammen mit den Atomen, an welche sie gebunden sind, einen fünf- bis siebengliedrigen Ring bilden, welcher substituiert sein kann und welcher gegebenenfalls 1 Sauerstoffatom aufweist

In einer **zweiten Ausgestaltung** der vorliegenden Erfindung sind Verbindungen der allgemeinen Formel (I) bevorzugt, in welchen der Rest R³ für gegebenenfalls substituierte Reste ausgewählt aus Alkyl und Alkenyl; oder Wasserstoff steht, oder R² und R³, zusammen mit den Atomen, an welche sie gebunden sind, einen fünf- bis siebengliedrigen Ring bilden, welcher substituiert sein kann.

Die vorstehend definierten bevorzugten und besonders bevorzugten Ausgestaltungen der Reste R¹ bis R³ können erfindungsgemäß beliebig mit den nachfolgenden unterschiedlichen Ausführungsformen für die Reste R⁴ bis R⁷ sowie X⁴ bis X⁷ kombiniert werden.

In einer **ersten Ausführungsform** der vorliegenden Erfindung sind von der vorliegenden Erfindung Hetarylamidine der allgemeinen Formel (I-1) umfasst, in welchen X⁴ gleich Stickstoff, X⁵ gleich C-R⁵, X⁶ gleich C-R⁶ und X⁷ gleich C-R⁷ sind (Pyrimidine).

In dieser ersten Ausführungsform stehen die Reste R⁵, R⁶ und R⁷, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocylyl oder C₃-C₁₀-Heterocyclyl steht und n für eine ganzzahlige Zahl von 0 bis 2 steht.

Des Weiteren ist in dieser ersten Ausführungsform bevorzugt, wenn R⁵ gleich gegebenenfalls substituiertes Alkyl, gegebenenfalls substituiertes Alkoxyalkyl; Carbocyclyl; Heterocyclyl; - OR^{b} oder -S(O)ₙR^{b} ist, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Speziell ist in dieser ersten Ausführungsform bevorzugt, wenn R⁵ gleich gegebenenfalls substituiertes Alkyl; gegebenenfalls substituiertes Alkoxyalkyl; gegebenenfalls substituiertes Phenyl; -OR^{b}' oder -S(O)ₙR^{b}' ist, wobei R^{b}' für gegebenenfalls substituierte Reste ausgewählt aus Phenyl, Pyridin-2-yl, Pyridin-3-yl oder Pyridin-4-yl steht und n für 0 oder 2 steht. Insbesondere ist in dieser ersten Ausführungsform bevorzugt, wenn R⁵ gleich Phenoxy, 4-Fluor-3-(trifluormethyl)phenoxy, 3-(Trifluormethyl)phenoxy, 3-(Trifluormethyl)phenoxy, 4-Chlor-3-(trifluormethyl)phenoxy, 2-Fluor-5-(trifluormethyl)phenoxy, 2-Fluor-3-(trifluormethyl)phenoxy, 2-Fluor-3-(trifluormethyl)phenoxy, 4-Methoxyphenoxy, 3,5-Bis(trifluormethyl)phenoxy, 3-Fluor-5-(trifluormethyl)phenoxy, , 3,5-Dichlorphenoxy, 3,5-Difluorphenoxy, 3-Chlor-4-fluorphenoxy, 3,4-Difluorphenoxy, 3-Chlor-4-(trifluormethyl)phenoxy, 4-Chlor-3-(trifluormethyl)phenoxy, Pyridin-2-ylsulfanyl, (4-Methylphenyl)sulfonyl, Diethoxymethyl, 3,4-Dichlorphenyl, 3-(Trifluormethyl)phenyl, Pyridin-3-yl, Pyridin-4-yl oder CH₃ ist.

Des Weiteren ist in dieser ersten Ausführungsform bevorzugt, wenn R⁶ gleich Wasserstoff; Halogen; Hydroxy; und gegebenenfalls substituiertes Alkyl ist.

Insbesondere ist in dieser ersten Ausführungsform bevorzugt, wenn R⁶ gleich Wasserstoff; Methyl oder Ethyl ist.

Des Weiteren ist in dieser ersten Ausführungsform bevorzugt, wenn R⁷ gleich Wasserstoff; Halogen; Hydroxy; einem gegebenenfalls substituierten Rest ausgewählt aus Alkyl und Alkoxy; oder -OR^{b} ist, wobei R^{b} für einen gegebenenfalls substituierten Rest ausgewählt aus C₁-C₁₂-Alkyl, C₃-C₁₀-Carbocyclyl oder C₃-C₁₀-Heterocyclyl steht.

Speziell ist in dieser ersten Ausführungsform bevorzugt R⁷ gleich einem gegebenenfalls substituierten Rest ausgewählt aus Alkyl und Alkoxy; oder -OR^{b} ist, wobei R^{b} für einen gegebenenfalls substituierten Rest ausgewählt aus C₃-C₁₀-Carbocyclyl steht.

Insbesondere ist in dieser ersten Ausführungsform bevorzugt, wenn R⁷ gleich Methyl; Propan-2-yl; Methoxy; Ethoxy oder 4-Fluor-3-(trifluormethyl)phenoxy ist,

In einer **zweiten Ausführungsform** der vorliegenden Erfindung sind von der vorliegenden Erfindung Hetarylamidine der allgemeinen Formel (1-2) umfasst, in welchen X⁵ gleich Stickstoff, X⁴ gleich C-R⁴, X⁶ gleich C-R⁶ und X⁷ gleich C-R⁷ sind (Pyrazine).

In dieser zweiten Ausführungsform stehen die Reste R⁴, R⁶ und R⁷, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Des Weiteren ist in dieser zweiten Ausführungsform bevorzugt, wenn R⁴ gleich Wasserstoff oder gegebenenfalls substituiertes Alkyl ist.

Insbesondere ist in dieser zweiten Ausführungsform bevorzugt, wenn R⁴ gleich Wasserstoff ist.

Des Weiteren ist in dieser zweiten Ausführungsform bevorzugt, wenn R⁶ gleich Wasserstoff oder ein gegebenenfalls substituierter Rest, ausgewählt aus Alkyl, Carbocyclyl und Heterocyclyl, ist

Insbesondere ist in dieser zweiten Ausführungsform bevorzugt, wenn R⁶ gleich Wasserstoff, 4-Fluor-3-(trifluormethyl)phenyl, 4-Chlor-3-(trifluormethyl)phenyl, Phenyl, 3-(Trifluormethyl)phenyl, 3-Chlor-4-(trifluormethyl)phenyl oder 3-(Trifluormethoxy)phenyl ist.

Des Weiteren ist in dieser zweiten Ausführungsform bevorzugt, wenn R⁷ gleich Wasserstoff; ein gegebenenfalls substituierter Rest, ausgewählt aus Alkyl, Carbocyclyl und Heterocyclyl, oder -OR^{b} ist, wobei R^{b} für einen gegebenenfalls substituierten Rest, ausgewählt aus C₁-C₁₂-Alkyl, C₃-C₁₀-Carbocyclyl oder C₃-C₁₀-Heterocyclyl steht.

Insbesondere ist in dieser zweiten Ausführungsform bevorzugt, wenn R⁷ gleich Wasserstoff oder 4-Chlor-3-(trifluormethyl)phenoxy ist.

In einer **dritten Ausführungsform** der vorliegenden Erfindung sind von der vorliegenden Erfindung Hetarylamidine der allgemeinen Formel (1-3) umfasst, in welchen X⁶ gleich Stickstoff, X⁴ gleich C-R⁴, X⁵ gleich C-R⁵ und X⁷ gleich C-R⁷ sind (Pyrimidine).

In dieser dritten Ausführungsform stehen die Reste R⁴, R⁵ und R⁷, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Des Weiteren ist in dieser dritten Ausführungsform bevorzugt, wenn R⁴ gleich Wasserstoff, gegebenenfalls substituiertes Alkyl oder Cyano ist.

Insbesondere ist in dieser dritten Ausführungsform bevorzugt, wenn R⁴ gleich Wasserstoff, Methyl oder Cyano ist.

Des Weiteren ist in dieser dritten Ausführungsform bevorzugt, wenn R⁵ gleich Wasserstoff; gegebenenfalls substituiertes Alkyl oder -OR^{b} ist, wobei R^{b} für gegebenenfalls substituiertes C₁-C₁₂-Alkyl, C₃-C₁₀-Carbocyclyl oder C₃-C₁₀-Heterocyclyl ist.

Insbesondere ist in dieser dritten Ausführungsform bevorzugt, wenn R⁵ gleich Wasserstoff; Methyl oder 4-Chlor-3-(trifluormethyl)phenoxy ist.

Des Weiteren ist in dieser dritten Ausführungsform bevorzugt, wenn R⁷ gleich Wasserstoff; Halogen; Hydroxy; ein gegebenenfalls substituierter Rest, ausgewählt aus Alkyl, Carbocyclyl und Heterocyclyl; -OR^{b} oder -S(O)ₙR^{b} ist, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Speziell ist in dieser dritten Ausführungsform bevorzugt R⁷ gleich Halogen; ein gegebenenfalls substituierter Rest, ausgewählt aus Carbocyclyl und Heterocyclyl; -OR^{b} oder -S(O)ₙR^{b} ist, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für 0 steht.

Insbesondere ist in dieser dritten Ausführungsform bevorzugt, wenn R⁷ gleich Chlor, 3-(Trifluormethyl)phenoxy, Methylsulfanyl, 4-Fluor-3-(trifluormethyl)phenoxy, 4-Chlor-3-(trifluormethyl)phenoxy, [1-Methyl-3-(trifluormethyl)-1H-pyrazol-5-yl]oxy, 3-(Trifluormethyl)phenyl, Pyridin-3-yl oder Pyridin-4-yl ist.

In einer **vierten Ausführungsform** der vorliegenden Erfindung sind von der vorliegenden Erfindung Hetarylamidine der allgemeinen Formel (1-4) umfasst, in welchen X⁷ gleich Stickstoff, X⁴ gleich C-R⁴, X⁵ gleich C-R⁵ und X⁶ gleich C-R⁶ sind (Pyridazine).

In dieser vierten Ausführungsform stehen die Reste R⁴, R⁵ und R⁶, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Des Weiteren ist in dieser vierten Ausführungsform bevorzugt, wenn R⁴ gleich Wasserstoff oder gegebenenfalls substituiertes Alkyl ist.

Insbesondere ist in dieser vierten Ausführungsform bevorzugt, wenn R⁴ gleich Wasserstoff oder Methyl ist.

Des Weiteren ist in dieser vierten Ausführungsform bevorzugt, wenn R⁵ gleich Wasserstoff ist.

Des Weiteren ist in dieser vierten Ausführungsform bevorzugt, wenn R⁶ gleich Wasserstoff; Halogen; ein gegebenenfalls substituierter Rest, ausgewählt aus Alkyl, Carbocyclyl und Heterocyclyl; -OR^{b} oder -S(O)ₙR^{b} ist, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für 0 steht.

Insbesondere ist in dieser vierten Ausführungsform bevorzugt, wenn R⁶ gleich Brom, Phenyl, 4-Chlorphenyl, 4-Fluorphenyl, 4-Methoxyphenyl, Phenylsulfanyl, Thiophen-3-yl oder Phenoxy ist.

In einer **fünften Ausführungsform** der vorliegenden Erfindung sind von der vorliegenden Erfindung Hetarylamidine der allgemeinen Formel (1-5) umfasst, in welchen X⁴ und X⁶ gleich Stickstoff, X⁵ gleich C-R⁵ und X⁷ gleich C-R⁷ sind (Triazine).

In dieser fünften Ausführungsform stehen die Reste R⁵ und R⁷, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Des Weiteren ist in dieser fünften Ausführungsform bevorzugt, wenn R⁵ gleich gegebenenfalls substituiertes Alkyl, Alkoxy oder Carbocyclyl ist

Insbesondere ist in dieser fünften Ausführungsform bevorzugt, wenn R⁵ gleich Methyl, Methoxy oder Phenyl ist.

Des Weiteren ist in dieser fünften Ausführungsform bevorzugt, wenn R⁷ gleich gegebenenfalls substituiertes Alkyl ist.

Insbesondere ist in dieser ersten Ausführungsform bevorzugt, wenn R⁵ gleich Methyl ist.

In einer **sechsten Ausführungsform** der vorliegenden Erfindung sind von der vorliegenden Erfindung Hetarylamidine der allgemeinen Formel (1-6) umfasst, in welchen X⁴ gleich C-R⁴, X⁵ gleich C-R⁵, X⁶ gleich C-R⁶ und X⁷ gleich C-R⁷ sind (Pyridine).

In dieser sechsten Ausführungsform stehen die Reste R⁴, R⁵, R⁶ und R⁷, jeweils unabhängig voneinander, für Wasserstoff; Halogen; Hydroxy; gegebenenfalls substituierte Reste ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b}, wobei R^{b} für gegebenenfalls substituierte Reste ausgewählt aus C₁-C₁₂-Alkyl, C₃-C₁₀-Carbocyclyl oder C₃-C₁₀-Heterocyclyl steht und n für eine ganzzahlige Zahl von 0 bis 2 steht.

Des Weiteren ist in dieser sechsten Ausführungsform bevorzugt, wenn R⁴ gleich Wasserstoff; einem gegebenenfalls substituierten Rest, ausgewählt aus Alkyl, Carbocyclyl und Heterocyclyl; oder -OR^{b} ist, wobei R^{b} für einen gegebenenfalls substituierten Rest, ausgewählt aus C₁-C₁₂-Alkyl, C₃-C₁₀-Carbocyclyl oder C₃-C₁₀-Heterocyclyl, steht.

Insbesondere ist in dieser sechsten Ausführungsform bevorzugt, wenn R⁴ gleich Wasserstoff, Methyl, Phenyl, 4-Chlor-3-(trifluormethyl)phenyl, 4-Fluor-3-(trifluormethyl)phenyl, 3-Chlor-4-(trifluormethyl)phenyl, [3-(Trifluormethyl)benzyl]oxy, 3-[(Propan-2-yloxy)carbonyl]phenyl, 3-(Trifluormethyl)phenyl, 5-Fluor-2-methoxyphenyl, (3,4-Dichlorbenzyl)oxy, (3,5-Difluorbenzyl)oxy, 6-Methoxypyridin-3-yl, (4-Fluorbenzyl)oxy, 4-tert-Butylphenyl oder 3,5-Dichlorphenyl ist.

Des Weiteren ist in dieser sechsten Ausführungsform bevorzugt, wenn R⁵ gleich Wasserstoff oder gegebenenfalls substituiertes Alkyl ist.

Insbesondere ist in dieser sechsten Ausführungsform bevorzugt, wenn R⁵ gleich Wasserstoff oder Methyl ist.

Des Weiteren ist in dieser sechsten Ausführungsform bevorzugt, wenn R⁶ gleich Wasserstoff; Halogen; einem gegebenenfalls substituierten Rest, ausgewählt aus Alkyl, Alkenyl, Alkinyl, Carbocyclyl und Heterocyclyl; Nitro oder Cyano ist.

Insbesondere ist in dieser sechsten Ausführungsform bevorzugt, wenn R⁶ gleich Wasserstoff; Methyl; Halogen; Cyano; Nitro; [4-Chlor-3-(trifluormethyl)phenyl]ethinyl; [3-(Trifluormethyl)phenyl]ethinyl, (E)-2-[3-(Trifluormethyl)phenyl]ethenyl, 6-(2,2,2-Trifluorethoxy)pyridin-3-yl, 6-tert-Butoxypyridin-3-yl, 6-(Propan-2-yloxy)pyridin-3-yl, 6-Chlorpyridin-3-yl, 6-Brompyridin-3-yl 4-Chlor-3-(trifluormethyl)phenyl, 4-Fluor-3-(trifluormethyl)phenyl, 3-Chlor-5-(trifluormethyl)phenyl, 3-Chlor-4-(trifluormethyl)phenyl, 3,5-Bis(trifluormethyl)phenyl, 3-(Trifluormethyl)phenyl, 4-(Trifluormethyl)phenyl, 6-Chlorpyridin-3-yl, 4-tert-Butylphenyl, 3,4-Dichlorphenyl, 2,4-Dichlorphenyl, 4-Phenoxyphenyl, 2-Chlorphenyl, 3-Chlorphenyl, 4-Chlorphenyl oder 3-Methylphenyl ist.

Des Weiteren ist in dieser sechsten Ausführungsform bevorzugt, wenn R⁷ gleich Wasserstoff; Halogen; einem gegebenenfalls substituierten Rest, ausgewählt aus Alkyl, Alkenyl, Alkinyl, Carbocyclyl und Heterocyclyl; oder -OR^{b} ist, wobei R^{b} für einen gegebenenfalls substituierten Rest, ausgewählt aus C₁-C₁₂-Alkyl, C₃-C₁₀-Carbocyclyl oder C₃-C₁₀-Heterocyclyl, steht.

Insbesondere ist in dieser sechsten Ausführungsform bevorzugt, wenn R7 gleich [1-Methyl-3-(trifluormethyl)-1H-pyrazol-5-yl]oxy, [2-(Trifluormethyl)pyridin-4-yl]oxy, 4-Fluor-3-(trifluormethyl)phenoxy, 3-(Trifluormethyl)phenoxy, 3-(Trifluormethyl)phenyl, 4-Fluor-3-(trifluormethyl)phenyl oder 4-tert-Butylphenyl ist.

Die vorliegende Erfindung betrifft darüber hinaus auch die Salze, N-Oxide, Metallkomplexe der zuvor beschriebenen Verbindungen und deren Stereoisomere.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden.

Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkyl-Gruppen mit 1 bis 20 Kohlenstoffatomen), Arylsulfonsäuren oder -disulfonsäuren (aromatische Gruppen wie Phenyl und Naphthyl welche ein oder zwei Sulfonsäuregruppen tragen), Alkylphosphon-säuren (Phosphonsäuren mit geradkettigen oder verzweigten Alkyl-Gruppen mit 1 bis 20 Kohlenstoffatomen), Arylphosphonsäuren oder - diphosphonsäuren (aromatische Reste wie Phenyl und Naphthyl, welche ein oder zwei Phosphonsäure-Gruppen tragen), wobei die Alkyl- bzw. Aryl-Gruppen weitere Substituenten tragen können, z.B. p-Toluolsulfonsäure, Salicylsäure, p-Aminosalicylsäure, 2-Phenoxybenzoesäure, 2-Acetoxybenzoesäure, Saccharin etc.

Die so erhältlichen Salze weisen ebenfalls fungizide Eigenschaften auf.

### Herstellung der erfindungsgemäßen Amidine

Die erfindungsgemäßen Amidine können durch die im Folgenden dargestellten Verfahren erhalten werden:
1. Die erfindungsgemäßen Verbindungen der allgemeinen Formel (I-1) gemäß erster **Ausführungsform** können nach folgendem Reaktionsschema ausgehend von Pyrimidinen der allgemeinen Formel (II-1) nach unterschiedlichen alternativen Methoden unter Verwendung von
   (i) Aminoacetalen der Formel (III) oder
   (ii) Amiden der Formel (IV) oder
   (iii) Aminen der Formel (V) in Gegenwart von Orthoestern der Formel (VI) gemäß dem nachfolgenden Reaktionsschema erhalten werden: 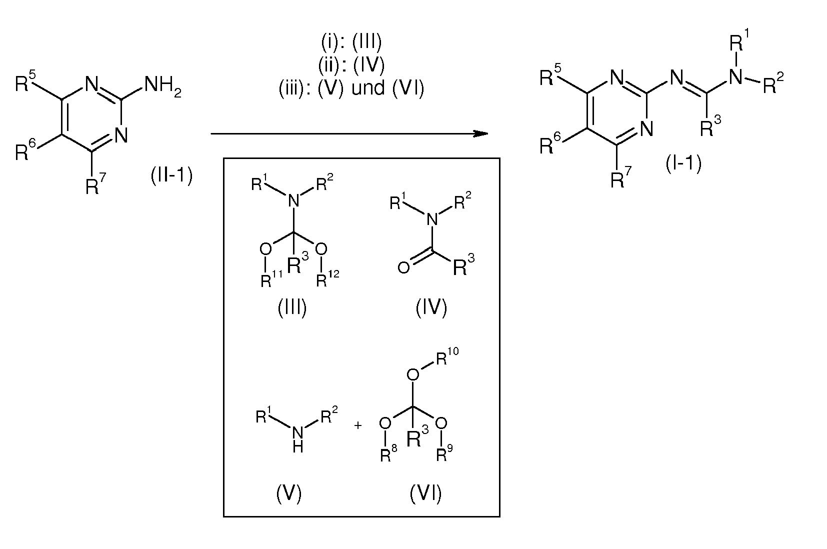 Die einzelnen alternativen Ausführungsformen (i) bis (iii) des erfindungsgemäßen Verfahrens sollen infolge kurz erläutert werden:
      (i) Gemäß einer erfindungsgemäßen Ausführungsform, die im obigen Schema als Schritt (i) dargestellt ist, werden die Verbindungen der allgemeinen Formel (II-1) mit Aminoacetalen der Formel (III), in der R¹, R² und R³ wie zuvor beschrieben definiert sind und R¹¹ und R¹² ausgewählt sind aus C₁₋₈-Alkyl-Gruppen, vorzugsweise aus C₂₋₆-Alkyl-Gruppen, besonders bevorzugt aus C₃₋₅-Alkyl-Gruppen und gemeinsam mit den O-Atomen, an die sie gebunden sind, einen fünf- oder sechsgliedrigen Ring bilden können, zu den erfindungsgemäßen Thiadiazolyloxyphenylamidinen der Formel (1-2) umgesetzt.
         Die Aminoacetale der Formel (III) sind aus den in JACS, 65, 1566 (1943) beschriebenen Formamiden durch Umsetzung mit Alkylierungsreagenzien, wie z.B. Dimethylsulfat, erhältlich.
         Die Reaktion gemäß Schritt (i) erfolgt vorzugsweise in Gegenwart einer Säure.
         Geeignete Säuren sind beispielsweise ausgewählt aus der Gruppe bestehend aus organischen und anorganischen Säuren, wobei p-Toluolsulfonsäure, Methansulfonsäure, Salzsäure (gasförmig, wässrig oder in organischer Lösung) oder Schwefelsäure.
         Die Reaktion gemäß Schritt (i) kann in einem Lösemittel durchgeführt werden, welches ausgewählt ist aus üblichen, bei den vorherrschenden Reaktionsbedingungen inerten Lösungsmitteln.
         Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.
         Besonders geeignete als Lösemittel ist N,N-Dimethylacetamid.
         Die Reaktion kann im Vakuum, bei Normaldruck oder unter Überdruck und bei Temperaturen von -20 bis 200 °C durchgeführt werden, vorzugsweise erfolgt die Reaktion bei Normaldruck und Temperaturen von 150 bis 200 °C. Die Umsetzung kann insbesondere in einer Mikrowelle durchgeführt werden.
      (ii) In einer alternativen erfindungsgemäßen Ausführungsform, die im obigen Schema als Schritt (ii) dargestellt ist, werden die Verbindungen der allgemeinen Formel (II-1) mit Amiden der Formel (IV), in der die Gruppen R¹, R² und R³ wie zuvor definiert sind, zu den erfindungsgemäßen Amidinen umgesetzt.
         Die Reaktion gemäß Schritt (ii) erfolgt ggf. in Gegenwart eines Halogenierungsmittels. Geeignete Halogenierungsmittel sind beispielsweise ausgewählt aus der Gruppe bestehend aus PCl₅, PCl₃, POCl₃ oder SOCl₂.
         Zudem kann die Reaktion alternativ in Gegenwart eines Kondensationsmittels erfolgen.
         Geeignete Kondensationsmittel sind solche, die üblicherweise zur Knüpfung von Amidbindungen verwendet werden, beispielhaft seien
         Säurehalogenidbildner wie z.B. Phosgen, Phosphortribromid, Phosphortrichlorid, Phosphorpentachlorid, Phosphortrichloridoxid oder Thionylchlorid; Anhydridbildner wie z.B. Chlorformiat, Methylchlorformiat, Isopropylchlorformiat, Isobutylchlorformiat oder Methansulfonylchlorid;
         Carbodiimine wie z.B. N,N'-Dicyclohexylcarbodiimin (DCC) oder andere übliche Kondensationsmittel wie z.B. Phosphorpentoxid, Polyphosphorsäure, N,N'-Carbodiimidazol, 2-Ethoxy-N-ethoxycarbonyl-1,2-dihydroquinolin (EEDQ), Triphenylphosphin/Tetrachlormethan oder Bromtripyrrolidinophosphoniumhexafluorophosphat genannt.
         Die Reaktion gemäß Schritt (ii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlor¬benzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzo¬nitril; Amide, wie beispielsweise N,N-Dimethylformamid (DMF), N,N-Di¬methyl¬acetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxi¬ethanol, Methoxyethanol, Diethylenglycolmonomethylether, Diethylenglycolmonoethylether oder Mischungen dieser verwendet.
      (iii) Gemäß einer weiteren alternativen erfindungsgemäßen Ausführungsform, die in obigenSchema als Schritt (iii) dargestellt ist, werden die Verbindungen der allgemeinen Formel (II-1) mit Aminen der Formel (V), in der die Gruppen R¹ und R² wie zuvor definiert sind in Gegenwart von Orthoestern der Formel (VI), in der R³ Wasserstoff ist und R⁸ bis R¹⁰ unabhängig voneinander ausgewählt sind aus C₁₋₈-Alkyl-Gruppen, vorzugsweise aus C₂₋₆-Alkyl-Gruppen, besonders bevorzugt aus C₃₋₅-Alkyl¬-Gruppen und gemeinsam mit den O-Atomen, an die sie gebunden sind, einen fünf- oder sechsgliedrigen Ring bilden können, zu den erfindungsgemäßen Thiadiazolyloxyphenylamidinen umgesetzt. Die Reaktion gemäß Schritt (iii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxyethanol, Methoxyethanol, Diethylenglycolmonomethylether, Diethylenglycolmonoethylether; oder Mischungen dieser mit Wasser sowie reines Wasser verwendet.
         Die Herstellung von entsprechenden mit Amin substituieren Pyrazinen der allgemeinen Formel (II) kann gemäß der für die sechste Ausführungsform beschriebene Reaktion erfolgen.
         Funktionalisierte Pyrimidine der allgemeinen Formel (II-1) sind durch Kupplungsreaktionen von entsprechenden halogenierten Pyrimidinen, beispielsweise durch Umsetzung mit Phenolen, zugänglich.
         Die entsprechend funktionalisierten halogenierten Aminopyrimidine sind entweder kommerziell erhältlich oder lassen sich gemäß verschiedener dem Fachmann geläufigen Methoden oder beispielsweise gemäß Science of Synthesis, 2004, Vol. 16 herstellen.
2. Die erfindungsgemäßen Verbindungen der allgemeinen Formel (1-2) gemäß **zweiter Ausführungsform** können nach folgendem Reaktionsschema ausgehend von Pyrazinen der allgemeinen Formel (II-2) nach unterschiedlichen alternativen Methoden unter Verwendung von
   (i) Aminoacetalen der Formel (III) oder
   (ii) Amiden der Formel (IV) oder
   (iii) Aminen der Formel (V) in Gegenwart von Orthoestern der Formel (VI) gemäß dem nachfolgenden Reaktionsschema erhalten werden: 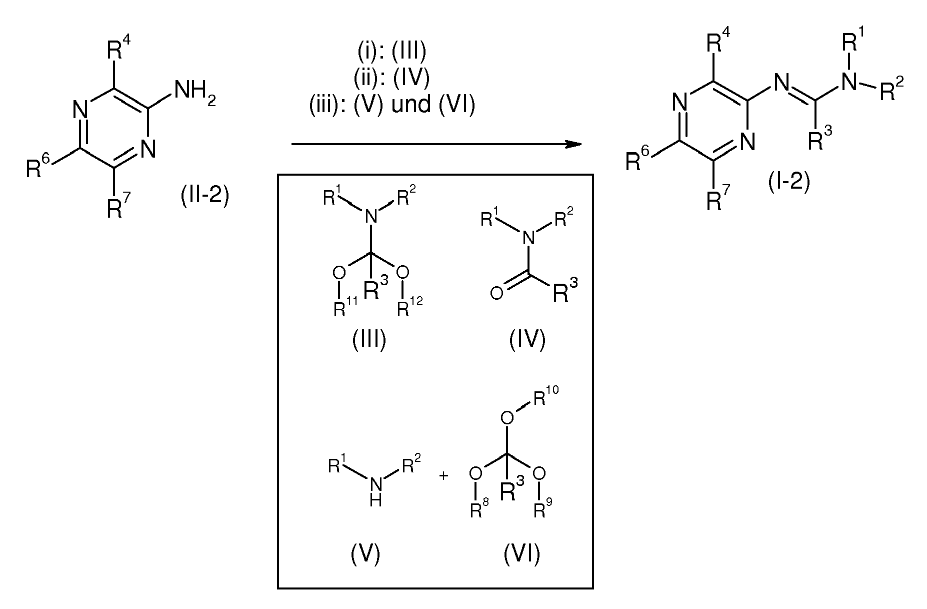 Die einzelnen alternativen Ausführungsformen (i) bis (iii) des erfindungsgemäßen Verfahrens wurden bereits vorstehend beschrieben. Insoweit wird auf obige Ausführungen verwiesen.
      Die Herstellung von entsprechenden mit Amin substituieren Pyrazinen der allgemeinen Formel (II) kann gemäß der für die sechste Ausführungsform beschriebene Reaktion erfolgen.
      Funktionalisierte Pyrazine der allgemeinen Formel (II-2) sind durch Kupplungsreaktionen von entsprechenden halogenierten Pyrazinen, beispielsweise durch Umsetzung mit Phenolen, zugänglich.
      Die entsprechend funktionalisierten halogenierten Aminopyrazine sind entweder kommerziell erhältlich oder lassen sich gemäß verschiedener dem Fachmann geläufigen Methoden oder beispielsweise gemäß Science of Synthesis, 2004, Vol. 16 herstellen.
3. Die erfindungsgemäßen Verbindungen der allgemeinen Formel (1-3) gemäß **dritter Ausführungsform** können nach folgendem Reaktionsschema ausgehend von Pyrimidinen der allgemeinen Formel (II-3) nach unterschiedlichen alternativen Methoden unter Verwendung von
   (i) Aminoacetalen der Formel (III) oder
   (ii) Amiden der Formel (IV) oder
   (iii) Aminen der Formel (V) in Gegenwart von Orthoestern der Formel (VI) gemäß dem nachfolgenden Reaktionsschema erhalten werden: 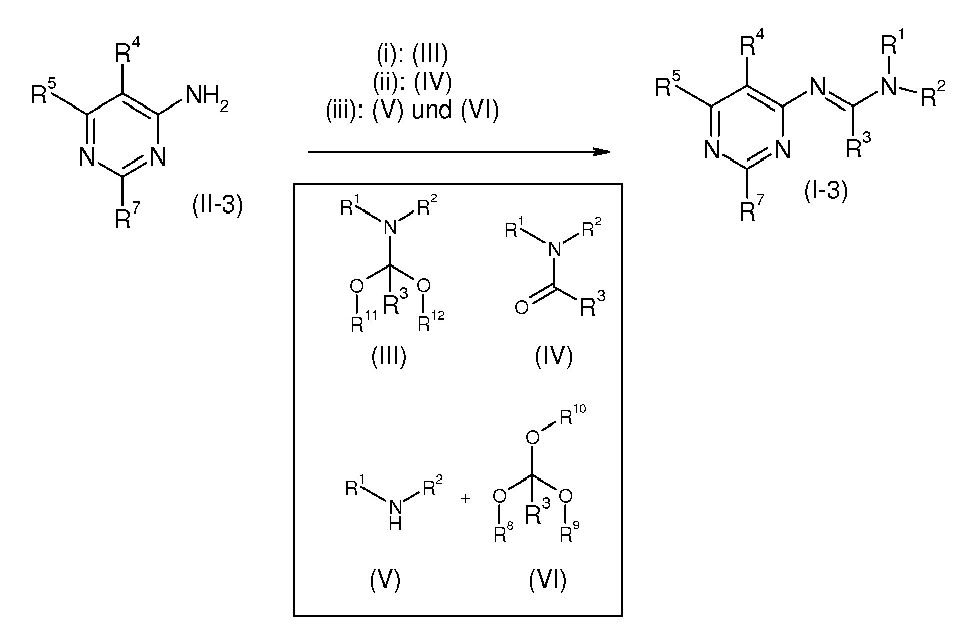 Die einzelnen alternativen Ausführungsformen (i) bis (iii) des erfindungsgemäßen Verfahrens wurden bereits vorstehend beschrieben. Insoweit wird auf obige Ausführungen verwiesen.
      Die Herstellung von entsprechenden mit Amin substituieren Pyrimidinen der allgemeinen Formel (II-3) kann gemäß der für die sechste Ausführungsform beschriebene Reaktion erfolgen.
      Funktionalisierte Pyrimidine der allgemeinen Formel (II-3) sind durch Kupplungsreaktionen von entsprechenden halogenierten Pyrimidinen, beispielsweise durch Umsetzung mit Phenolen, zugänglich.
      Die entsprechend funktionalisierten halogenierten Aminopyrimidine sind entweder kommerziell erhältlich oder lassen sich gemäß verschiedener dem Fachmann geläufigen Methoden oder beispielsweise gemäß Science of Synthesis, 2004, Vol. 16 herstellen.
4. Die erfindungsgemäßen Verbindungen der allgemeinen Formel (1-4) gemäß **vierter Ausführungsform** können nach folgendem Reaktionsschema ausgehend von Pyridazinen der allgemeinen Formel (II-4) nach unterschiedlichen alternativen Methoden unter Verwendung von
   (i) Aminoacetalen der Formel (III) oder
   (ii) Amiden der Formel (IV) oder
   (iii) Aminen der Formel (V) in Gegenwart von Orthoestern der Formel (VI) gemäß dem nachfolgenden Reaktionsschema erhalten werden: 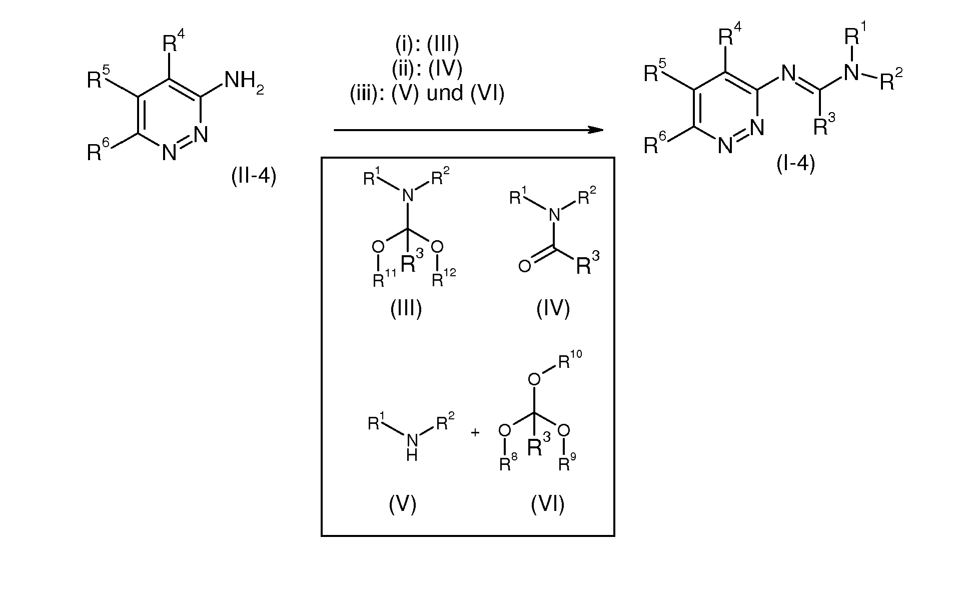 Die einzelnen alternativen Ausführungsformen (i) bis (iii) des erfindungsgemäßen Verfahrens wurden bereits vorstehend beschrieben. Insoweit wird auf obige Ausführungen verwiesen.
      Die Herstellung von entsprechenden mit Amin substituieren Pyridazinen der allgemeinen Formel (II-4) kann gemäß der für die sechste Ausführungsform beschriebene Reaktion erfolgen.
      Die entsprechend funktionalisierten halogenierten Aminopyridazine sind entweder kommerziell erhältlich oder lassen sich gemäß verschiedener dem Fachmann geläufigen Methoden oder beispielsweise gemäß Science of Synthesis, 2004, Vol. 16 herstellen.
      Funktionalisierte Pyridazine der allgemeinen Formel (II-4) sind durch Kupplungsreaktionen von entsprechenden halogenierten Pyridazinen, beispielsweise durch Umsetzung mit Phenolen, zugänglich.
5. Die erfindungsgemäßen Verbindungen der allgemeinen Formel (1-5) gemäß **fünfter Ausführungsform** können nach folgendem Reaktionsschema ausgehend von Triazinen der allgemeinen Formel (II-5) nach unterschiedlichen alternativen Methoden unter Verwendung von
   (i) Aminoacetalen der Formel (III) oder
   (ii) Amiden der Formel (IV) oder
   (iii) Aminen der Formel (V) in Gegenwart von Orthoestern der Formel (VI) gemäß dem nachfolgenden Reaktionsschema erhalten werden: 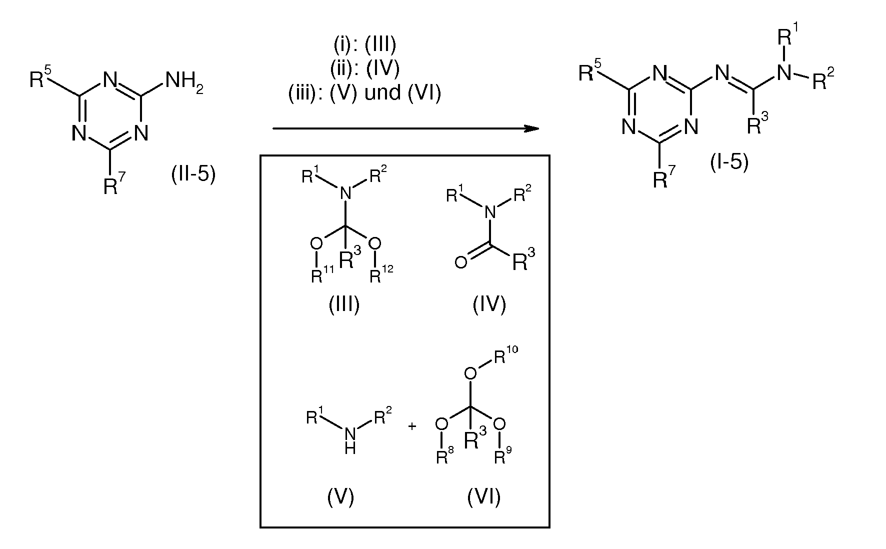 Die einzelnen alternativen Ausführungsformen (i) bis (iii) des erfindungsgemäßen Verfahrens wurden bereits vorstehend beschrieben. Insoweit wird auf obige Ausführungen verwiesen.
      Die Herstellung von entsprechenden mit Amin substituieren Triazinen der allgemeinen Formel (II-5) kann gemäß der für die sechste Ausführungsform beschriebene Reaktion erfolgen.
      Funktionalisierte Triazine der allgemeinen Formel (II-5) sind durch Kupplungsreaktionen von entsprechenden halogenierten Pyrazinen, beispielsweise durch Umsetzung mit Phenolen, zugänglich.
      Die entsprechend funktionalisierten halogenierten Aminotriazine sind entweder kommerziell erhältlich oder lassen sich gemäß verschiedener dem Fachmann geläufigen Methoden oder beispielsweise gemäß Science of Synthesis, 2004, Vol. 17 herstellen.
6. Die erfindungsgemäßen Verbindungen der allgemeinen Formel (I-6) gemäß **sechster Ausführungsform** können nach folgendem Reaktionsschema erhalten werden:

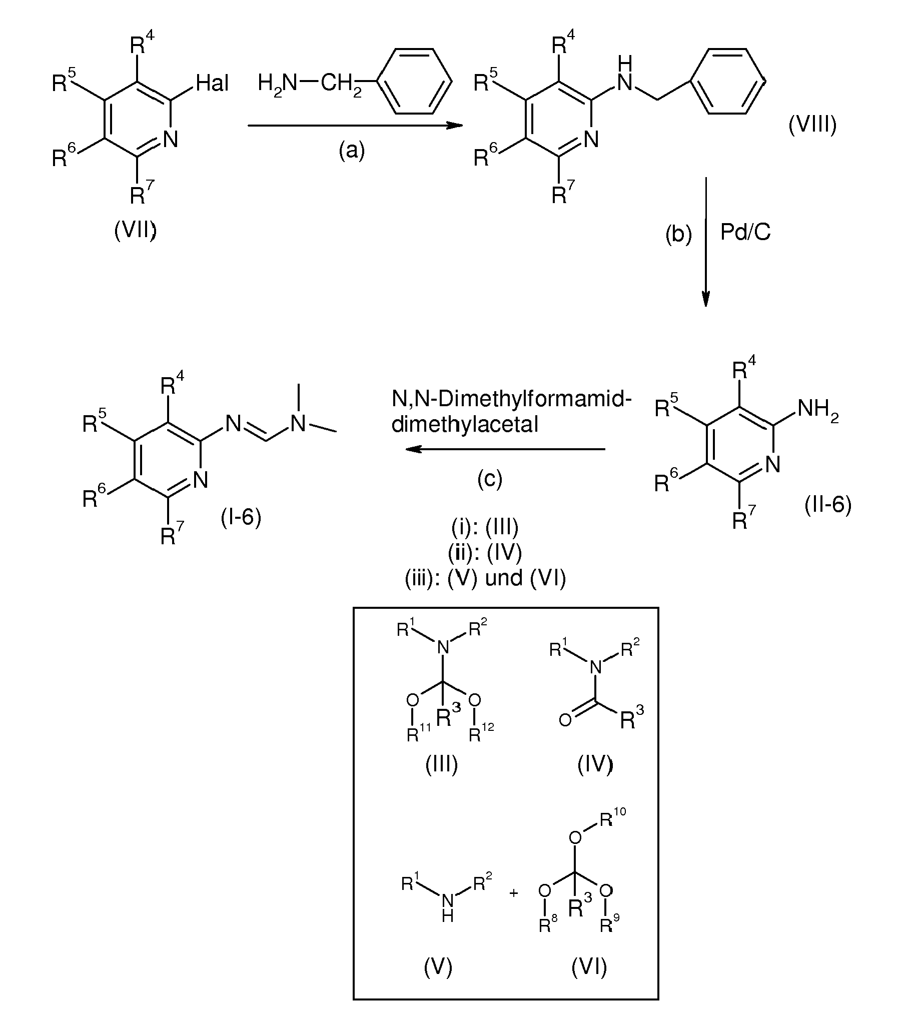

### Verfahrensschritt (a):

In Verfahrensschritt (a) werden entsprechende Halogen-Pyridine der allgemeinen Formel (VII) zunächst mit Benzylamin in Gegenwart von Alkalimetallhalogeniden, bevorzugt Cäsiumfluorid gekuppelt.

Die Reaktion kann dabei in einem Lösemittel durchgeführt werden, welches ausgewählt ist aus üblichen, bei den vorherrschenden Reaktionsbedingungen inerten Lösungsmitteln.

Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.

Die Reaktion kann im Vakuum, bei Normaldruck oder unter Überdruck und bei Temperaturen von -20 bis 200 °C durchgeführt werden, vorzugsweise erfolgt die Reaktion bei Normaldruck und Temperaturen von 50 bis 150 °C.

Die Halogen-Pyridine der allgemeinen Formel (VII) sind entweder kommerziell erhältlich oder lassen sich gemäß verschiedener dem Fachmann geläufigen Methoden oder beispielsweise gemäß Science of Synthesis, 2005, Vol. 15 herstellen.

### Verfahrensschritt (b):

In Verfahrensschritt (b) wird unter Einwirkung eines Palladium-haltigen Katalysators eine freie Aminfunktion an dem Heterocyclus erzeugt. Die benzylischen Schutzgruppen können hydrogenolytisch mit einer Wasserstoffquelle (z.B. Wasserstoff, Ammoniumfomiat, Ameisensäure oder Cyclohexen) in Gegenwart eines Katalysators (z.B. Palladium auf Aktivkohle oder Palladiumhydroxid auf Aktivkohle) entfernt werden.

Zu diesem Zweck wird die Verbindung der allgemeinen Formel (6) mit einem Palladium-haltigen Katalysator in einem Lösemittel erwärmt. Die Reaktion kann dabei in einem Lösemittel durchgeführt werden, welches ausgewählt ist aus üblichen, bei den vorherrschenden Reaktionsbedingungen inerten Lösungsmitteln.

Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.

Besonders geeignete als Lösemittel sind Alkohole in Kombination mit Cyclohexan, beispielsweise Methanol in Kombination mit Cyclohexan.

### Verfahrensschritt (c):

In Verfahrensschritt (c) erfolgt die Amidinbildung durch Umsetzung der Verbindung der allgemeinen Formel (II-6) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI)

Die einzelnen alternativen Ausführungsformen (i) bis (iii) des erfindungsgemäßen Verfahrens wurden bereits vorstehend beschrieben. Insoweit wird auf obige Ausführungen verwiesen.

Funktionalisierung an dem Pyridinring der Heteroamidine der allgemeinen Formel (I-6) können im Anschluss an den Verfahrensschritt (c) beispielsweise durch Verwendung entsprechender halogenierter Pyridine der allgemeinen Formel (VII), d.h. mindestens ein Rest von R⁴, R⁵, R⁶ und R⁷ ist gleich Halogen, vorzugsweise Iod, Brom oder Chlor, und deren Umsetzung, beispielsweise deren palladiumvermittelten Umsetzung (Suzuki-Kupplung), erhalten werden.

Des Weiteren können an dem Pyridinring über Sauerstoff gebundene Substituenten (d.h. Verbindungen der allgemeinen Formel (I), in welchen mindestens ein Rest von R⁴, R⁵, R⁶ und R⁷ gleich -OR^{b} ist (R^{b} steht dabei für gegebenenfalls substituierte Reste, ausgewählt aus C₁-C₁₂-Alkyl, C₃-C₁₀-Carbocyclyl oder C₃-C₁₀-Heterocyclyl), dadurch erhalten werden, dass in dem Verfahrensschritt (a) eine Verbindung der allgemeinen Formel (VII) als Edukt verwendet wird, welche bereits einen entsprechenden Oxysubstituenten trägt. Der Zugang zu derartigen Verbindungen ist literaturbeschrieben und kann z.B. ausgehend aus den entsprechenden Halogenpyridinen durch Umsetzung mit Alkoholen der Form HOR^{b} erfolgen (Bioorganic & Medicinal Chemistry, 2008, 16, 4963-4983).

Aryl- oder alkylsubstitueierte Ausgangsverbindungen der allgemeinen Formel (1) sind ausgehend von entsprechend halogenierten Pyridinverbindungen durch palladiumvermittelte Kupplungsreaktionen, beispielsweise durch Suzuki-Kupplung, erhältlich.

Die entsprechend funktionalisierten halogenierten Pyridine sind entweder kommerziell erhältlich oder lassen sich gemäß verschiedener dem Fachmann geläufigen Methoden oder beispielsweise gemäß Science of Synthesis, 2005, Vol. 15 herstellen. Ebenso lassen sich weitere Aminopyridine gemäß Science of Synthesis, 2005, Vol. 15 herstellen.

### Bekämpfung von unerwünschten Mikroorganismen

Die erfindungsgemäßen Amidine weisen eine starke mikrobizide Wirkung auf und können zur Bekämpfung von unerwünschten Mikroorganismen, wie Fungi (Pilze) und Bakterien, im Pflanzenschutz und im Materialschutz eingesetzt werden.

### Pflanzenschutz

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Alternaria-Arten, wie beispielsweise Alternaria brassicicola
Aphanomyces-Arten, wie beispielsweise Aphanomyces euteiches
Ascochyta-Arten, wie beispielsweise Ascochyta lentis
Aspergillus-Arten, wie beispielsweise Aspergillus flavus
Cladosporium-Arten, wie beispielsweise Cladosporium herbarum
Cochliobolus-Arten, wie beispielsweise Cochliobolus sativus
(Konidienform: Drechslera, Bipolaris Syn: Helminthosporium);
Colletotrichum-Arten, wie beispielsweise Colletotrichum coccodes;
Fusarium-Arten, wie beispielsweise Fusarium culmorum;
Gibberella-Arten, wie beispielsweise Gibberella zeae;
Macrophomina-Arten, wie beispielsweise Macrophomina phaseolina
Monographella-Arten, wie beispielsweise Monographella nivalis;
Penicillium-Arten, wie beispielsweise Penicillium expansum
Phoma-Arten, wie beispielsweise Phoma lingam
Phomopsis-Arten, wie beispielsweise Phomopsis sojae;
Phytophthora Arten, wie beispielsweise Phytophthora cactorum;
Pyrenophora-Arten, wie beispielsweise Pyrenophora graminea
Pyricularia-Arten, wie beispielsweise Pyricularia oryzae;
Pythium-Arten, wie beispielsweise Pythium ultimum;
Rhizoctonia-Arten, wie beispielsweise Rhizoctonia solani;
Rhizopus-Arten, wie beispielsweise Rhizopus oryzae
Sclerotium-Arten, wie beispielsweise Sclerotium rolfsii;
Septoria-Arten, wie beispielsweise Septoria nodorum;
Typhula-Arten, wie beispielsweise Typhula incarnata;
Verticillium-Arten, wie beispielsweise Verticillium dahliae
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraums nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

### Mycotoxine

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

### Materialschutz

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

### Formulierungen

Die vorliegende Erfindung betrifft ein Mittel zum Bekämpfen unerwünschter Mikroorganismen, umfassend wenigstens eines der erfindungsgemäßen Isothiazolyloxyphenylamidine.

Die erfindungsgemäßen Isothiazolyloxyphenylamidine können dazu in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die zuvor beschriebenen Formulierungen können in einem erfindungsgemäßen Verfahren zum Bekämpfen unerwünschter Mikroorganismen verwendet werden, bei dem die erfindungsgemäßen Isothiazolyloxyphenylamidine auf die Mikroorganismen und/oder in deren Lebensraum ausgebracht werden.

### Saatgutbehandlung

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

### Mischung mit bekannten Fungiziden, Bakteriziden, Akariziden, Nematiziden oder Insektiziden

Die erfindungsgemäßen Amidine können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Isothiazolyloxyphenylamidine können daher sowohl in medizinische als auch in nicht-medizinische Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz der erfindungsgemäßen Isothiazolyloxyphenylamidine als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

### GMOs

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-StreßBedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfojrc.it/gmp_browse.aspx und http://www.agbios.com/dbase.php).

Die vorliegende Erfindung wird anhand der nachfolgenden Beispiele näher erläutert, welche die vorliegende Erfindung jedoch keinesfalls einschränken.

### Herstellungsbeispiele:

### (1) Herstellung von Verbindungen gemäß erster Ausführunsgform

### N-Ethyl-N'-{5-ethyl-4-[4-fluor-3-(trifluormethyl)phenoxy]-6-methylpyrimidin-2-yl}-N-methylformamidin

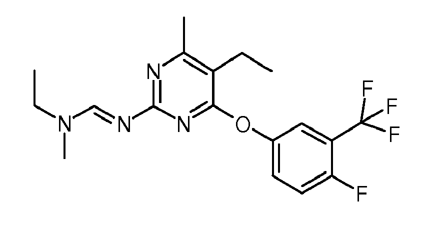

200 mg (0.63 mmol) 2-Amino-5-ethyl-4-[4-fluor-3-(trifluormethyl)phenoxy]-6-methylpyrimidin und 168 mg (1.27 mmol) N,N-Ethylmethylformamiddimethylacetal werden in 4 ml N,N-Dimethylformamid gelöst und in der Mikrowelle (180°C, 5400 sec.) zur Reaktion gebracht. Anschliessend wird das Reaktionsgemisch vom Lösungsmittel befreit und säulenchromatographisch aufgearbeitet. Man erhält 170 mg Produkt (95% Reinheit, 66% Ausbeute).

Synthese der Ausgangsverbindungen:

### 2-Amino-5-ethyl-4-[4-fluor-3-(trifluormethyl)phenoxy]-6-methylpyrimidin

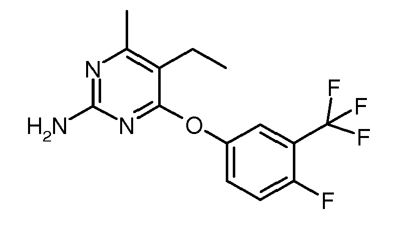

520 mg (2.99 mmol) 4-Fluor-3-Trifluormethylphenol werden in 8 ml Dimethylacetamid 15 min gerührt und mit 472 mg (2.75 mmol) 4-Chlor-5-ethyl-6-methylpyrimidin-2-amin versetzt und in der Mikrowelle (180°C, 5400sec) zur Reaktion gebracht. Anschliessend wird das Reaktionsgemisch vom Lösungsmittel befreit und säulenchromatographisch aufgearbeitet. Man erhält 479 mg Produkt (90% Reinheit, 50% Ausbeute).

### (2) Herstellung von Verbindungen gemäß zweiter Ausführunsgform

### N'-{6-[4-Chlor-3-(trifluormethyl)phenoxy]pyrazin-2-yl}-N,N-Dimethylformamidin

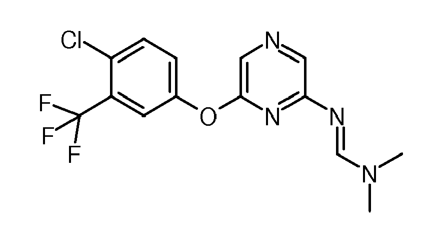

200 mg (0.69 mmol) 6-[4-Chlor-3-(trifluormethyl)phenoxy]-2-aminopyrazin und 164 mg (1.38 mmol) N,N-Ethylmethylformamiddimethylacetal werden in 4 ml N,N-Dimethylformamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 146 mg Produkt (90% Reinheit, 55% Ausbeute).

### Synthese der Ausgangsverbindungen:

### 6-[4-Chlor-3-(trifluormethyl)phenoxy]-2-aminopyrazin

1.08 g (5.5 mmol) 2-Chlor-5-hydroxybenzotrifluorid und 0.65 g (5 mmol) 6-Chlor-2-pyrazinamin werden in 7.5 ml DMF gelöst und mit 0.9 g (6.5 mmol) Kaliumcarbonat versetzt und 3 h zum Rückfluß erhitzt. Anschließend werden nochmals 1.7 g (13 mmol) Kaliumcarbonat zugesetzt und weitere 2 h zum Rückfluß erhitzt. Nach erneuter Zugabe von 1.7 g (13 mmol) Kaliumcarbonat wird weitere 16 h zum Rückfluß erhitzt. Nach Abkühlen wird die Reaktionsmischung auf Eiswasser gegossen, 2 mal mit Essigester ausgeschüttelt, die organische Phase abgetrennt, über Natriumsulfat getrocknet, filtriert und im Vakuum zur Trockne eingeengt. Man erhält 1.08 g (90% Reinheit, 66% Ausbeute).

### N'-{5-[4-Chlor-3-(trifluormethyl)phenyl]pyrazin-2-yl}-N,N-Dimethylformamidin

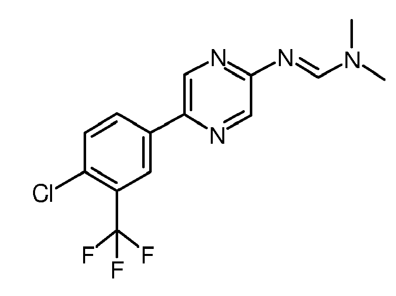

220 mg (0.80 mmol) 6-[4-Chlor-3-(trifluormethyl)phenoxy]-2-aminopyrazin und 191 mg (1.61 mmol) N,N-Ethylmethylformamiddimethylacetal werden in 4 ml N,N-Dimethylformamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 215 mg Produkt (90% Reinheit, 73% Ausbeute).

### Synthese der Ausgangsverbindungen:

### 5-[4-Chlor-3-(trifluormethyl)phenoxy]-2-aminopyrazin

1 g (5.74 mmol) 5-Brom-2-pyrazinamin und 1.35 g (6.0 mmol) 4-Chlor-3-trifluormethylphenylboronsäure werden in einem Gemisch aus 300 ml Dioxan und 30 ml Wasser vorgelegt und mit 2.38 g (17 mmol) Kaliumcarbonat und 201 mg (0.29 mmol) Bis(triphenylphosphin)palladium(II)chlorid versetzt und für 4 h zum Rückfluß erhitzt. Nach Abkühlen der Reaktionsmischung wird die Dioxanphase abgetrennt, zur Trockne einrotiert und der Rückstand in Ethylacetat aufgenommen. Nach Waschen mit Wasser, Trocknen über Natriumsulfat und filtrieren wird das Lösungsmittel im Vakuum entfernt. Nach säulenchromatographischer Aufreinigung erhält man 373 mg Produkt (90% Reinheit, 21 % Ausbeute).

### (3) Herstellung von Verbindungen gemäß dritter Ausführunsgform

### N'-{2-[4-Chlor-3-(trifluormethyl)phenoxy]-6-methylpyrimidin-4-yl}-N,N-Ethylmethyl-formamidin

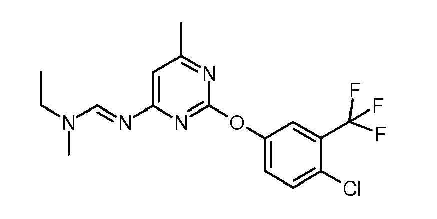

120 mg (0.40 mmol) 4-Amino-2-[4-Chlor-3-(trifluormethyl)phenoxy]-6-methylpyrimidin und 105 mg (0.80 mmol) N,N-Ethylmethylformamiddimethylacetal werden in 4 ml N,N-Dimethylformamid gelöst und in der Mikrowelle (200°C, 3600 sec.) zur Reaktion gebracht. Anschliessend wird das Reaktionsgemisch vom Lösungsmittel befreit und säulenchromatographisch aufgearbeitet. Man erhält 130 mg Produkt (85% Reinheit, 75% Ausbeute).

### Synthese der Ausgangsverbindungen:

### 4-Amino-2-[4-Chlor-3-(trifluormethyl)phenoxy]-6-methylpyrimidin

266 mg (1.29 mmol) 4-Chlor-3-trifluormethylphenol werden in 3 ml Dimethylacetamid gelöst, mit 46 mg Natrium hydrid versetzt und 15 min gerührt und dann mit 185 mg (1.29 mmol) 2-Chlor-6-methylpyrimidin-4-amin versetzt und in der Mikrowelle (165°C, 7200sec) zur Reaktion gebracht. Anschliessend wird das Reaktionsgemisch vom Lösungsmittel befreit und säulenchromatographisch aufgearbeitet. Man erhält 190mg Produkt (85% Reinheit, 41% Ausbeute).

### (4) Herstellung von Verbindungen gemäß vierter Ausführunsgform

### N,N-Ethylmethyl-N'-(4-methyl-6-phenylpyridazin-3-yl)formamidin

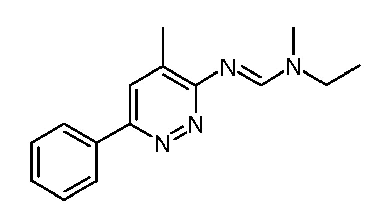

250 mg (1.35 mmol) 3-Amino-4-methyl-6-phenylpyridazin und 359 mg (2.70 mmol) N,N-Ethylmethylformamiddimethylacetal werden in 4 ml N,N-Dimethylformamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 316 mg Produkt (90% Reinheit, 82% Ausbeute).

### N,N-Ethylmethyl-N'-(6-phenoxypyridazin-3-yl))formamidin

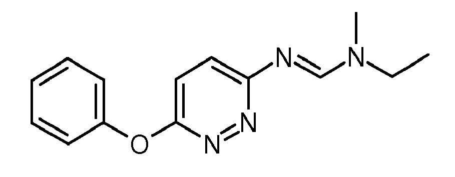

250 mg (1.34 mmol) 3-Amino-6-phenoxypyridazin und 355 mg (2.67 mmol) N,N-Ethylmethylformamiddimethylacetal werden in 4 ml N,N-Dimethylformamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 395 mg Produkt (90% Reinheit, 78% Ausbeute).

### Synthese der Ausgangsverbindungen

### 3-Amino-6-phenoxypyridazin:

Kann gemäß Patent US2891953, 1959, American Cyanamide hergestellt werden.

### (5) Herstellung von Verbindungen gemäß fünfter Ausführunsgform

### N,N-Dimethyl-N'-(4-methyl-6-phenyl-1,3,5-triazin-2-yl)imidoformamid

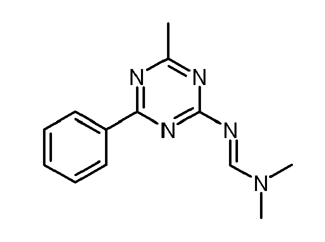

200 mg (1.07 mmol) 4-methyl-6-phenyl-1,3,5-triazin-2-amin und 255 mg (2.15 mmol) N,N-Ethylmethylformamiddimethylacetal werden in 2 ml N,N-Dimethylacetamid gelöst und in der Mikrowelle (80°C, 5400 sec.) zur Reaktion gebracht. Anschliessend wird das Reaktionsgemisch vom Lösungsmittel befreit und säulenchromatographisch aufgearbeitet. Man erhält 203 mg Produkt (90% Reinheit, 70% Ausbeute).

### N-Ethyl-N'-(4-methoxy-6-methyl-1,3,5-triazin-2-yl)-N-methylimidoformamid

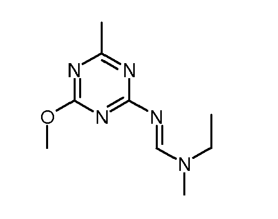

250 mg (1.78 mmol) 4-methoxy-6-methyl-1,3,5-triazin-2-amine und 475 mg (3.6 mmol) N,N-Ethylmethylformamiddimethylacetal werden in 2 ml N,N-Dimethylacetamid gelöst und in der Mikrowelle (100°C, 4500 sec.) zur Reaktion gebracht. Anschliessend wird das Reaktionsgemisch vom Lösungsmittel befreit und säulenchromatographisch aufgearbeitet. Man erhält 210 mg Produkt (90% Reinheit, 50% Ausbeute).

### N-Ethyl-N'-(4,6-dimethyl-1,3,5-triazin-2-yl)-N-methylimidoformamid

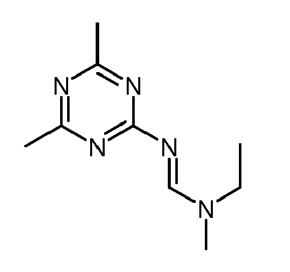

250 mg (1.78 mmol) 4,6-Dimethyl-1,3,5-triazin-2-amin und 475 mg (3.6 mmol) N,N-Ethylmethylformamiddimethylacetal werden in 2 ml N,N-Dimethylacetamid gelöst und in der Mikrowelle (100°C, 4500 sec.) zur Reaktion gebracht. Anschliessend wird das Reaktionsgemisch vom Lösungsmittel befreit und säulenchromatographisch aufgearbeitet. Man erhält 215 mg Produkt (95% Reinheit, 57% Ausbeute).

### (6) Herstellung von Verbindungen gemäß sechster Ausführunsgform

### N,N-Dimethyl-N'-(6-[3-trifluoromethylphenoxy]-2-pyridyl)formamidin

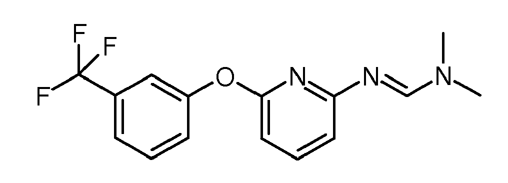

270 mg (1.06 mmol) 2-Amino-6-(3-trifluormethylphenoxy)-pyridin und 253 mg (2.12 mmol) N,N-Dimethylformamiddimethylacetal werden in 4 ml N,N-Dimethylacetamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 294 mg Produkt (90% Reinheit, 80% Ausbeute).

Synthese der Ausgangsverbindungen:

### 2-Amino-6-(3-trifluormethylphenoxy)-pyridin

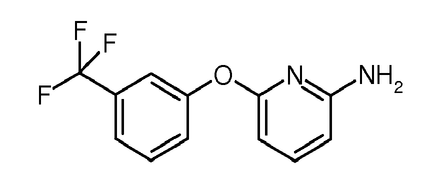

990 mg (2.87 mmol) 2-Benzylamino-6-(3-trifluormethylphenoxy)-pyridin und 100 mg Pd (10%ig auf Kohle) werden in 390 ml Methanol suspendiert und mit 15 ml Cyclohexen 4 h refluxiert. Anschliessend wird das Reaktionsgemisch filtriert, im Vakuum vom Lösungsmittel befreit und dreimal mit Wasser/Ethylacetat extrahiert. Die vereingten organischen Phasen werden über Natriumsulfat getrocknet, filtriert und im Vakuum vom Lösungsmittel befreit. Anschliessend wird das Rohprodukt säulenchromatographisch aufgereinigt (283 mg, 90% Reinheit, 35% Ausbeute).

### 2-Benzylamino-6-(3-trifluormethylphenoxy)-pyridin

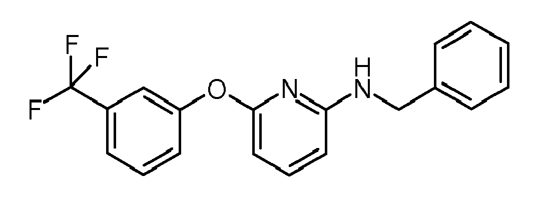

2.00 g (7.33 mmmol) 2-Chloro-6-(3-trifluormethylphenoxy)-pyridin und 824 mg (7.70 mmol) Benzylamin werden in 25 ml 1-Methyl-2-pyrrolidon gelöst, mit 1.22 g (8.06 mmol) Cäsiumfluorid versetzt und in der Mikrowelle (190°C, 4h.) zur Reaktion gebracht. Anschliessend wird das Reaktionsgemisch vom Lösungsmittel befreit und und dreimal mit Wasser/Ethylacetat extrahiert. Die vereingten organischen Phasen werden über Natriumsulfat getrocknet, filtriert und im Vakuum vom Lösungsmittel befreit. Anschliessend wird das Rohprodukt säulenchromatographisch aufgereinigt. Man erhält 992 mg Produkt (70% Reinheit, 27 % Ausbeute).

### N,N-Dimethyl-N'-(3-methyl-5-(4-fluor-3-trifluoromethyl-phenyl)-2-pyridyl)formamidin

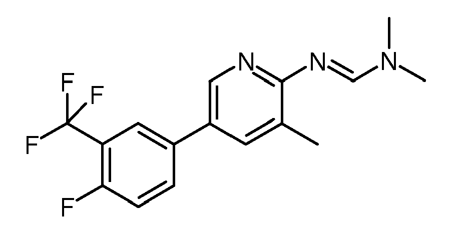

350 mg (1.21 mmol) N,N-Dimethyl-N'-(5-jod-3-methyl-2-pyridyl)formamidin und 42 mg (0.06 mmol) Bis(triphenylphosphin)palladium(II)chlorid werden in 15 ml Dioxan vorgelegt, 30 min bei Raumtemperatur gerührt und anschliessend mit 264 mg (1.27 mmol) 4-Fluor-3-trifluormethylphenylboronsäure, 502 mg (3.63 mmol) Kaliumcarbonat und 2 ml Wasser versetzt. Die Reaktionsmischung wird 4 h refluxiert, abgekühlt, vom Lösungsmittel befreit und und dreimal mit Wasser/Ethylacetat extrahiert. Die vereingten organischen Phasen werden über Natriumsulfat getrocknet, filtriert und im Vakuum vom Lösungsmittel befreit. Anschliessend wird das Rohprodukt säulenchromatographisch aufgereinigt. Man erhält 224 mg Produkt (90% Reinheit, 51 % Ausbeute).

### Synthese der Ausgangsverbindungen:

### N,N-Dimethyl-N'-(5-jod-3-methyl-2-pyridyl)formamidin

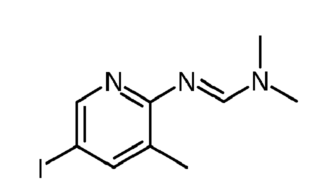

3.00 g (12.8 mmol) 2-Amino-5-jod-3-methylpyridin und 3.05 g (25.6 mmol) N,N-Dimethylformamiddimethylacetal werden in 15 ml N,N-Dimethylacetamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 3.10 g Produkt (90% Reinheit, 75% Ausbeute).

### N,N-Dimethyl-N'-(5-methyl-3-[3-trifluormethylphenyl]-pyrid-2-yl)formamidin

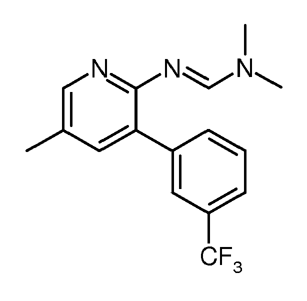

220 mg (0.87 mmol) 2-Amino-5-methyl-3-(3-trifluormethylphenyl)pyridin und 207 mg (1.74 mmol) N,N-Dimethylformamiddimethylacetal werden in 4 ml N,N-Dimethylacetamid gelöst und in der Mikrowelle (180°C, 5400 sec.) zur Reaktion gebracht. Anschliessend wird das Reaktionsgemisch vom Lösungsmittel befreit und säulenchromatographisch aufgearbeitet. Man erhält 255 mg Produkt (90% Reinheit, 86% Ausbeute).

### Synthese der Ausgangsverbindungen:

### 2-Amino-5-methyl-3-(3-trifluormethylphenyl)pyridin

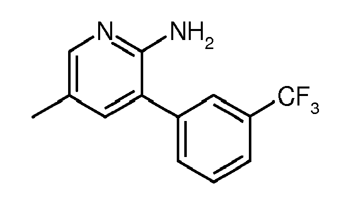

2.00 g (10.69 mmol) 2-Amino-3-brom-5-methylpyridin und 375 mg (0.53 mmol) Bis(triphenylphosphin)palladium(II)chlorid werden in 300 ml Dioxan vorgelegt, 30 min bei Raumtemperatur gerührt und anschliessend mit 2.31 g (11.23 mmol) 3-Trifluormethylphenylboronsäure, 4.43 g (32.08 mmol) Kaliumcarbonat und 30 ml Wasser versetzt. Die Reaktionsmischung wird 4 h refluxiert, abgekühlt, vom Lösungsmittel befreit und und dreimal mit Wasser/Ethylacetat extrahiert. Die vereingten organischen Phasen werden über Natriumsulfat getrocknet, filtriert und im Vakuum vom Lösungsmittel befreit. Anschliessend wird das Rohprodukt säulenchromatographisch aufgereinigt. Man erhält 1.29 g Produkt (90% Reinheit, 43% Ausbeute).

### N,N-Ethylmethyl-N'-(6-[2-phenoxyphenyl]-2-pyridyl)formamidin

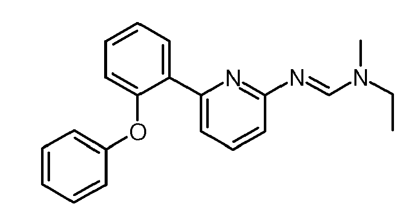

343 mg (1.30 mmol) 2-Amino-6-(2-phenoxyphenyl)pyridin und 253 mg (1.83 mmol) N,N-Ethylmethylformamiddimethylacetal werden in 10 ml Toluol gelöst und 18h bei 80°C gerührt. Anschliessend wird das Reaktionsgemisch vom Lösungsmittel befreit und säulenchromatographisch aufgearbeitet. Man erhält 188 mg Produkt (88% Reinheit, 38% Ausbeute).

### Synthese der Ausgangsverbindungen:

### 2-Amino-6-(2-phenoxyphenyl)pyridin

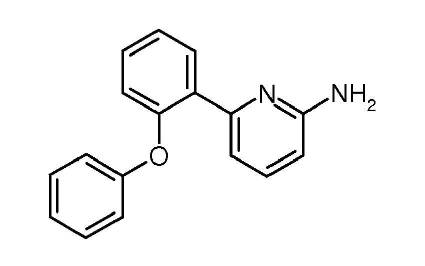

500 mg (2.89 mmol) 2-Amino-6-brompyridin und 804 mg (3.75 mmol) 2-Phenoxyphenylboronsäure werden unter Argon-Schutzgasatmosphäre in 9 ml 1-Butanol vorgelegt und mit 3.76 g (11.5 mmol) Cäsiumcarbonat, 67 mg (0.05 mmol) Tetrakis(triphenylphosphin)palladium(0) und 3 ml Wasser versetzt. Das Reaktionsgemisch wird 18 h bei 80°C gerührt, vom Lösungsmittel befreit und und dreimal mit Wasser/Ethylacetat extrahiert. Die vereingten organischen Phasen werden über Natriumsulfat getrocknet, filtriert und im Vakuum vom Lösungsmittel befreit. Anschliessend wird das Rohprodukt säulenchromatographisch aufgereinigt. Man erhält 520 mg Produkt (99% Reinheit, 68% Ausbeute).

### N'-{3-[(3,5-difluorobenzyl)oxy]pyridin-2-yl}-N-ethyl-N-methylimidoformamid

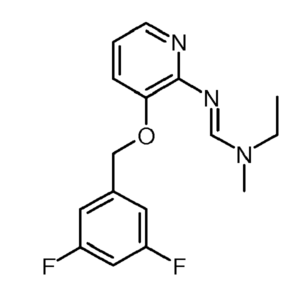

248 mg (1.48 mmol) 3-[(3,5-difluorobenzyl)oxy]pyridin-2-amin und 220 mg (0.93 mmol) N,N-Ethylmethylformamiddimethylacetal werden in 4 ml N,N-Dimethylacetamid gelöst und in der Mikrowelle (180°C, 6000 sec.) zur Reaktion gebracht. Anschliessend wird das Reaktionsgemisch vom Lösungsmittel befreit und säulenchromatographisch aufgearbeitet. Man erhält 204 mg Produkt (90% Reinheit, 65% Ausbeute).

### Synthese der Ausgangsverbindungen

### 3-[(3,5-difluorobenzyl)oxy]pyridin-2-amin

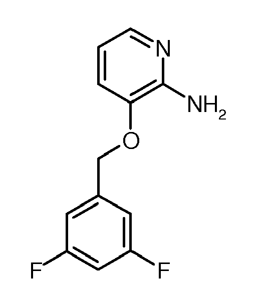

3-[(3,5-difluorobenzyl)oxy]pyridin-2-amin kann z.B. analog zu WO9315055 oder WO 2004004720 hergestellt werden.

Analog zu den vorbeschriebenen Verfahren wurden hergestellt:

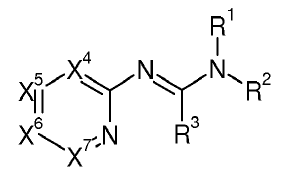

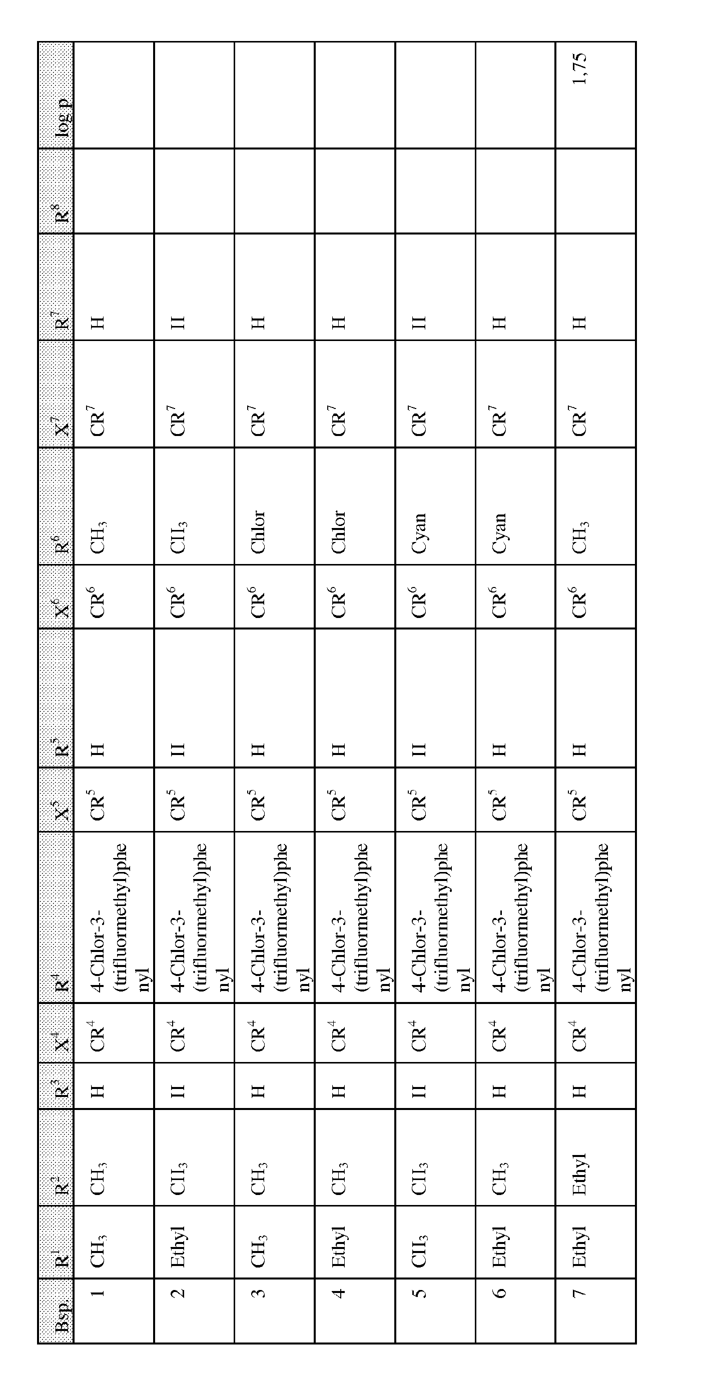

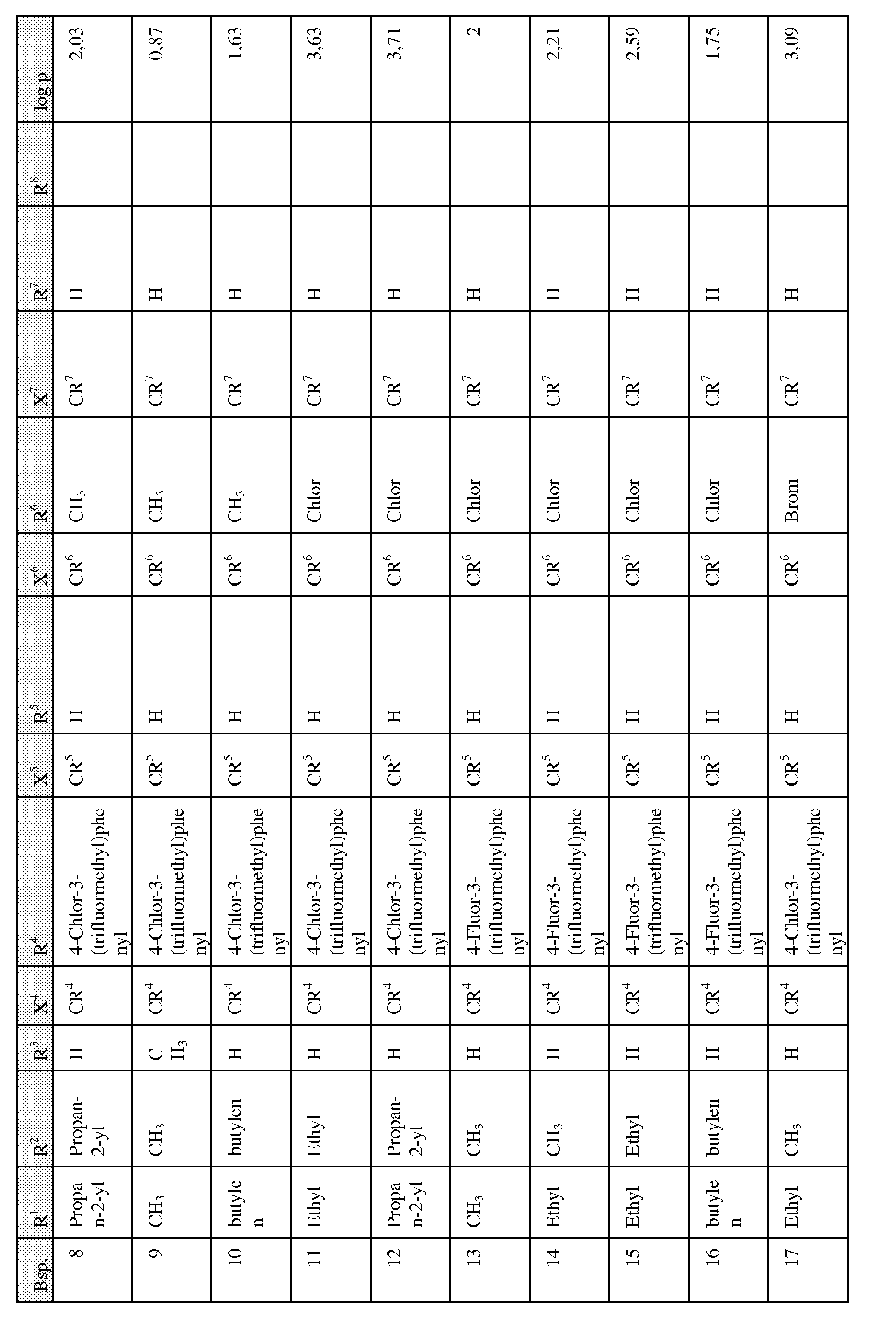

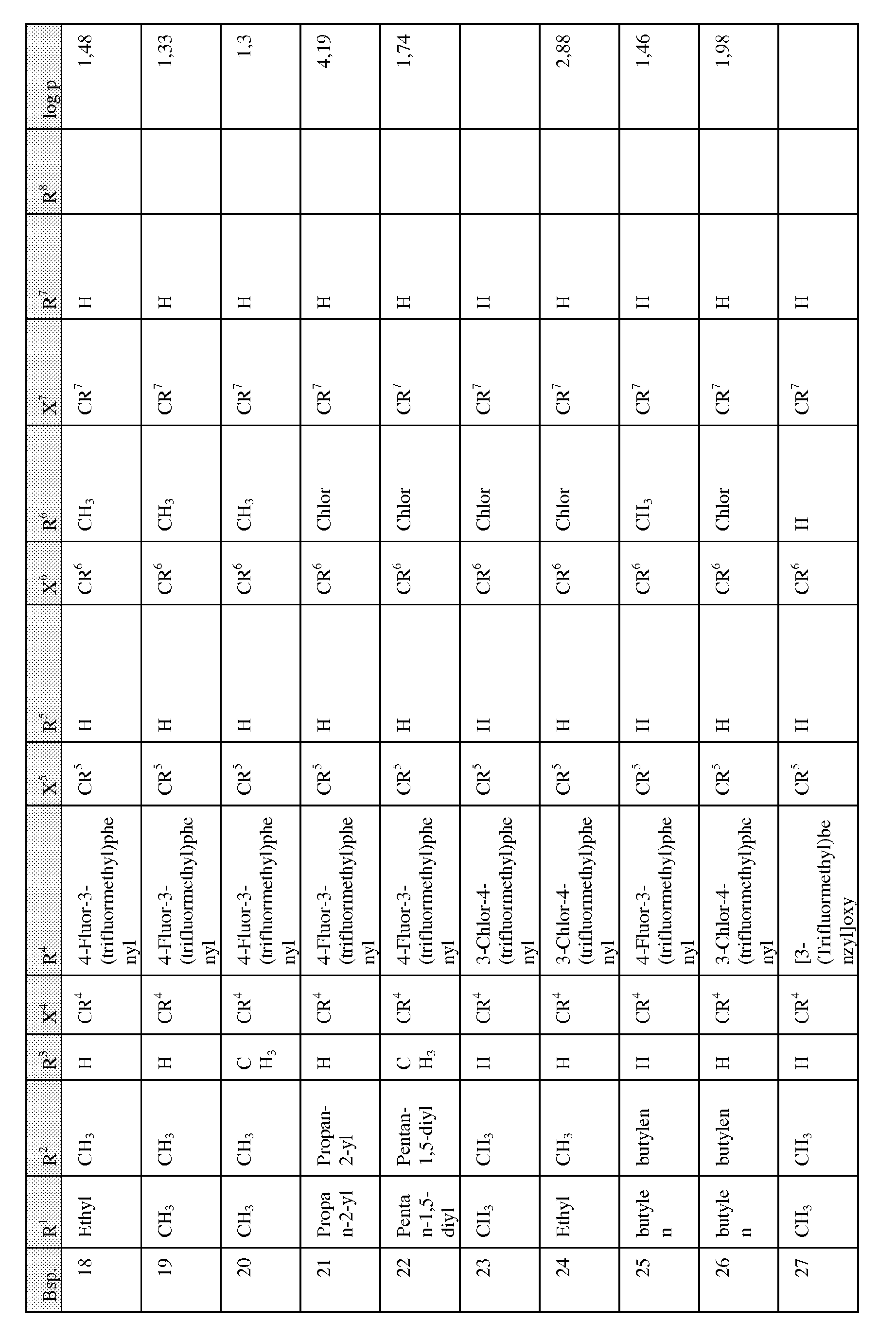

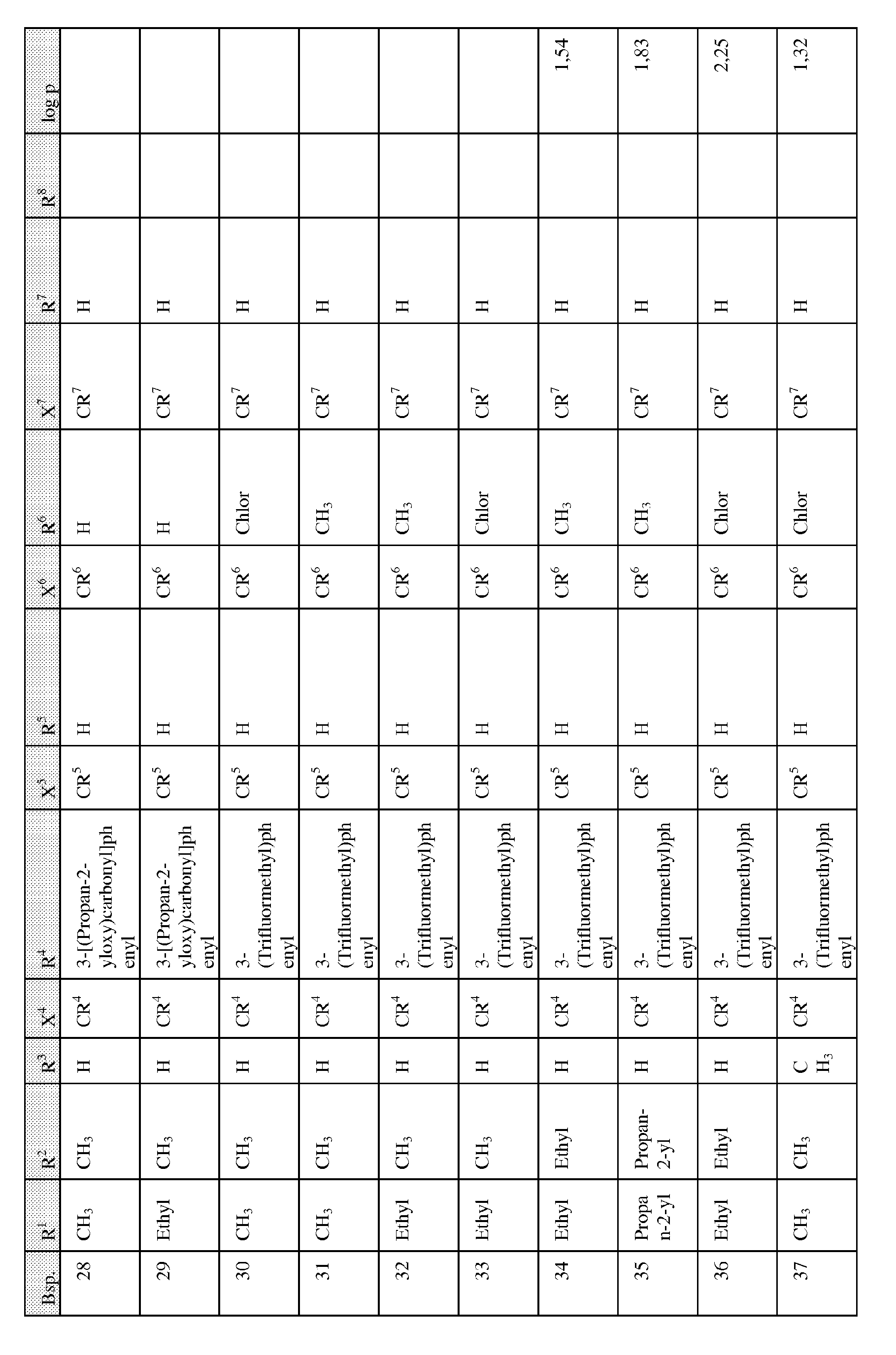

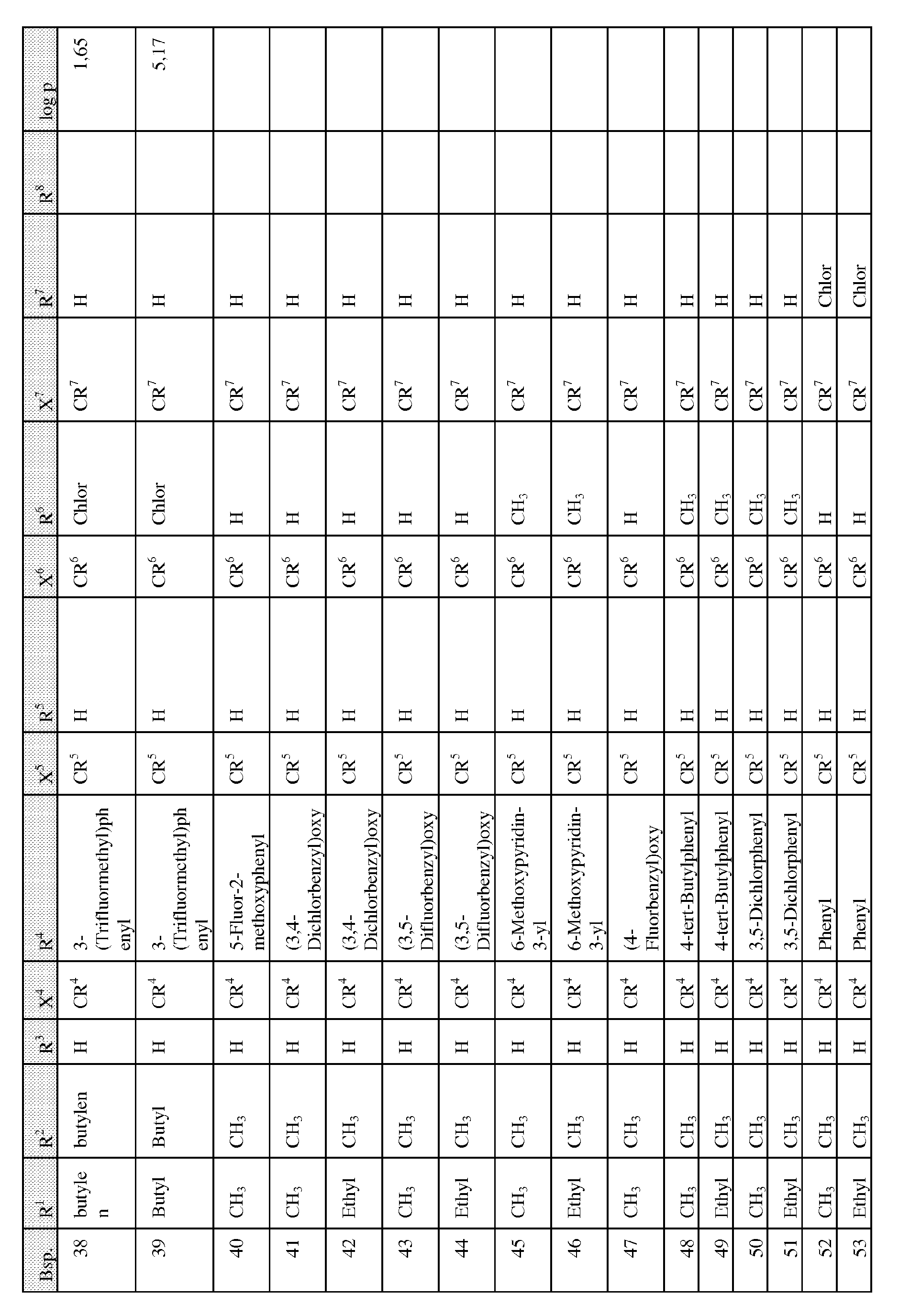

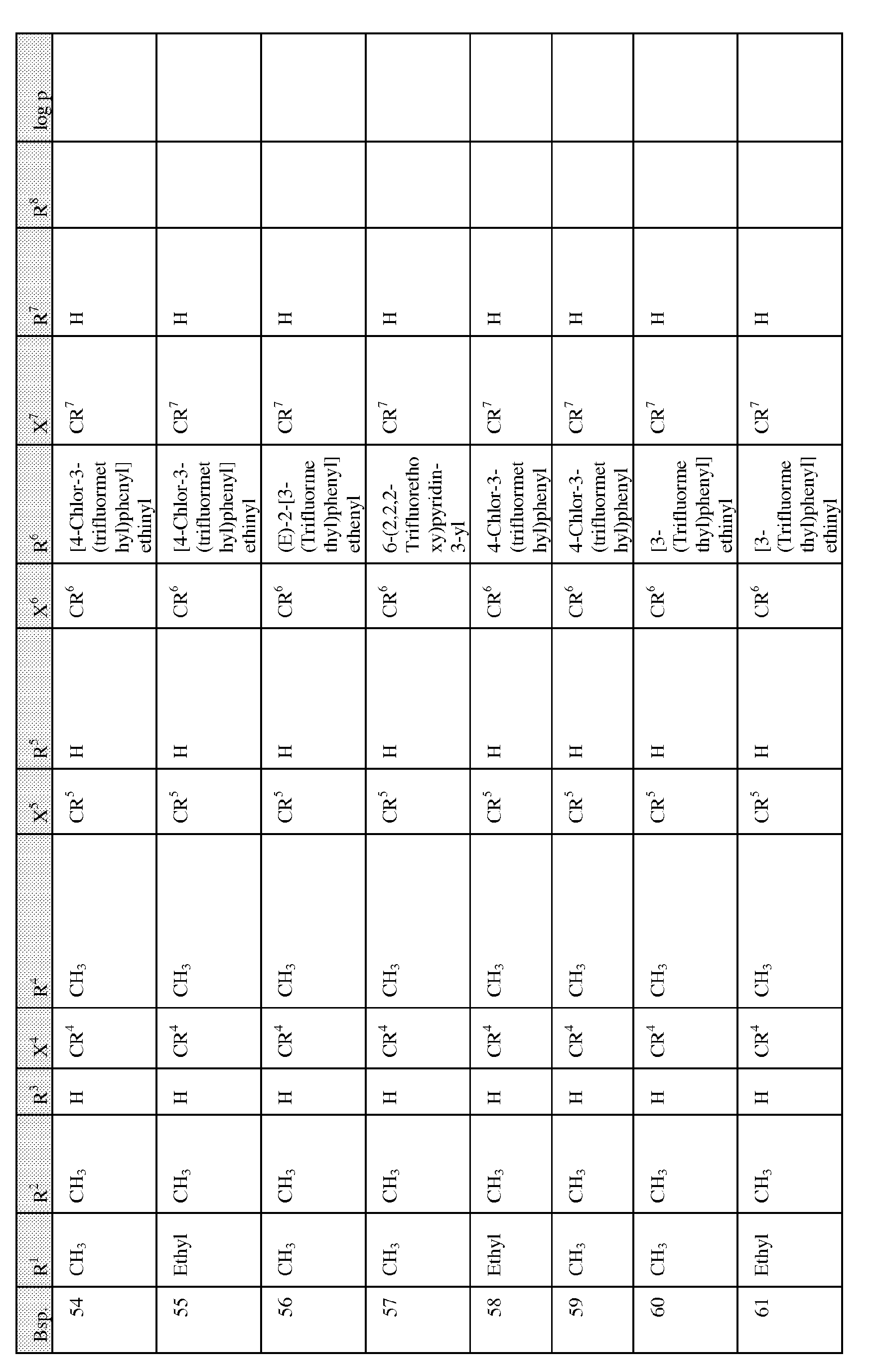

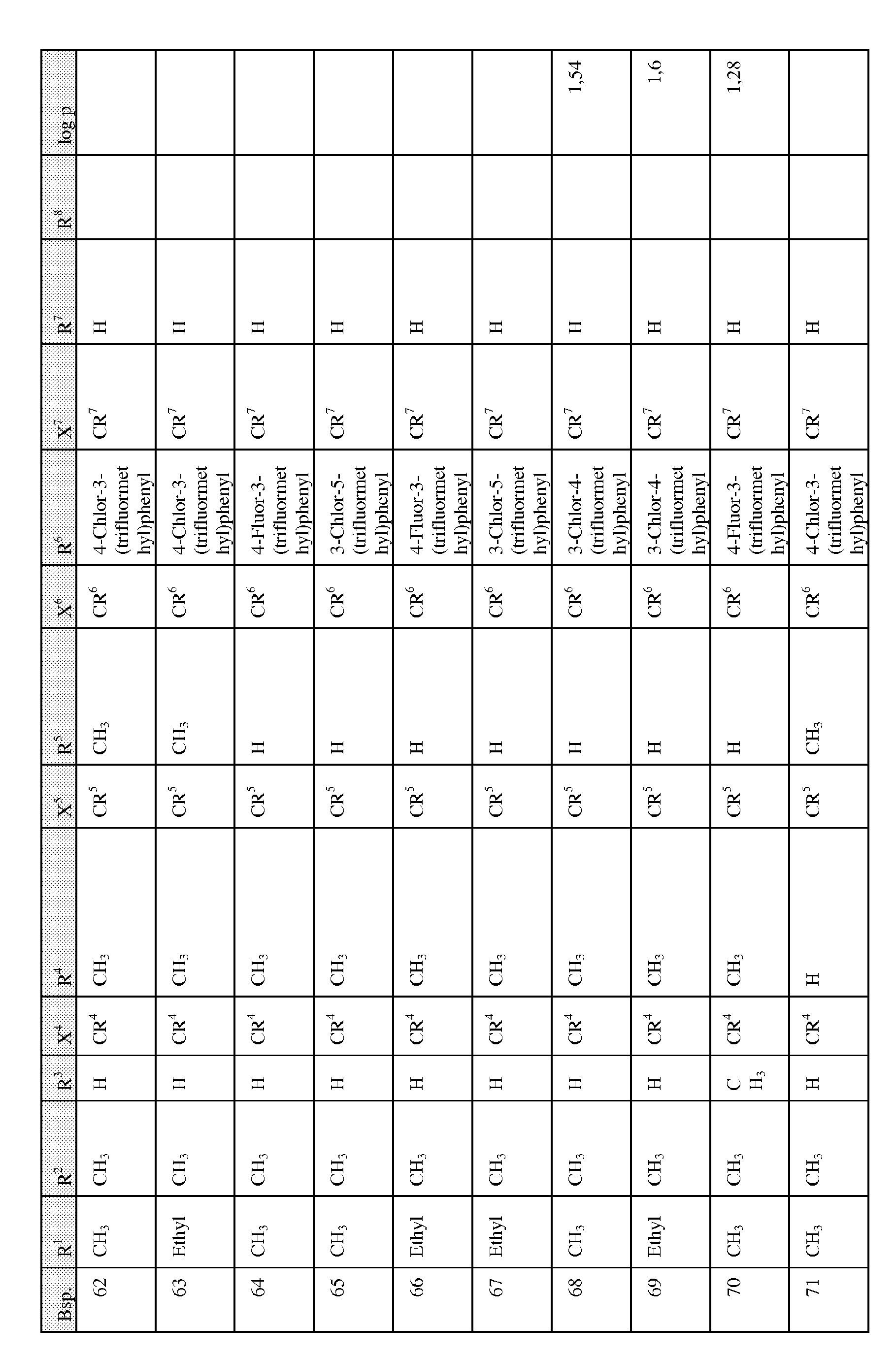

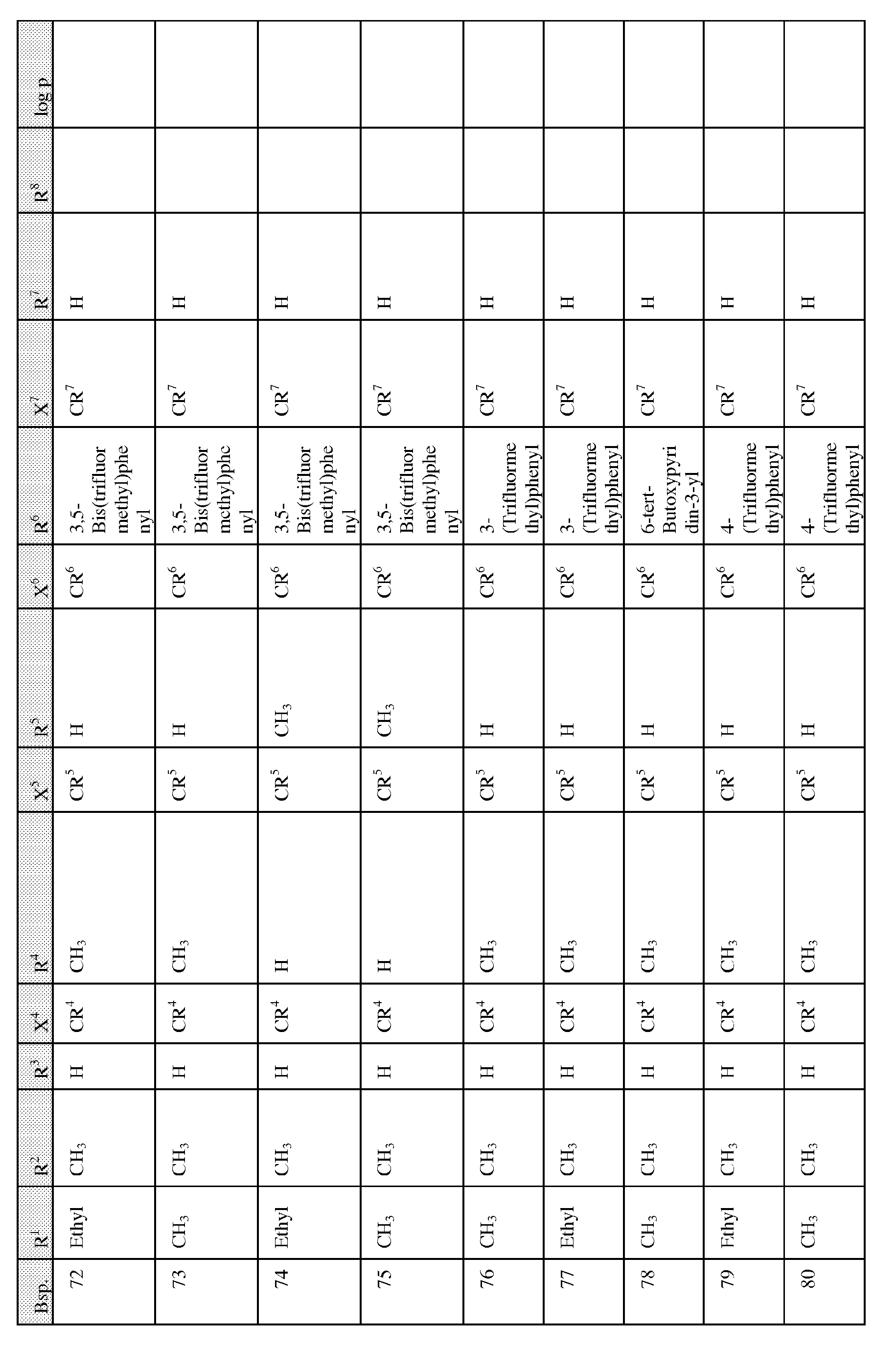

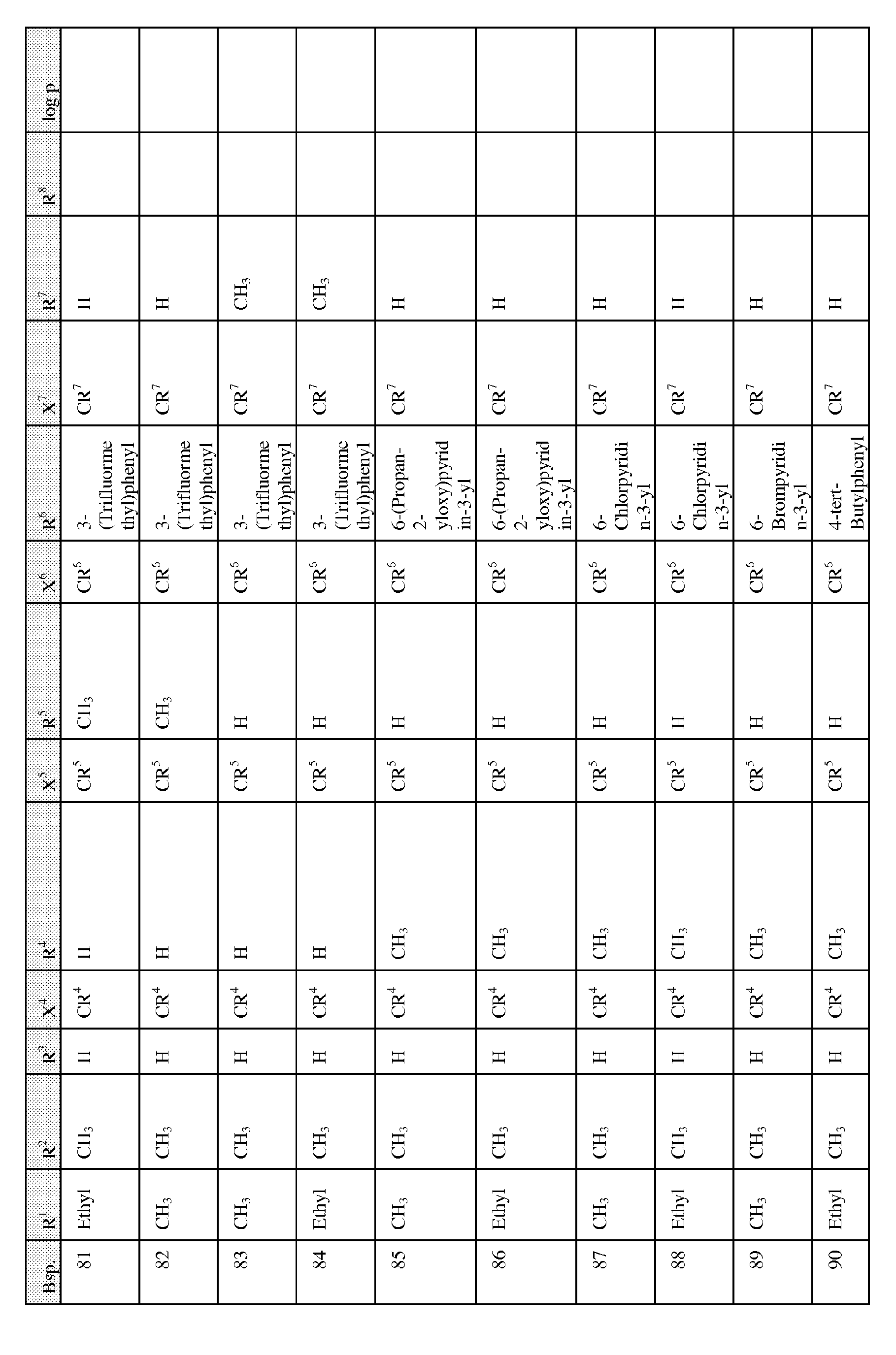

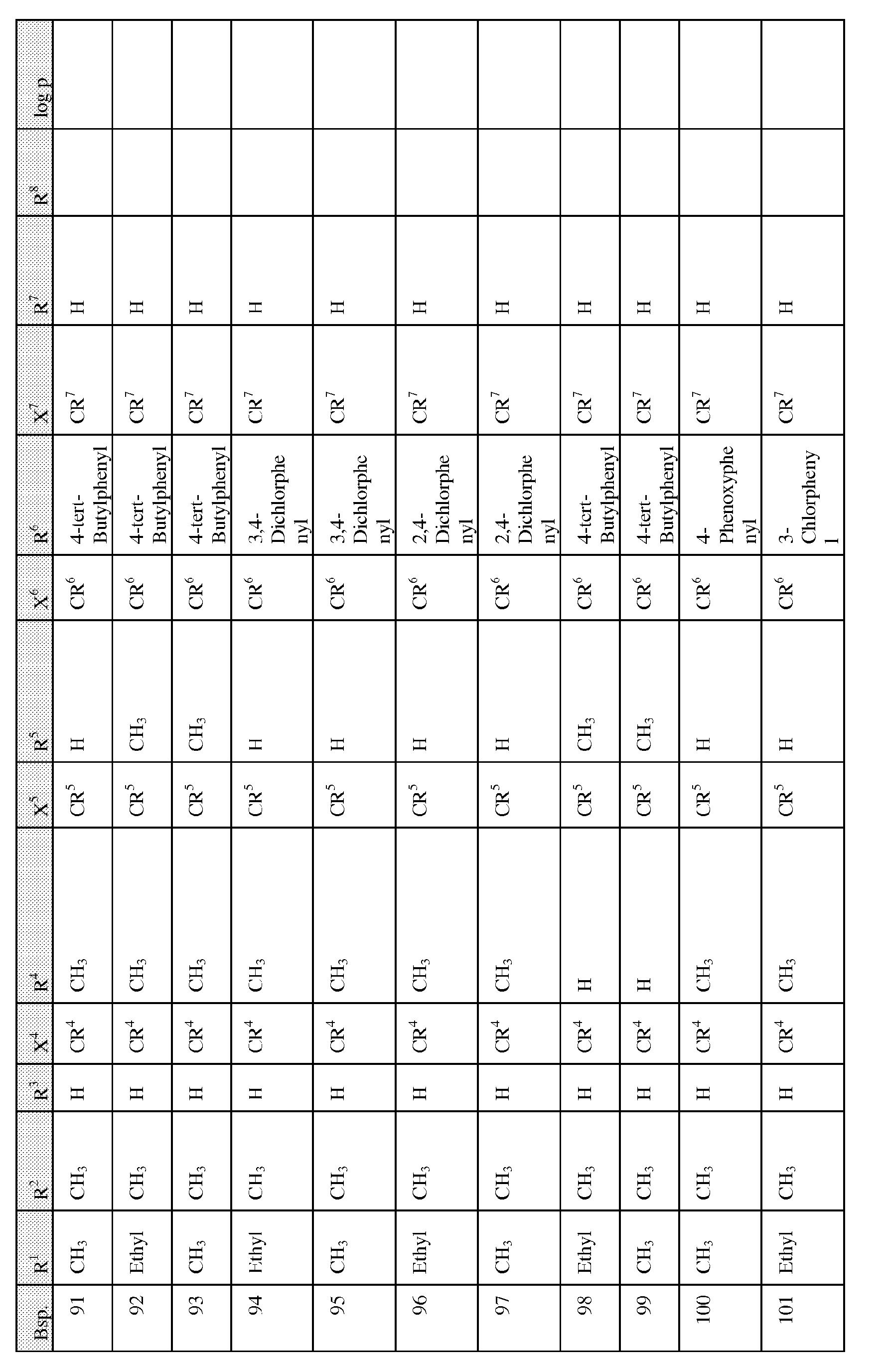

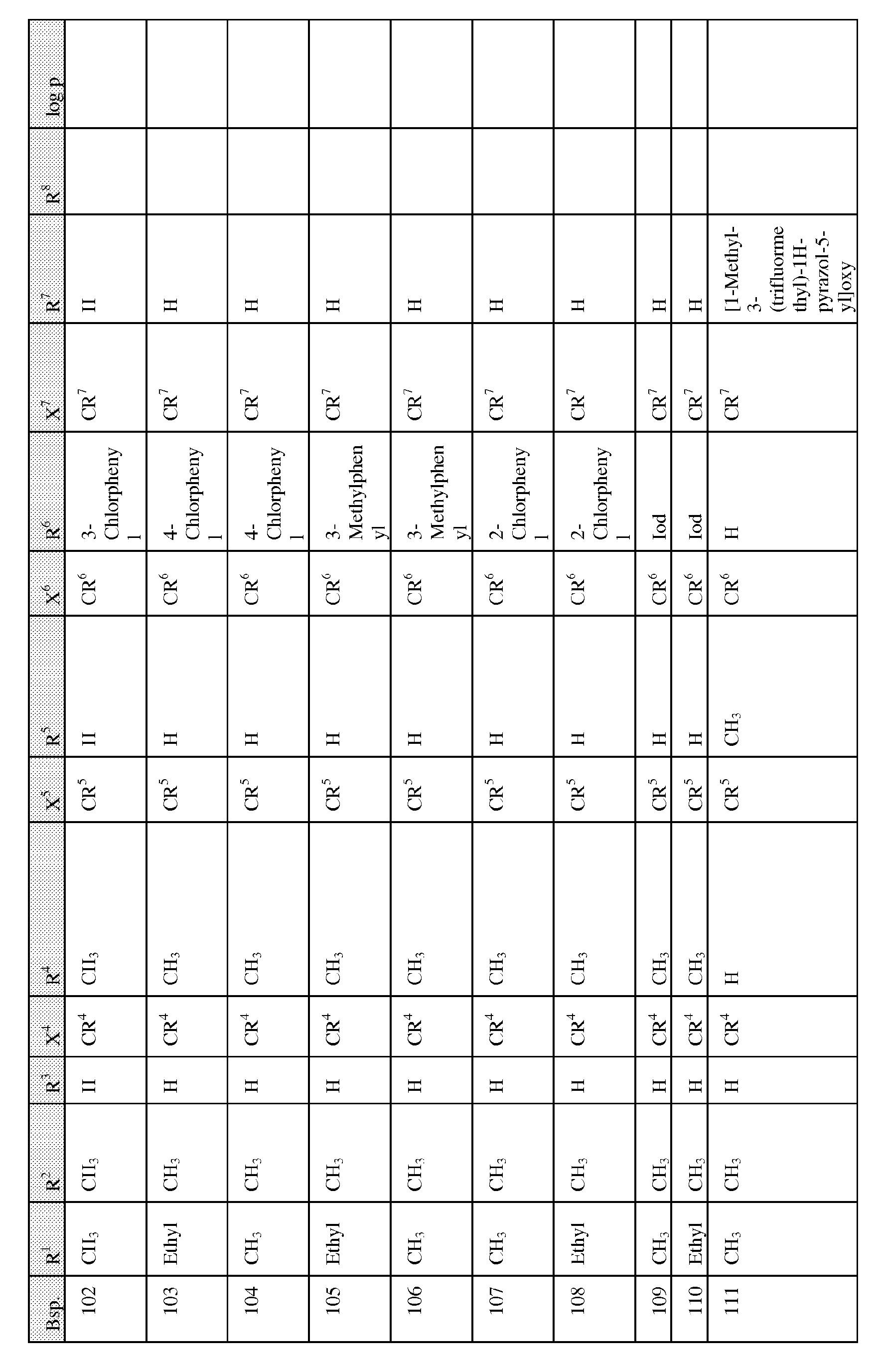

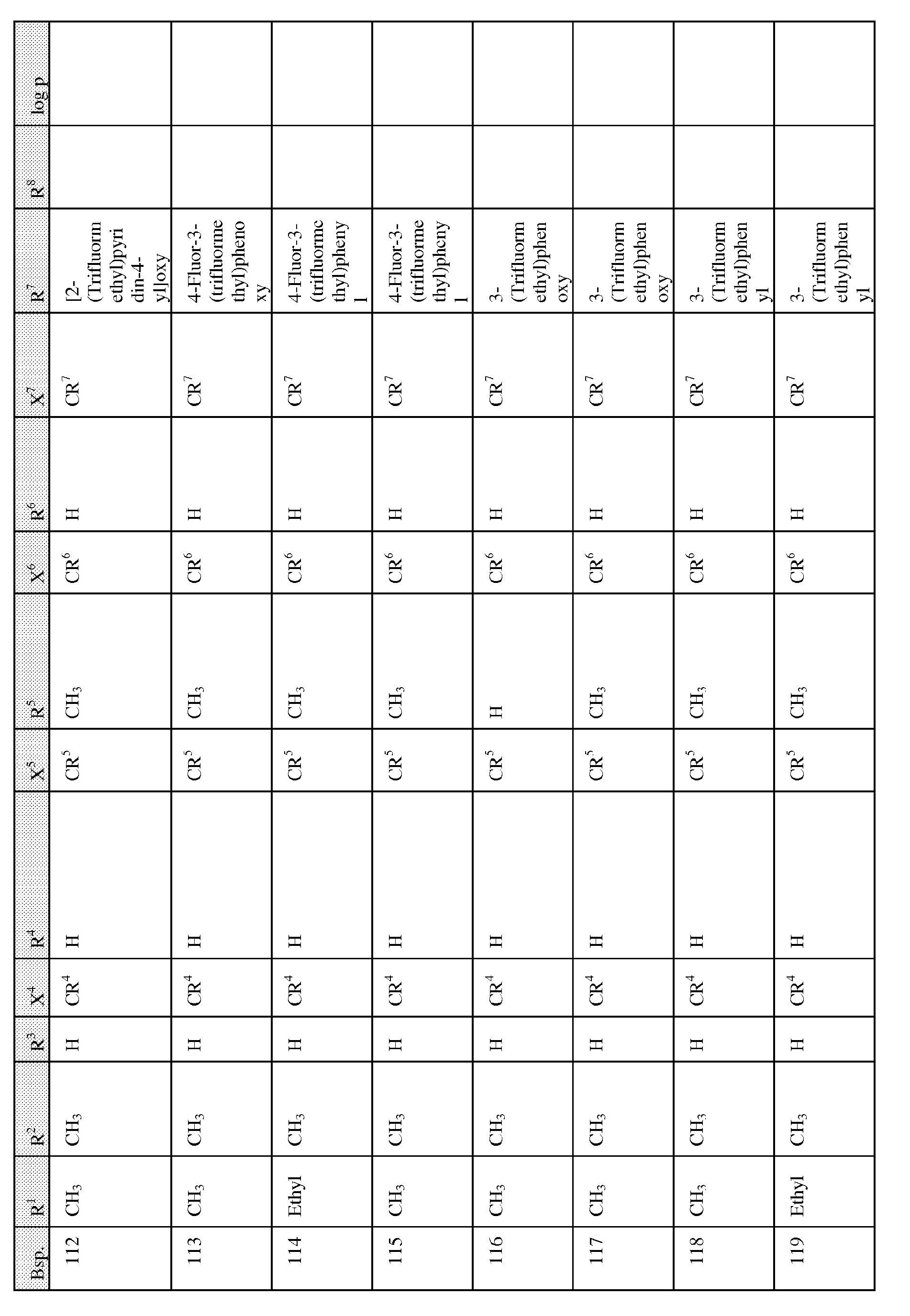

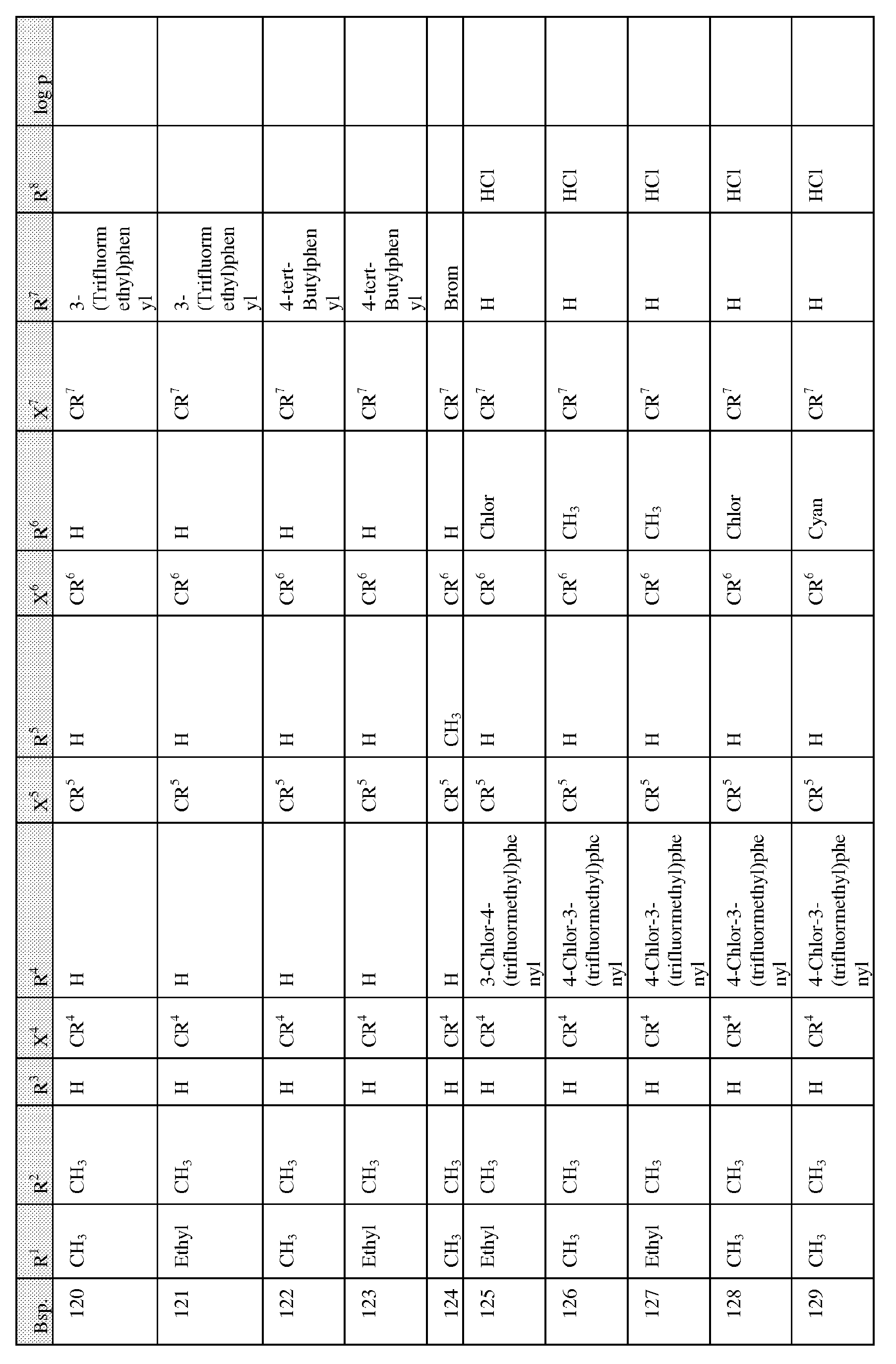

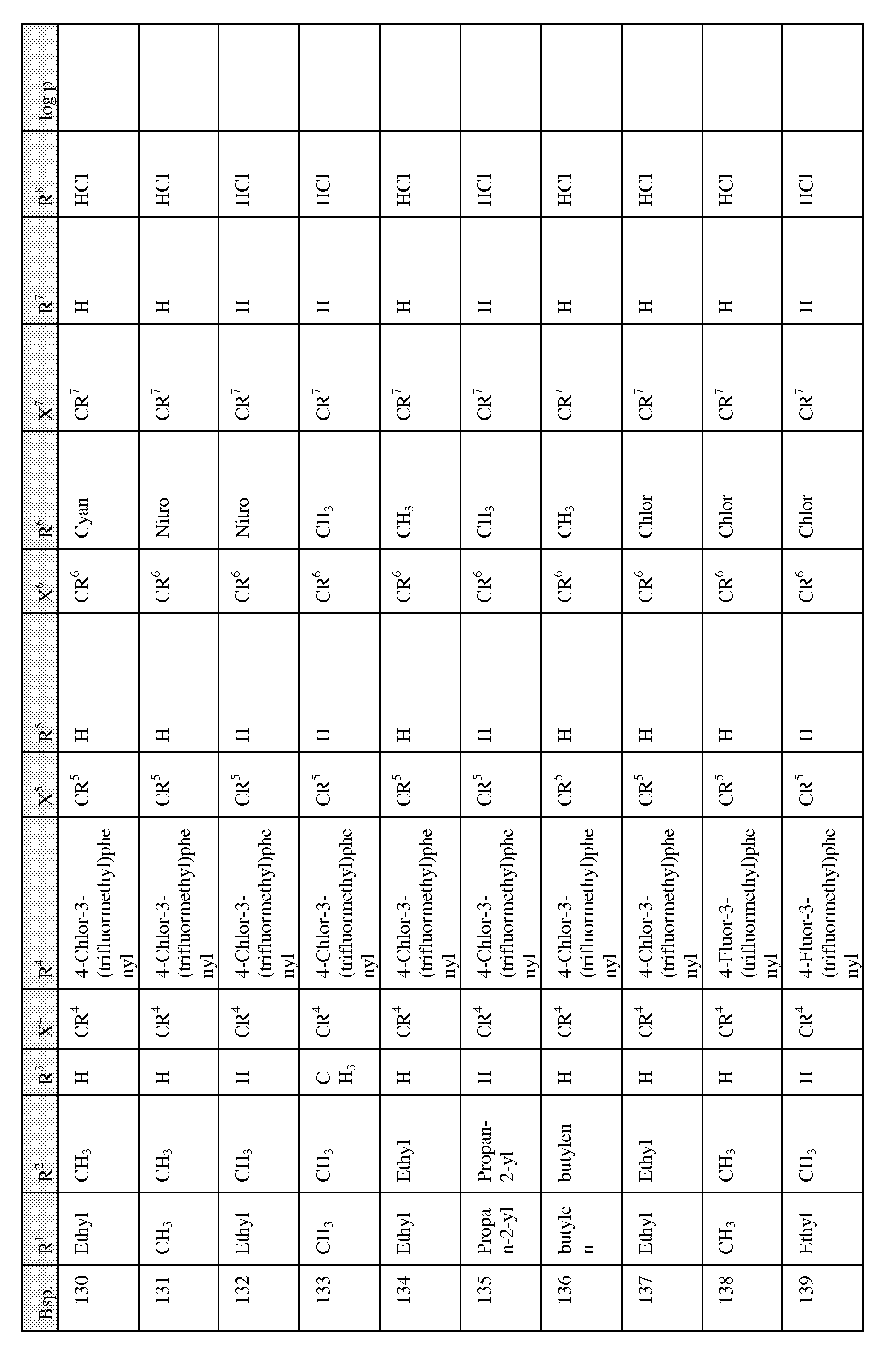

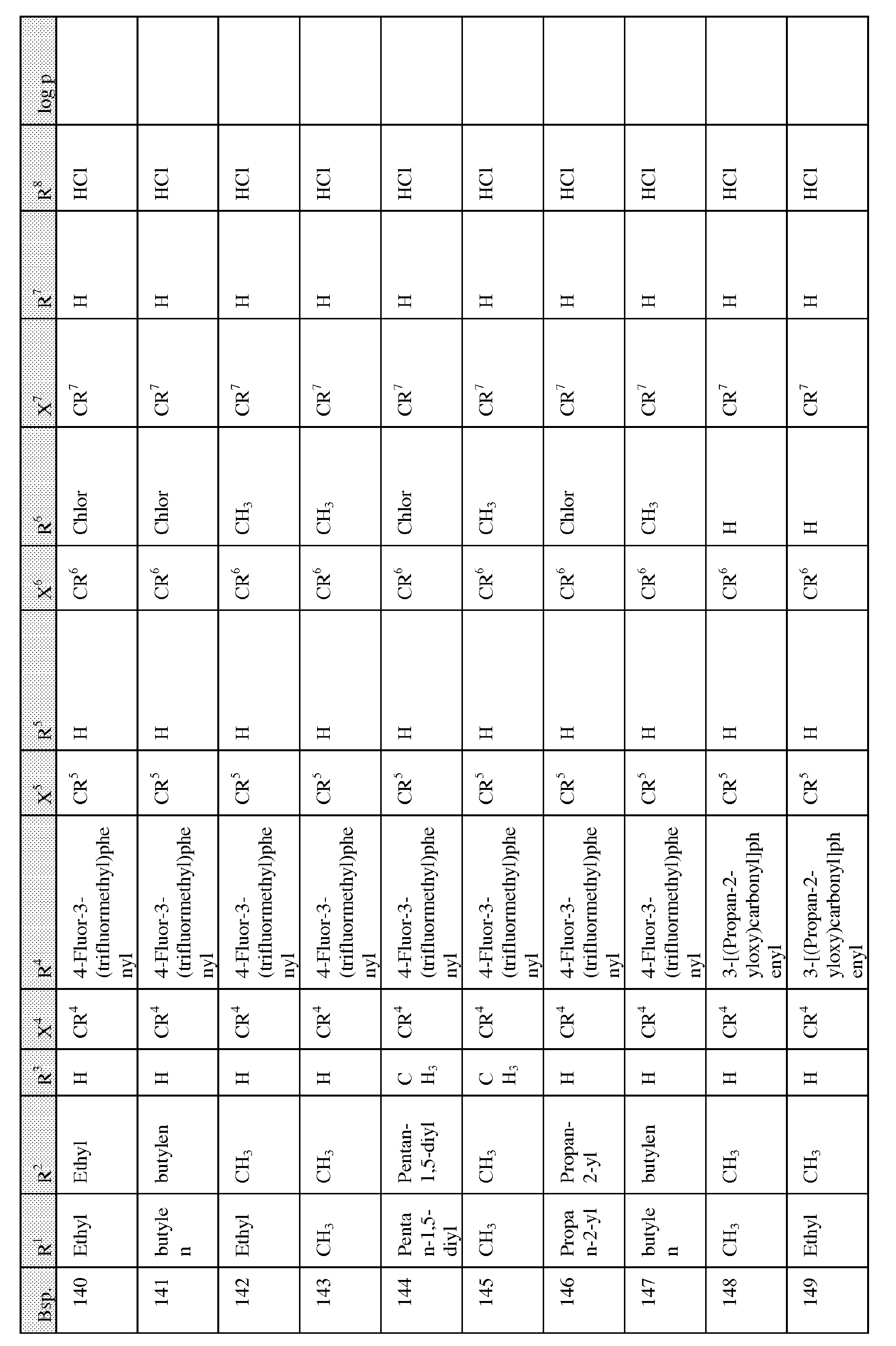

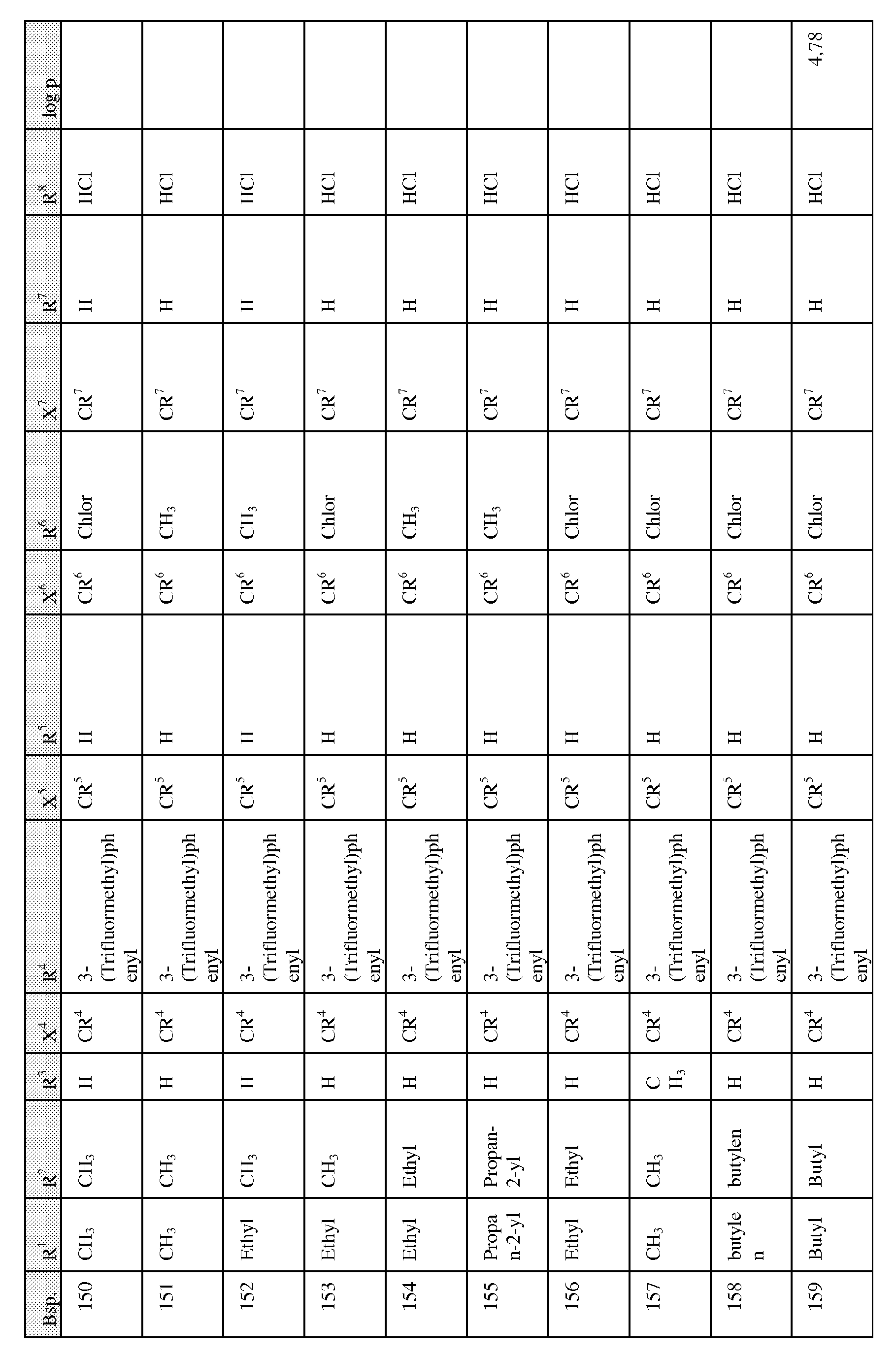

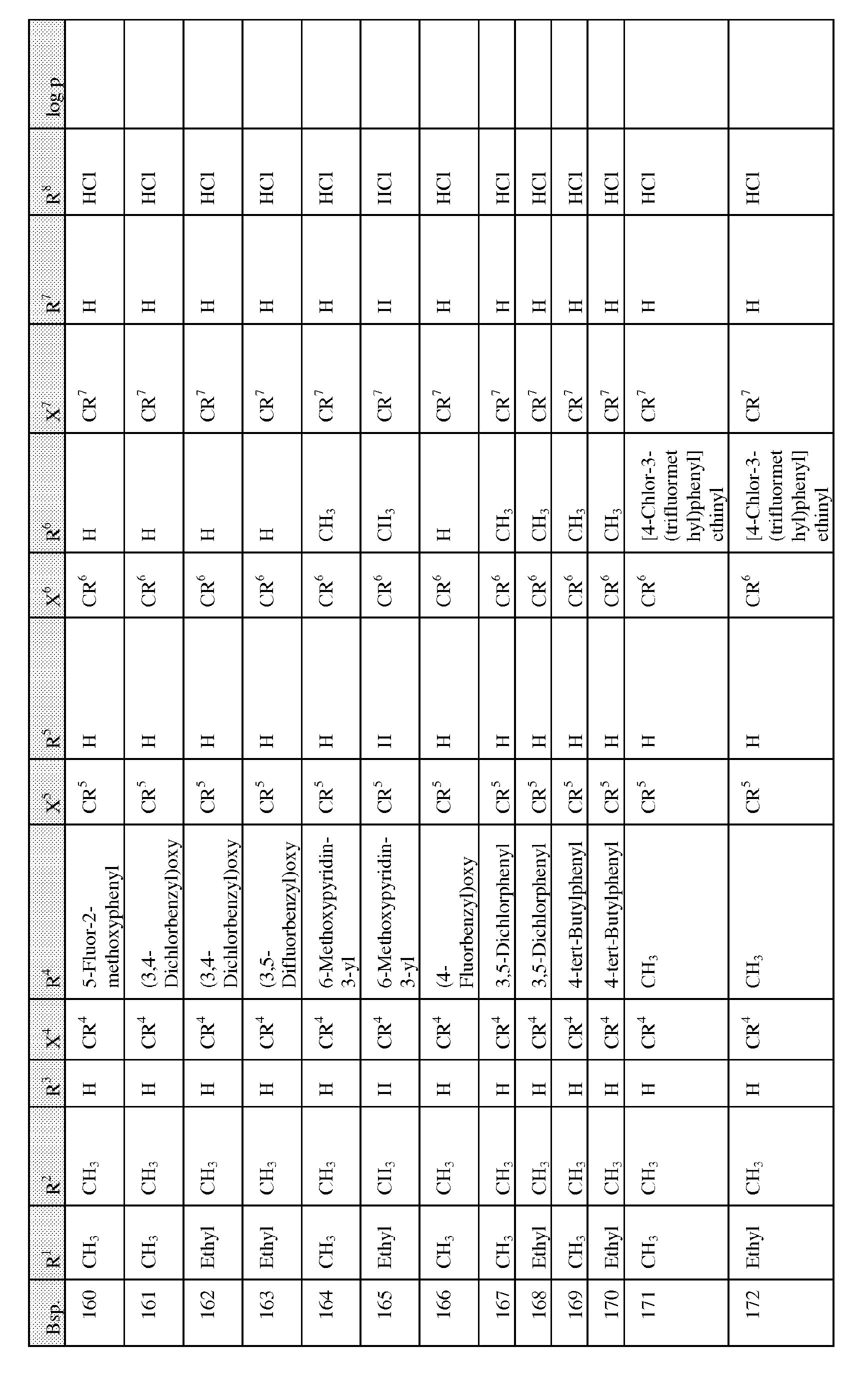

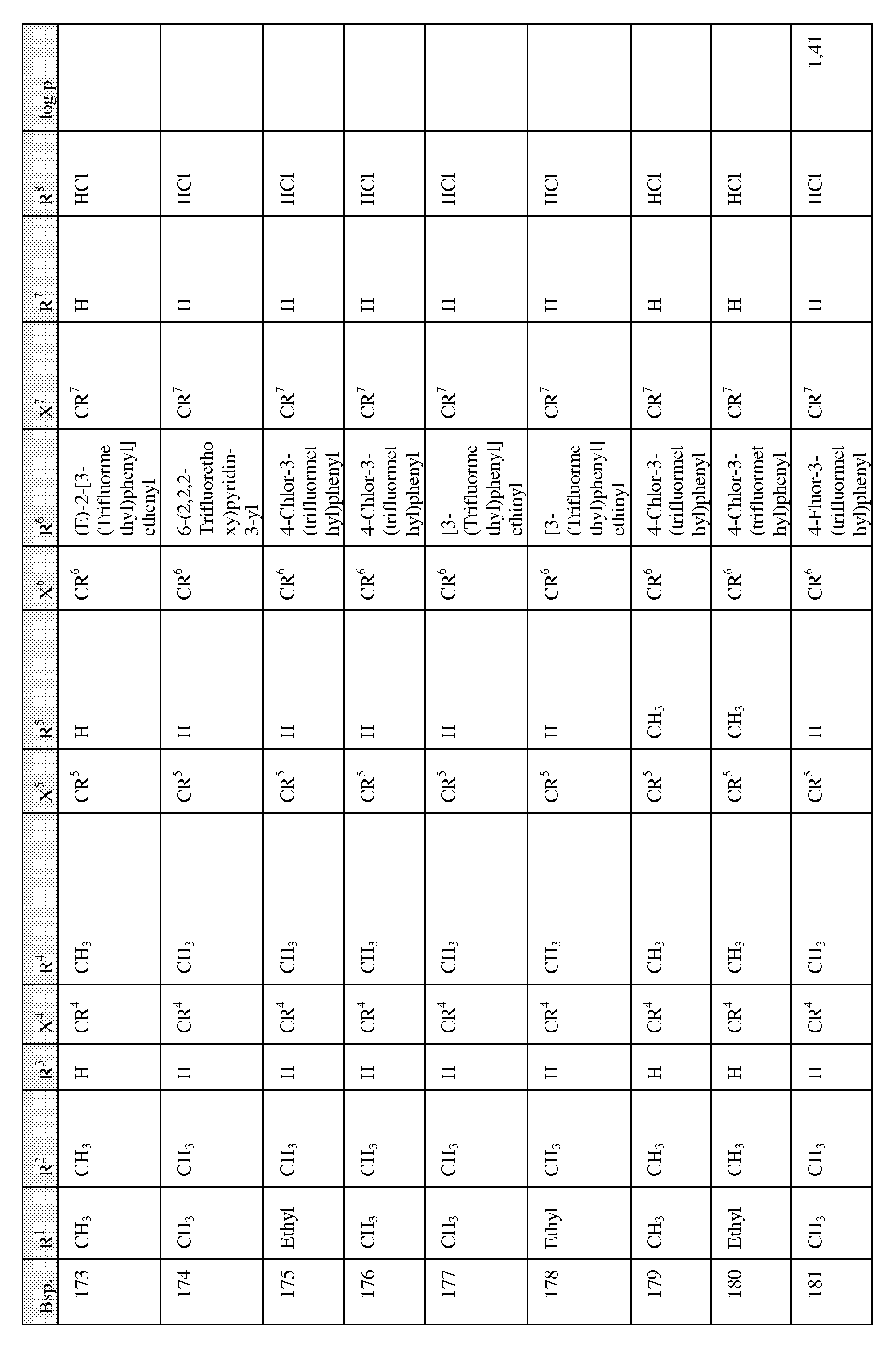

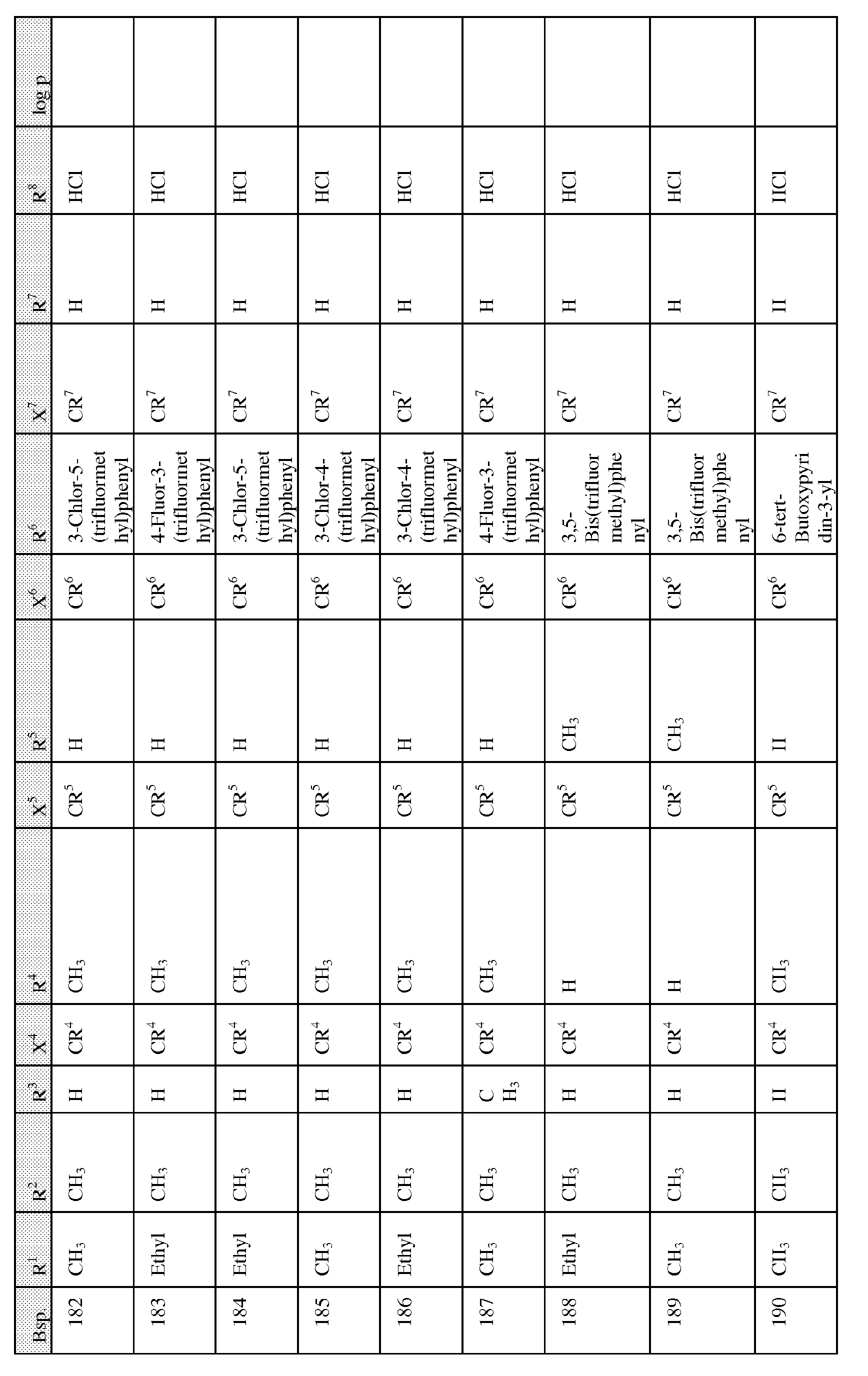

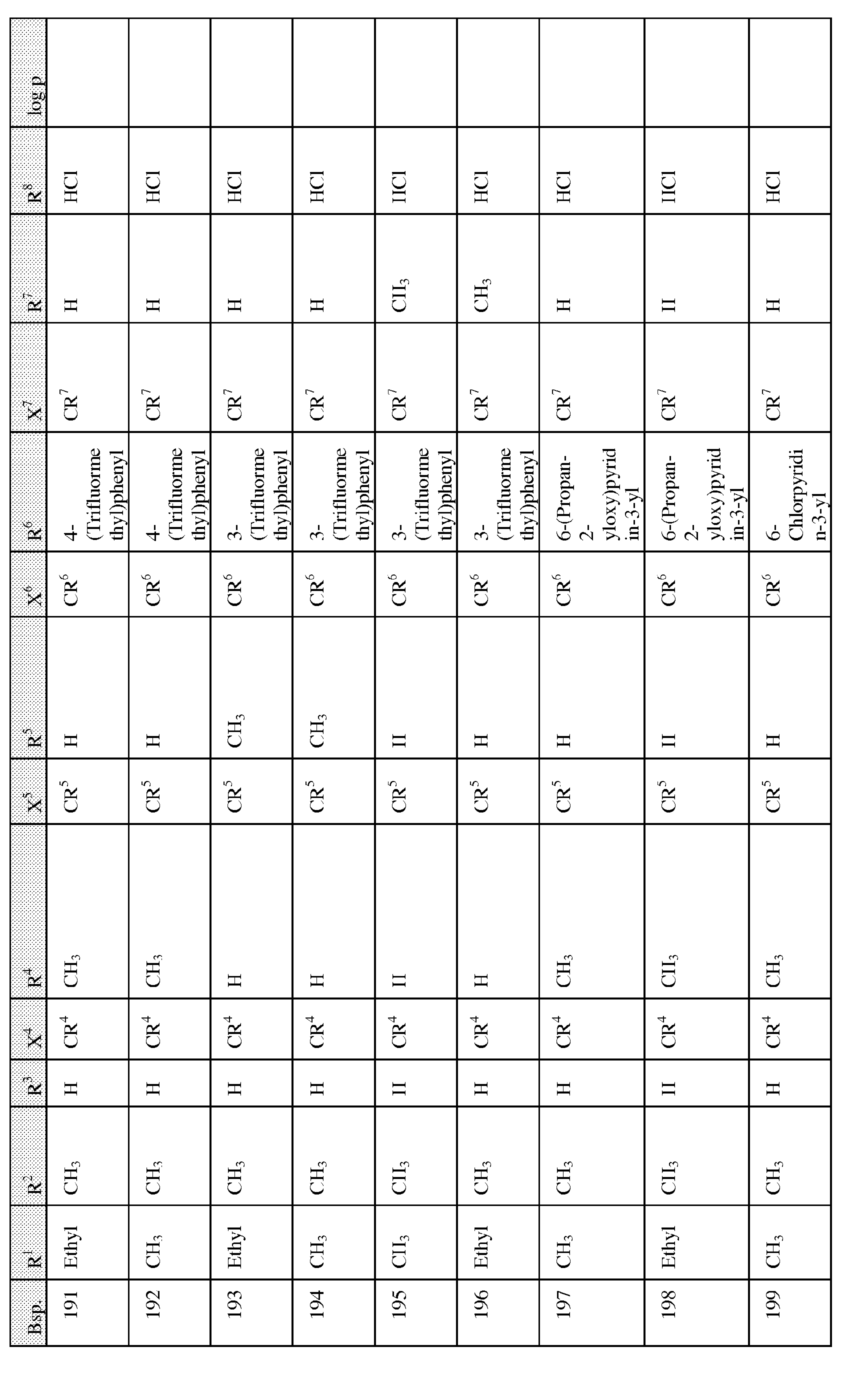

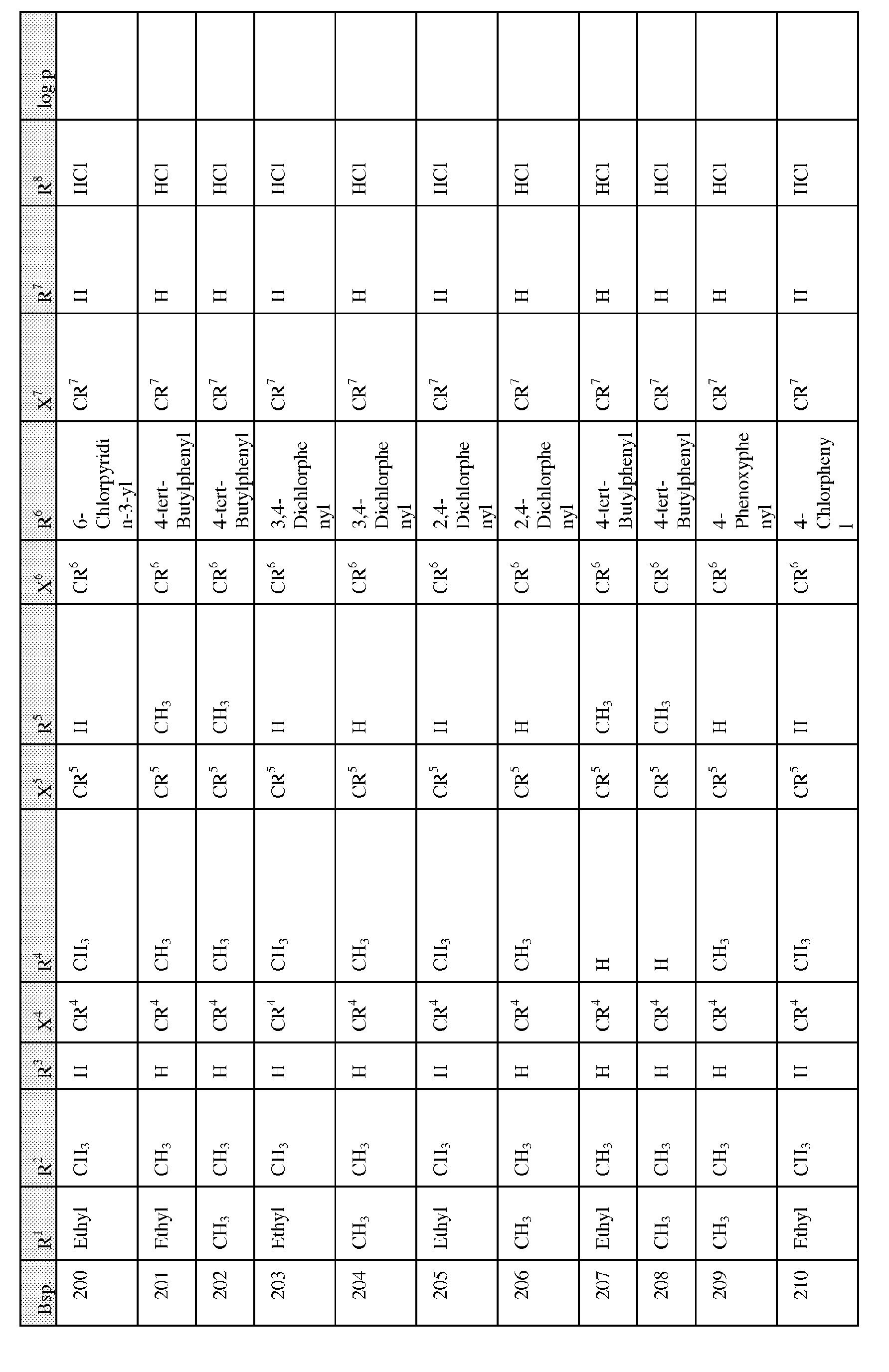

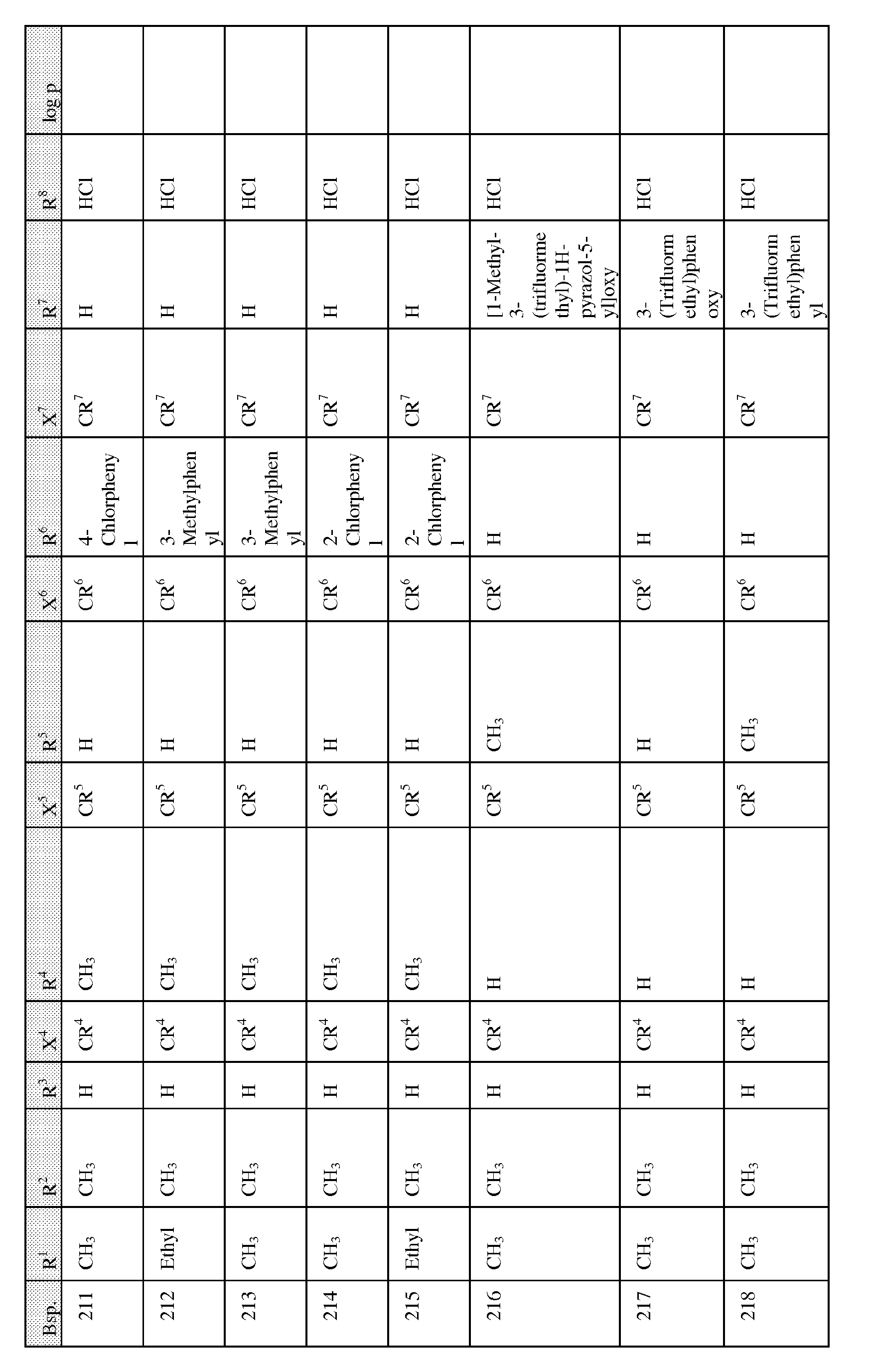

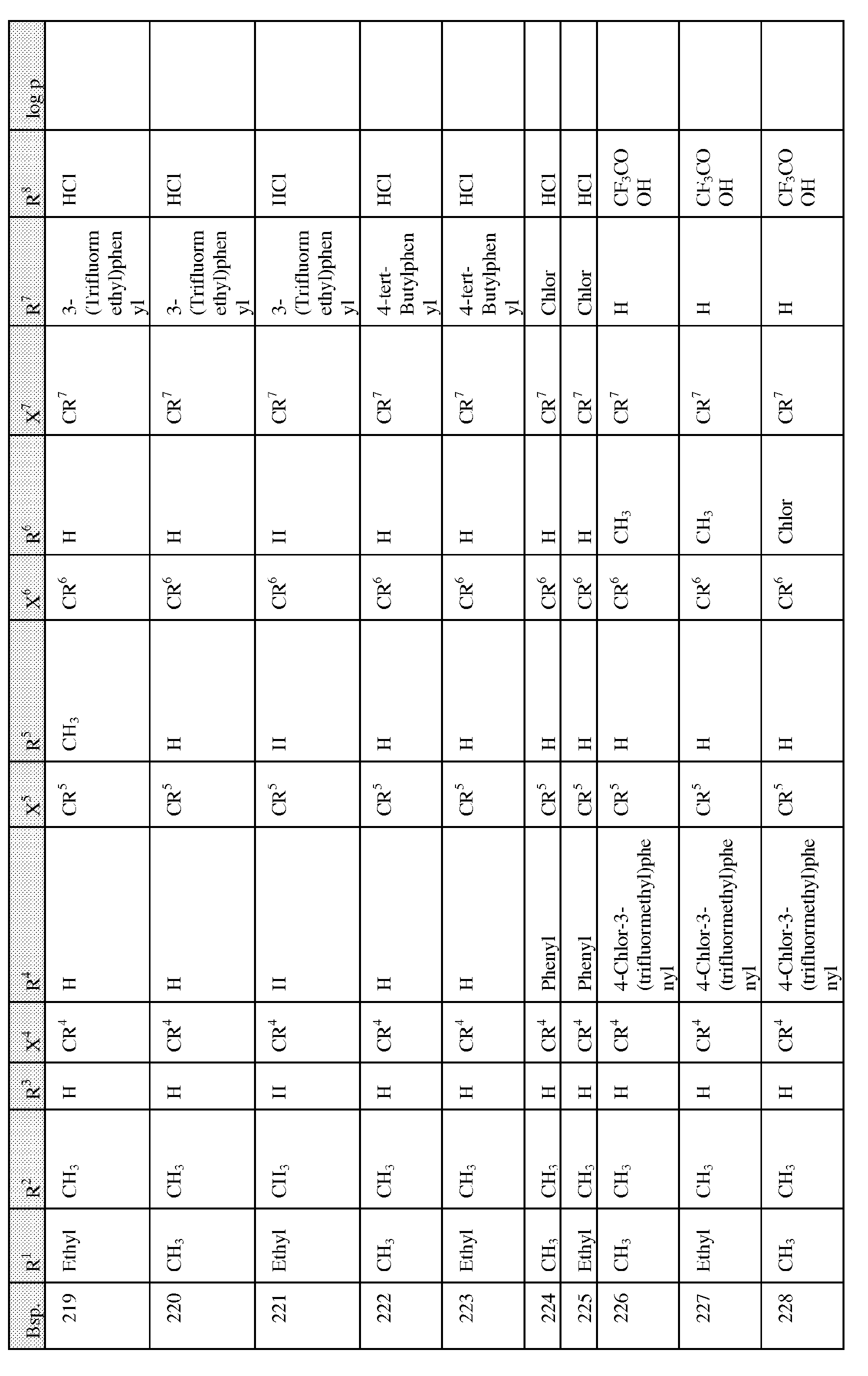

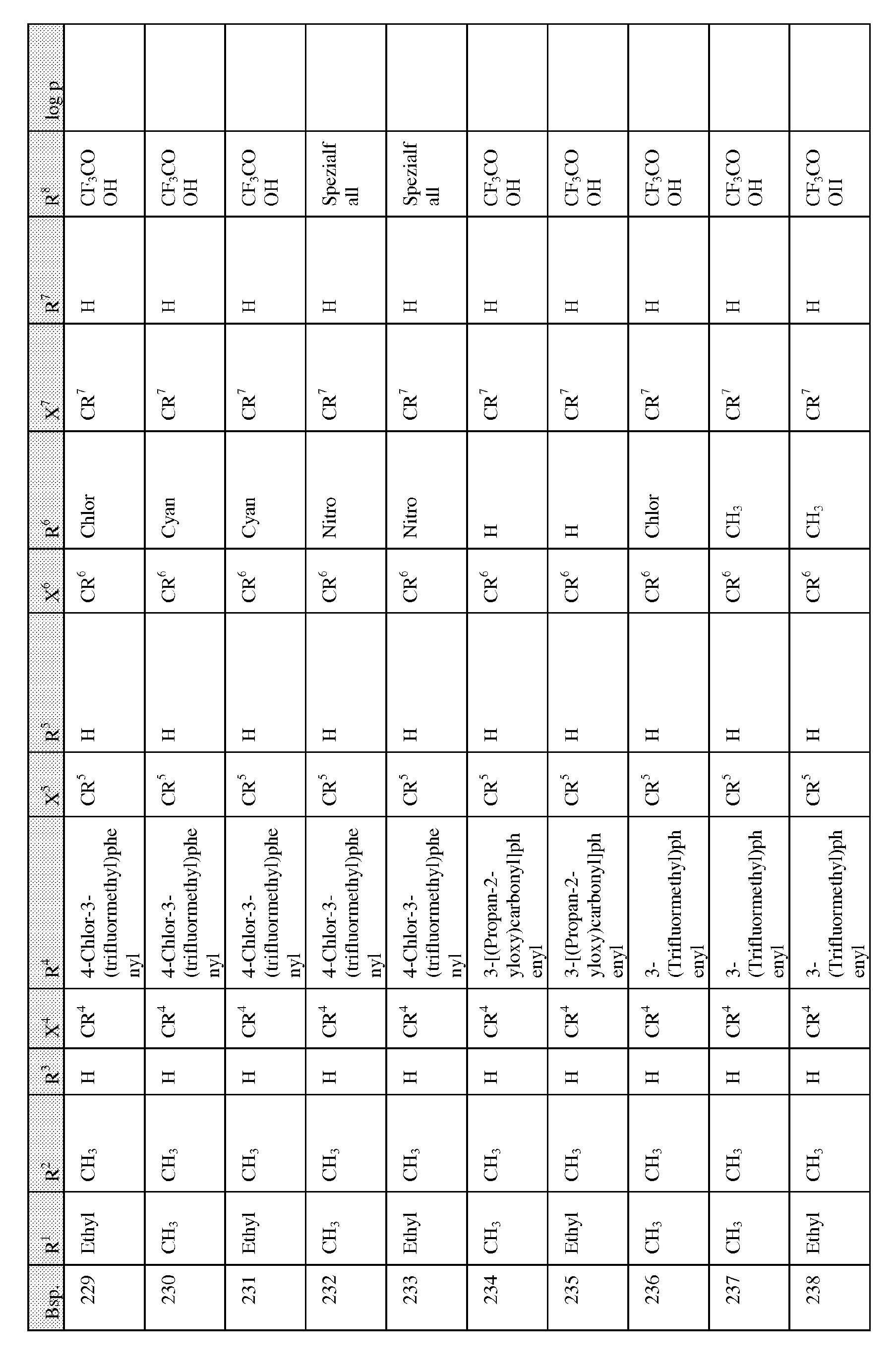

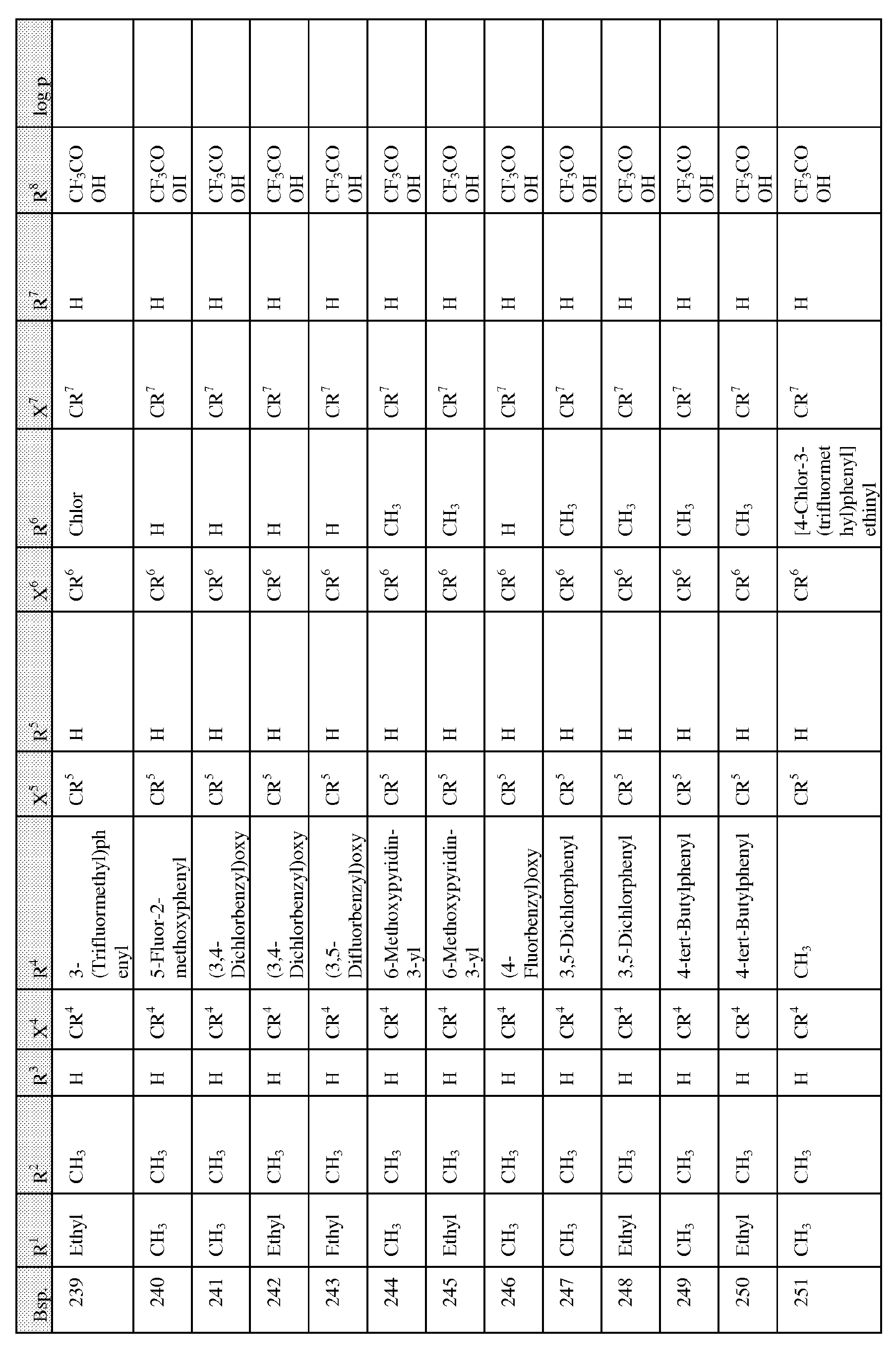

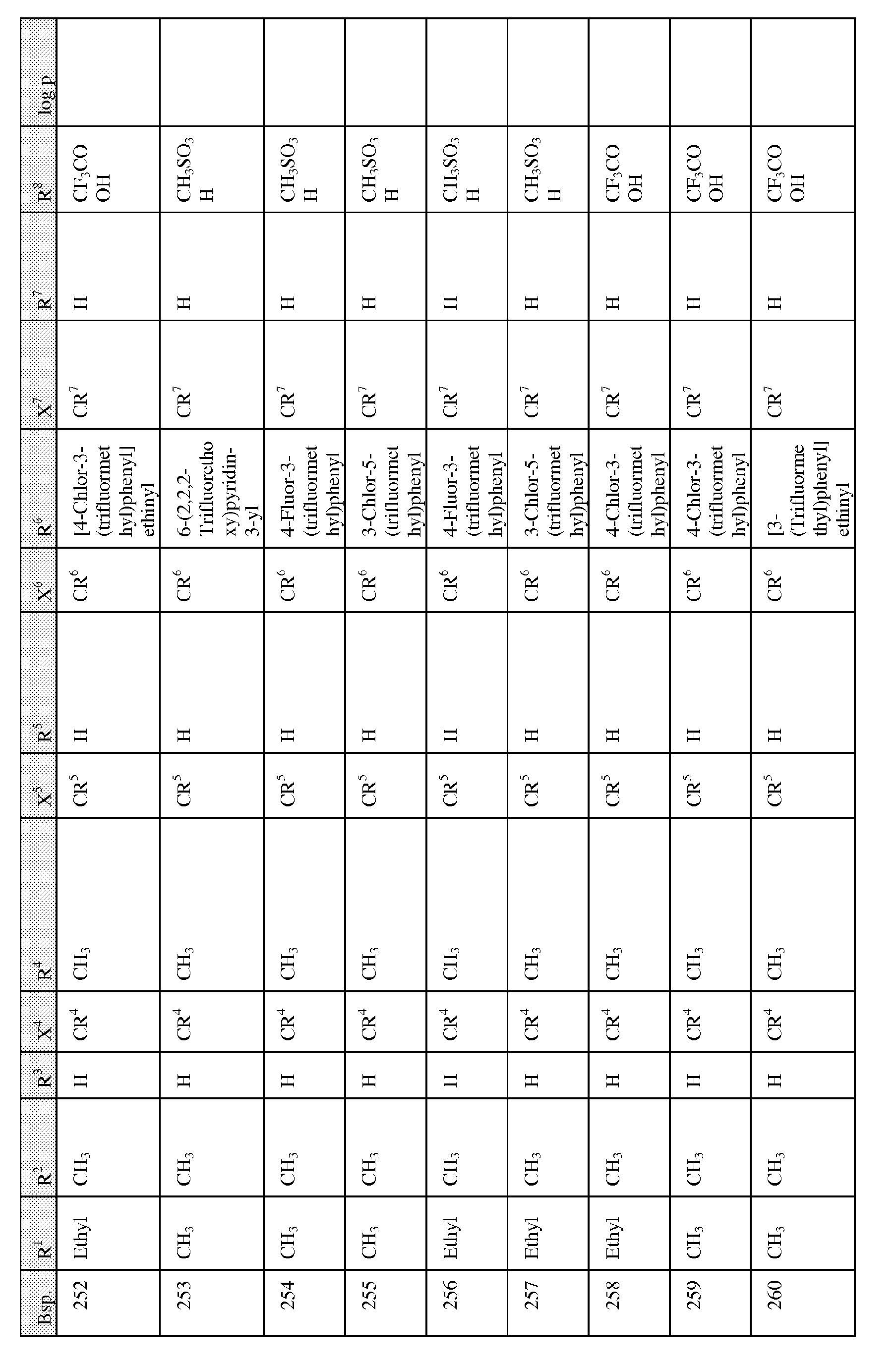

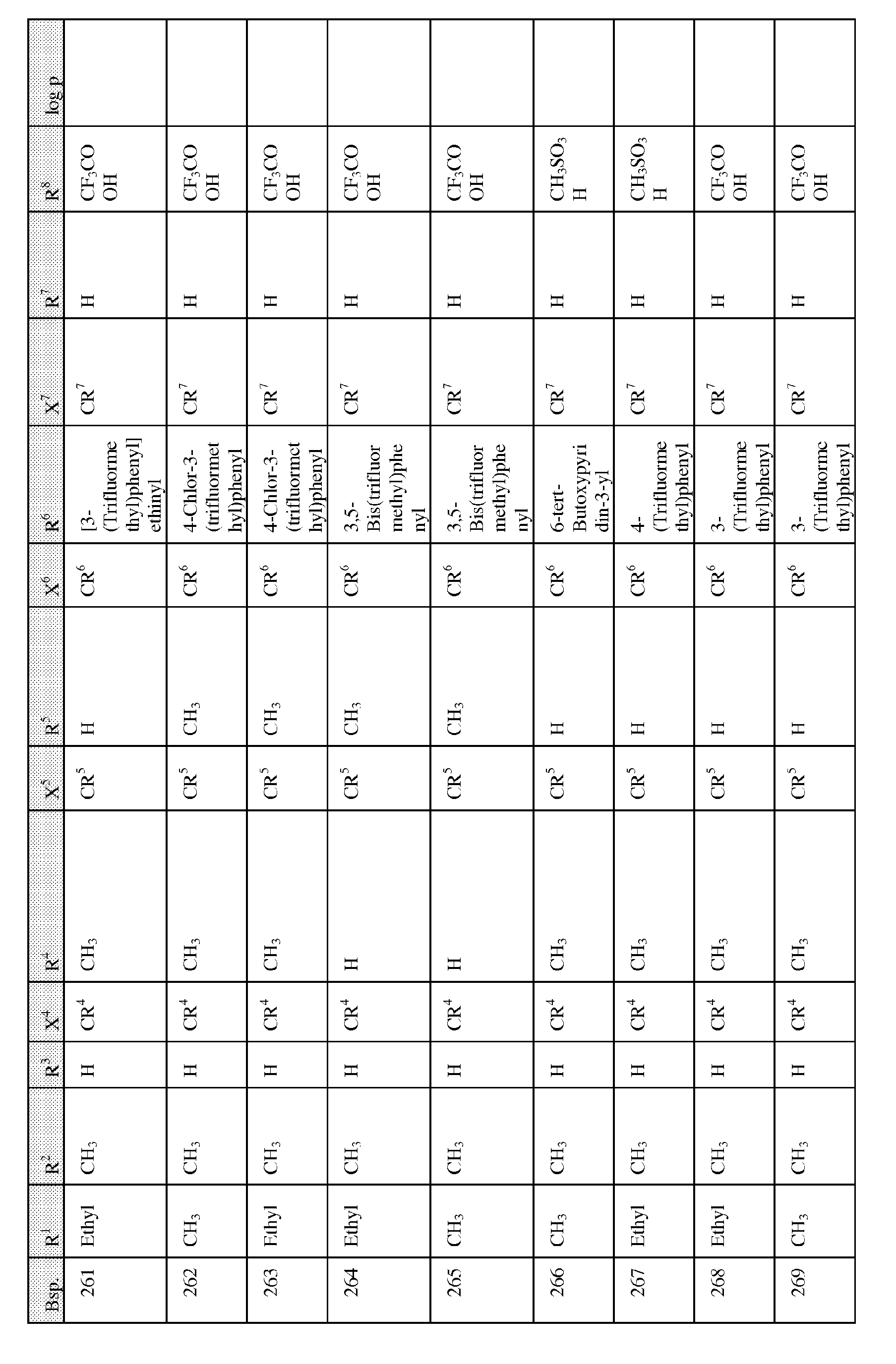

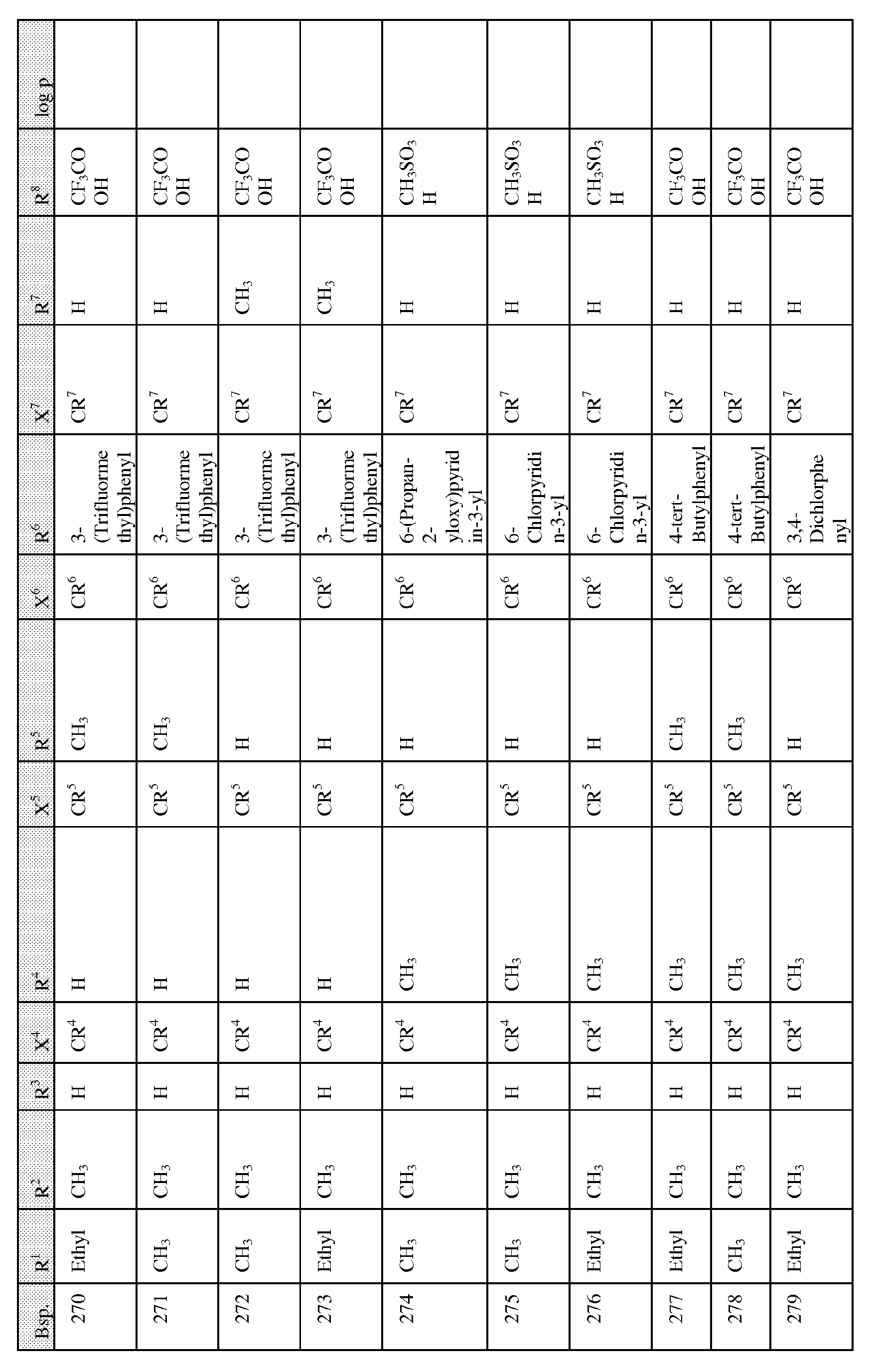

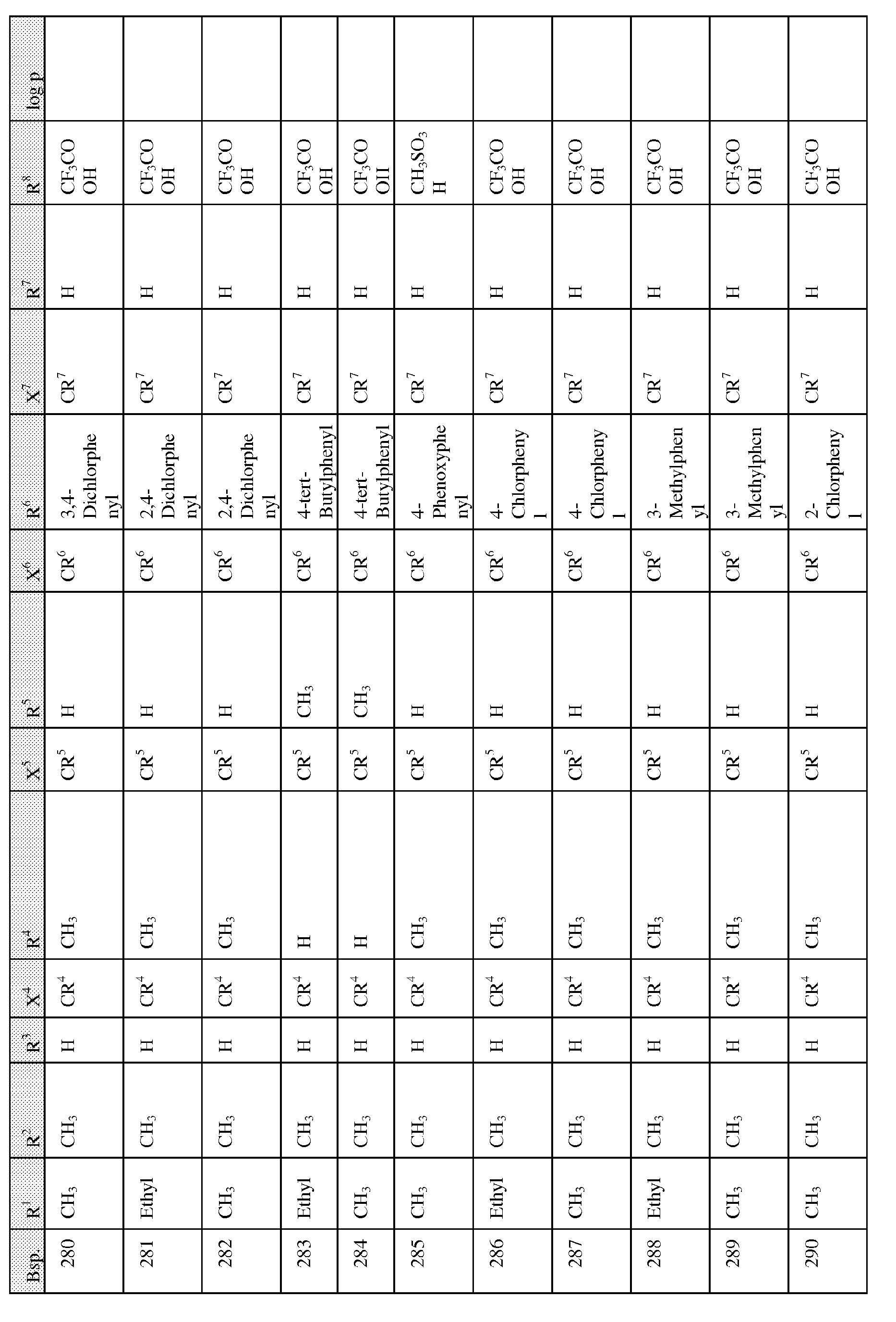

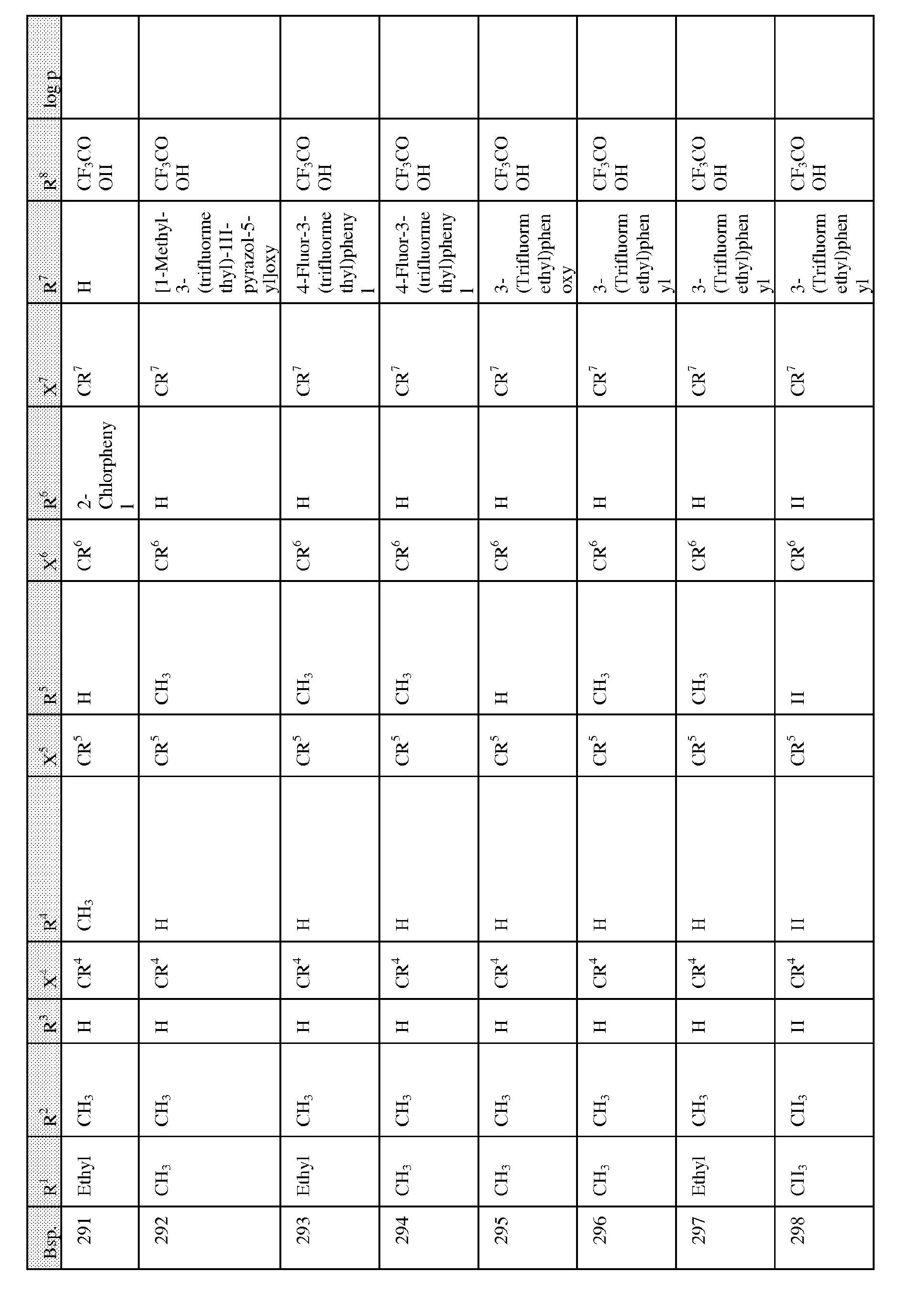

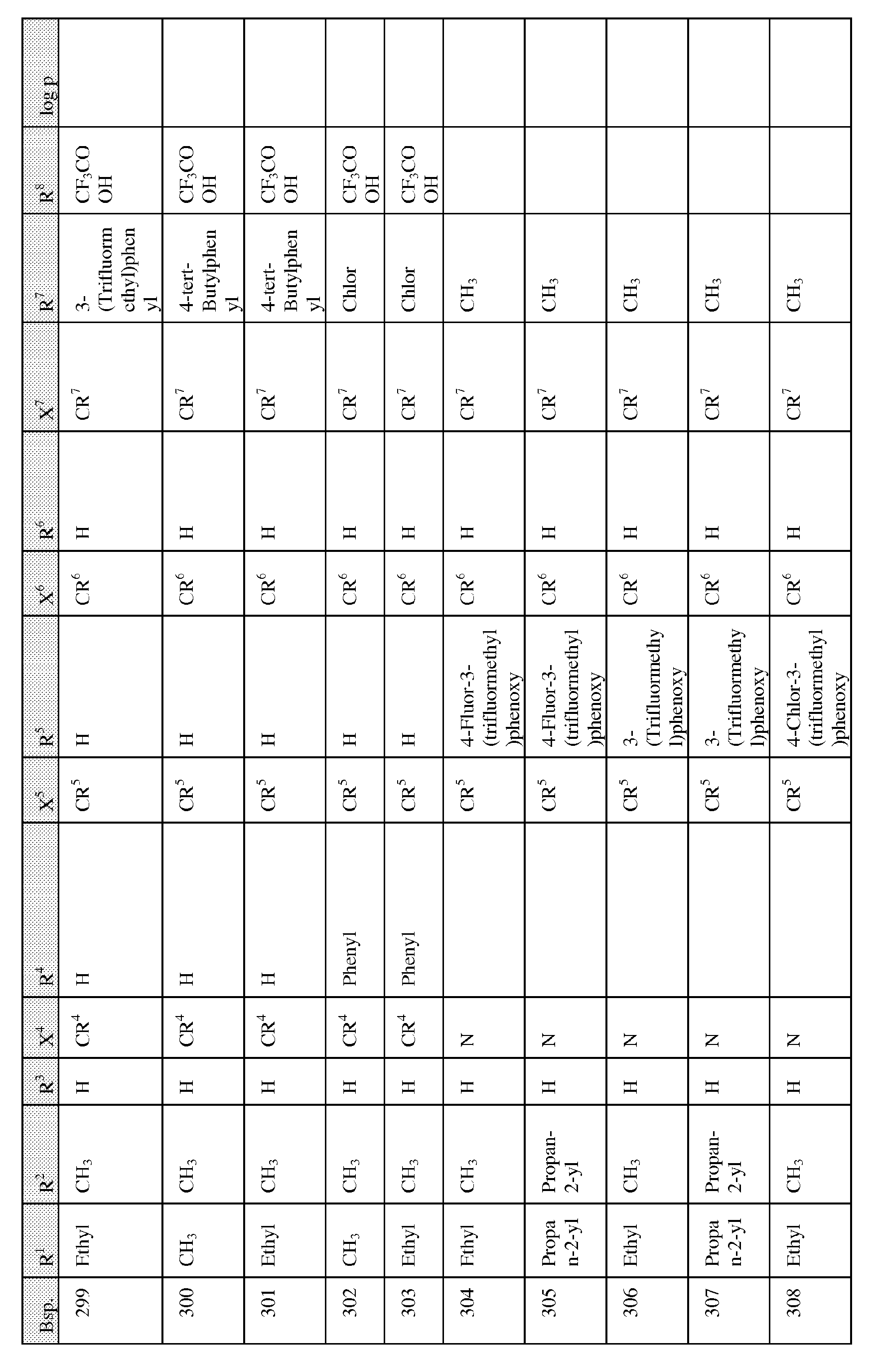

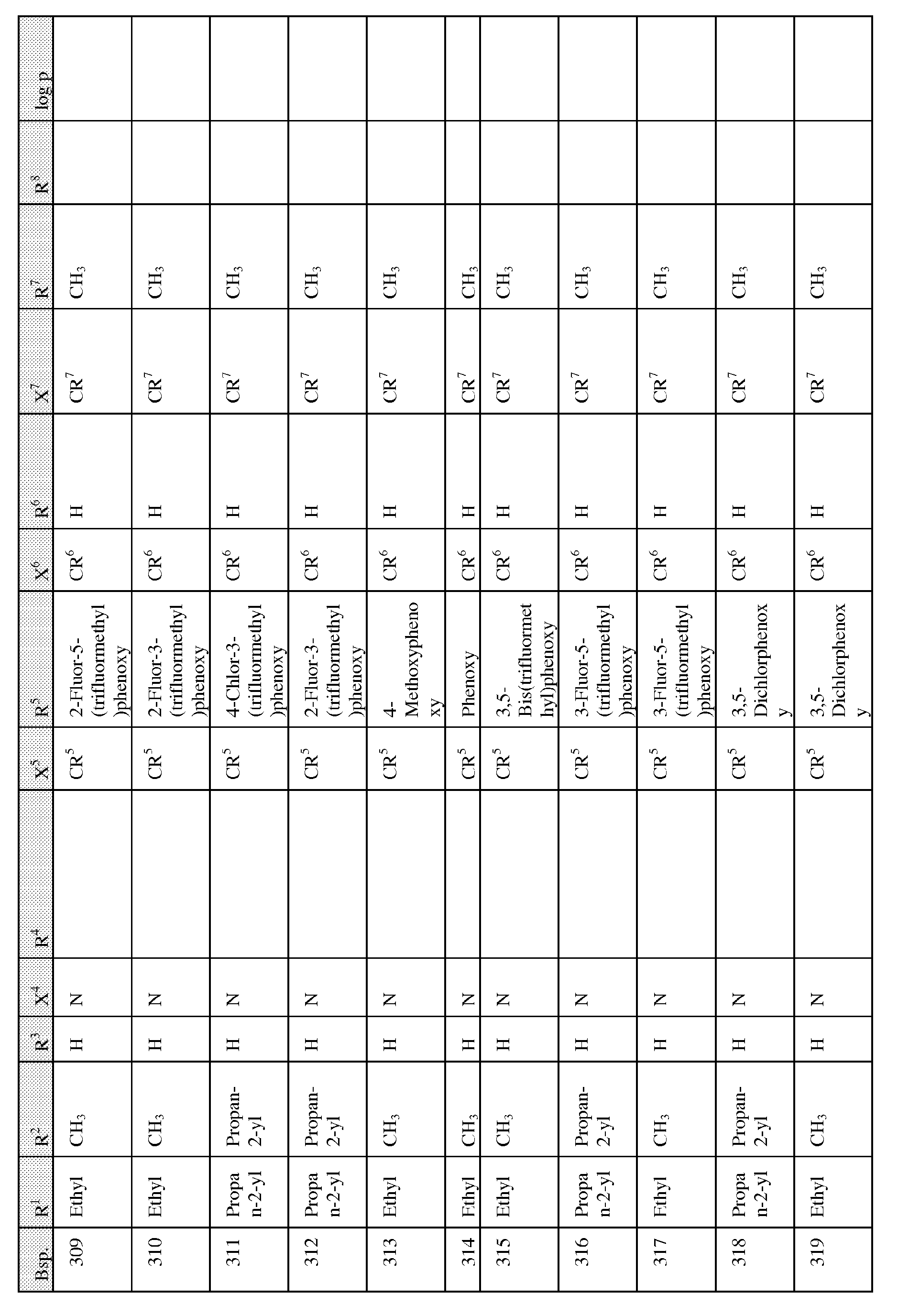

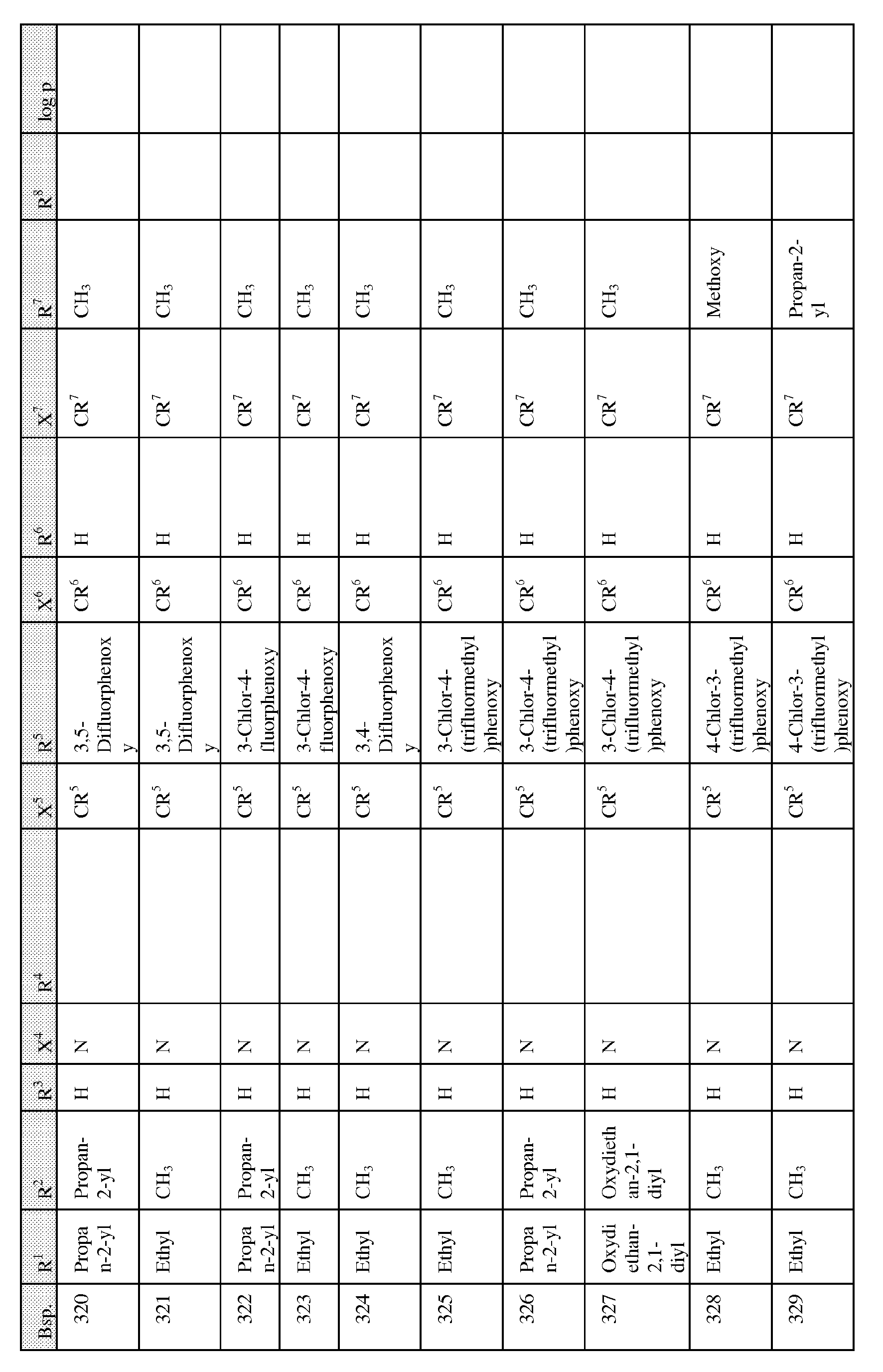

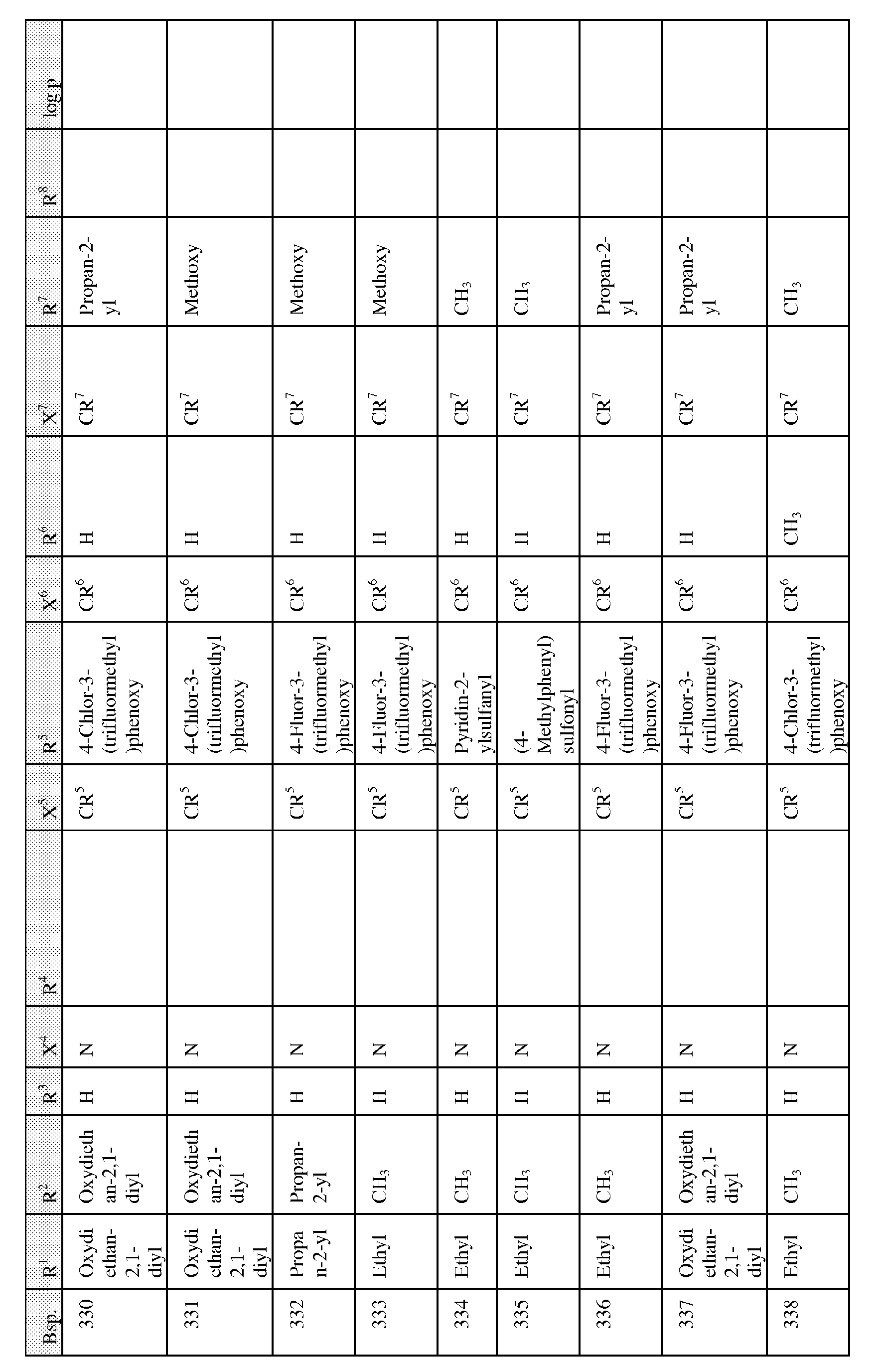

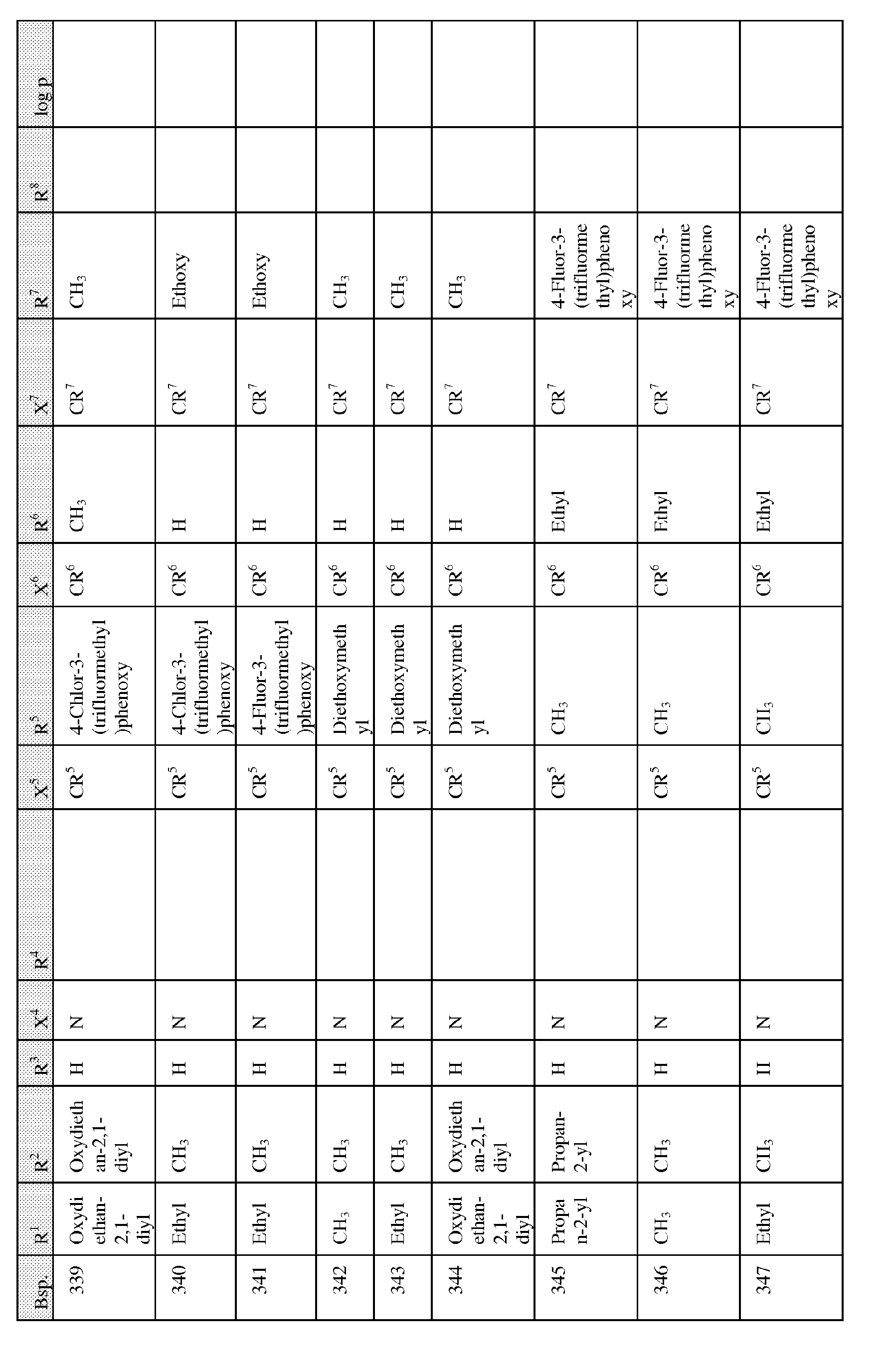

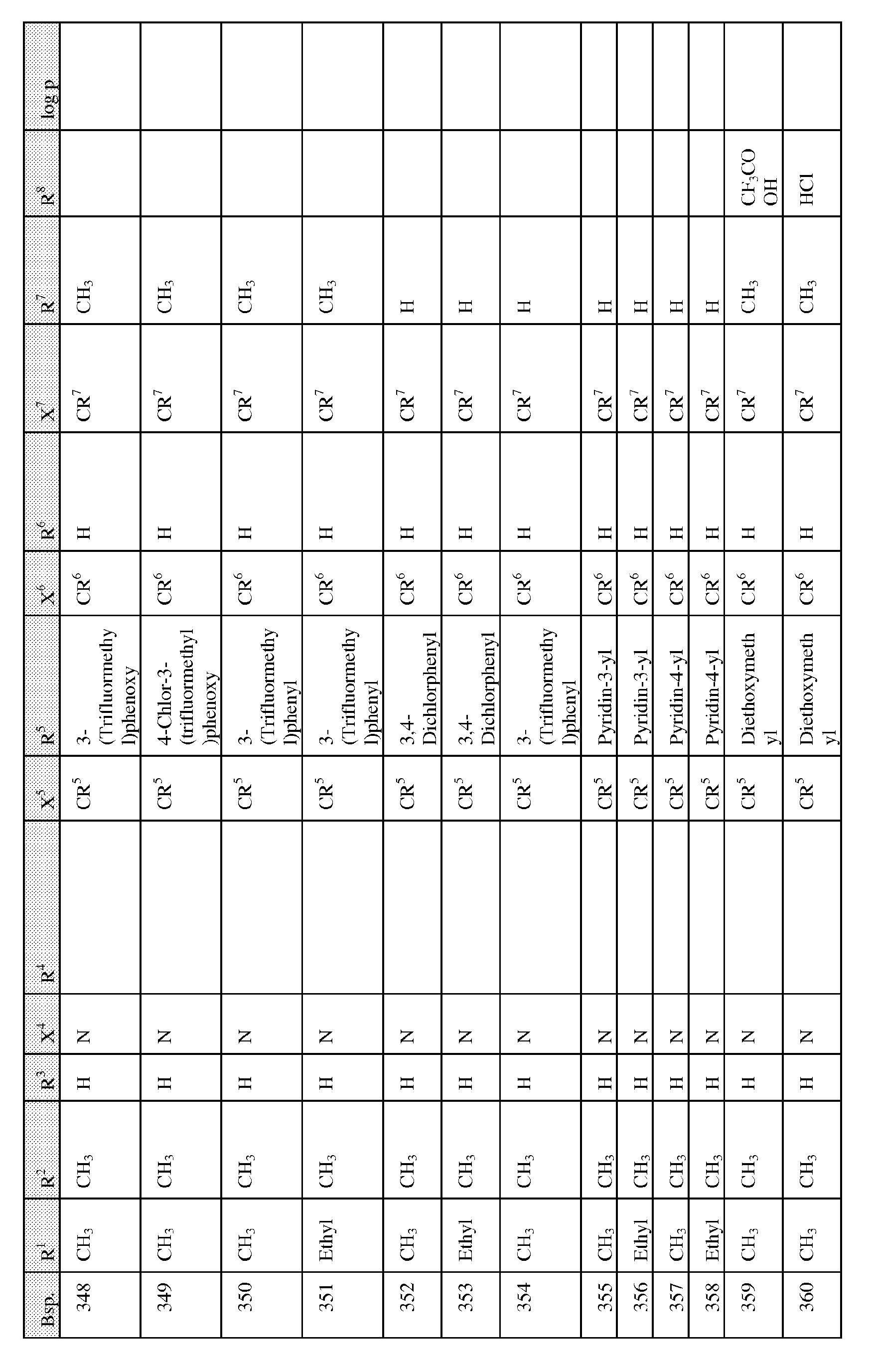

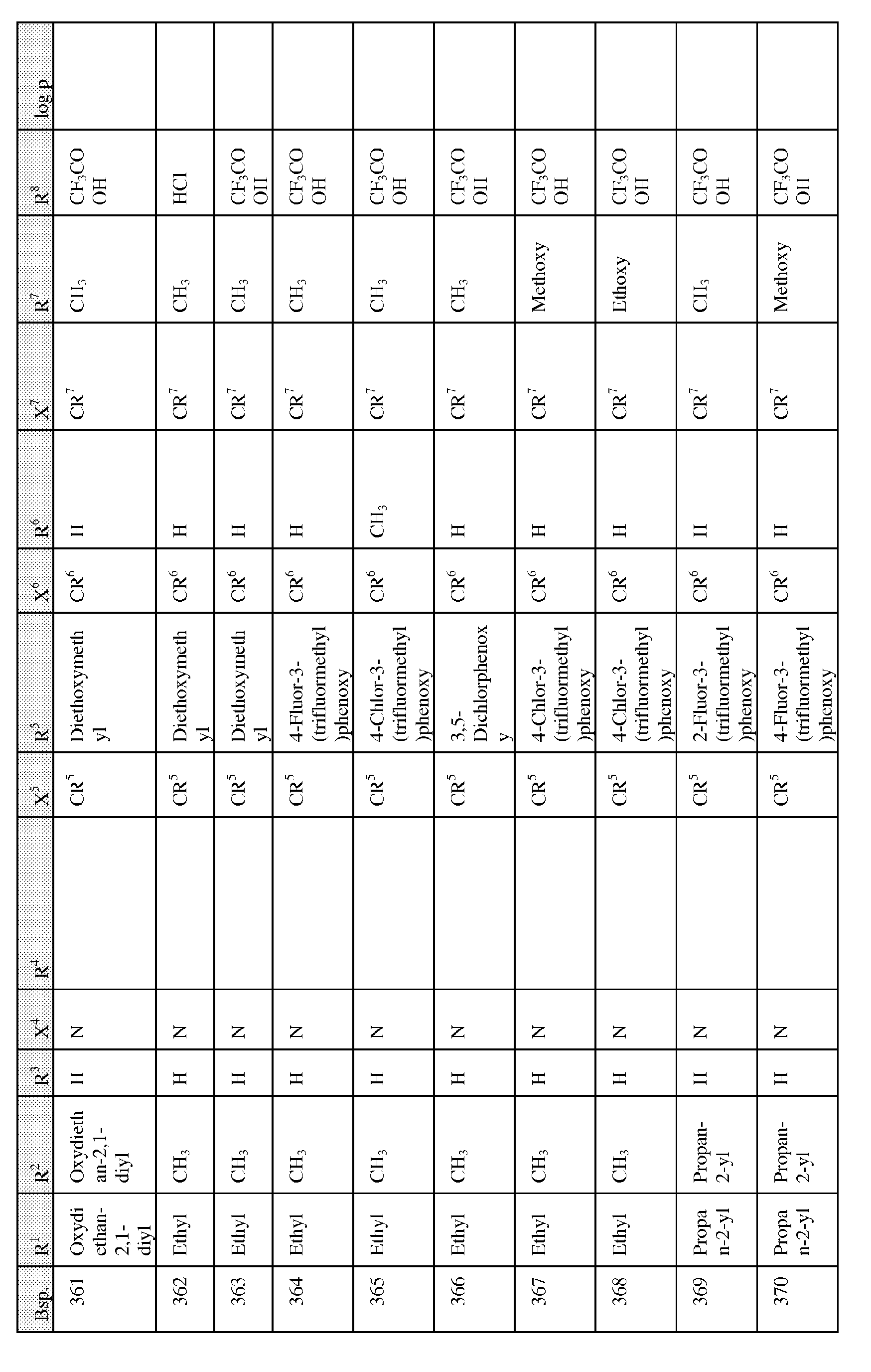

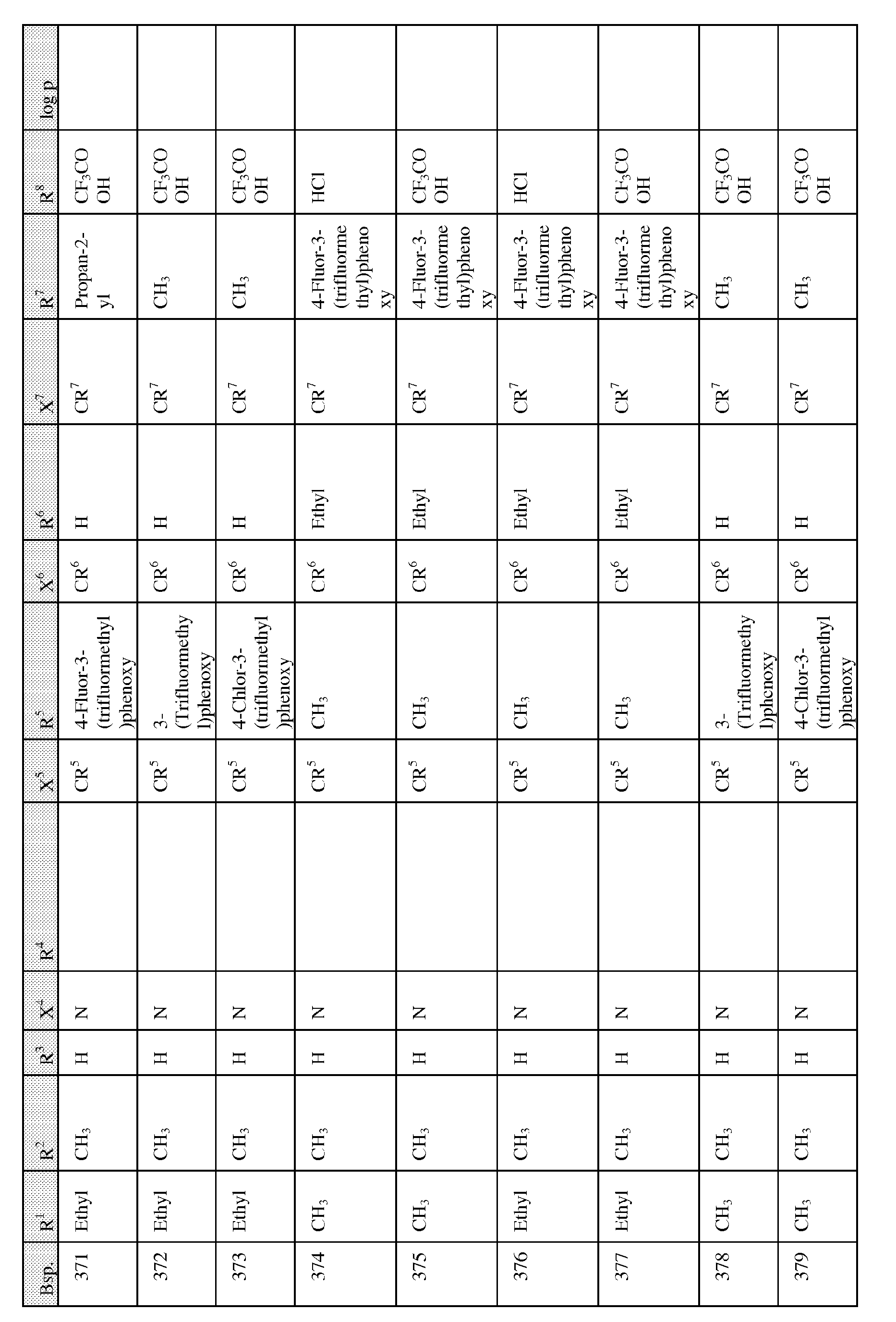

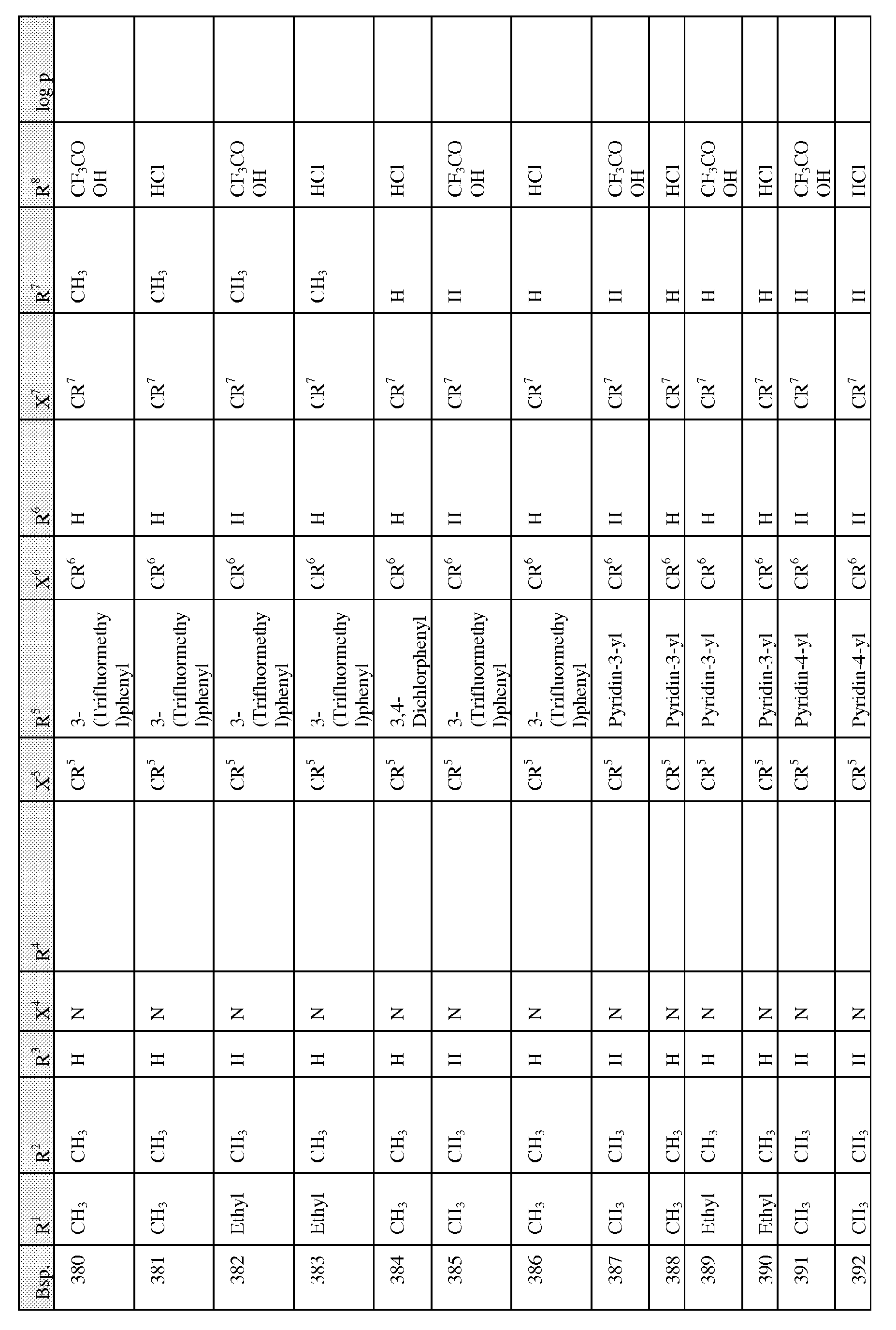

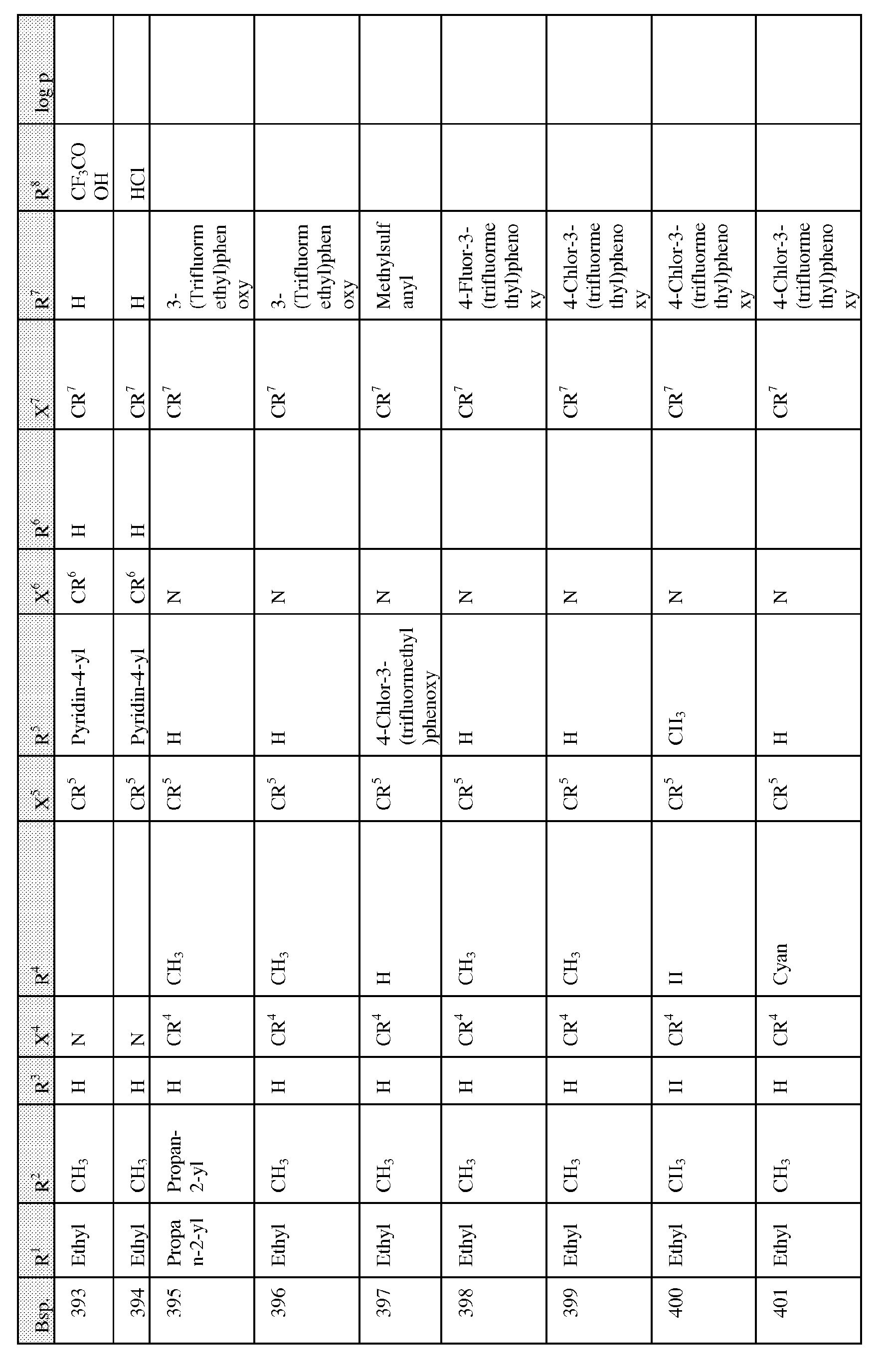

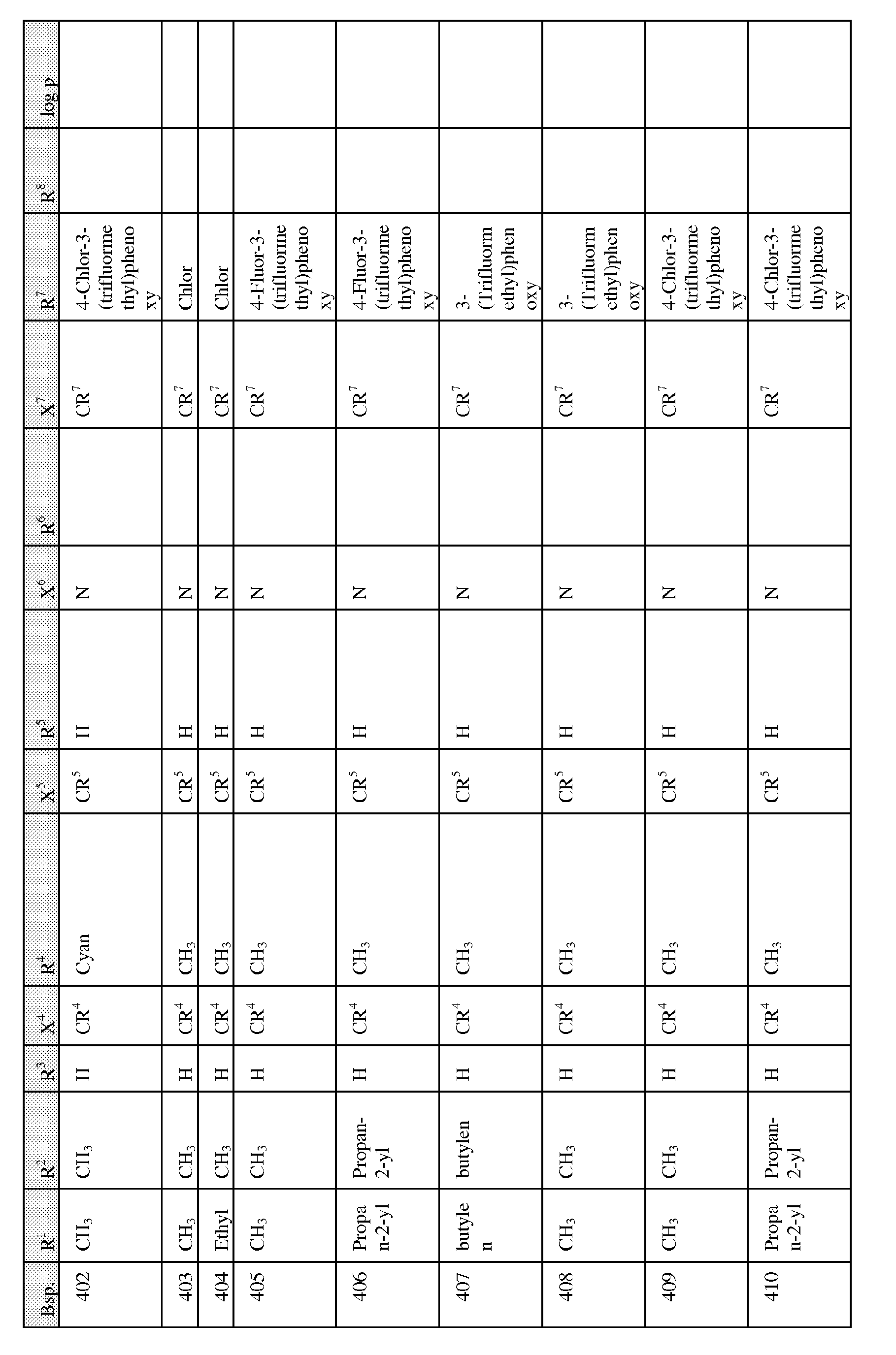

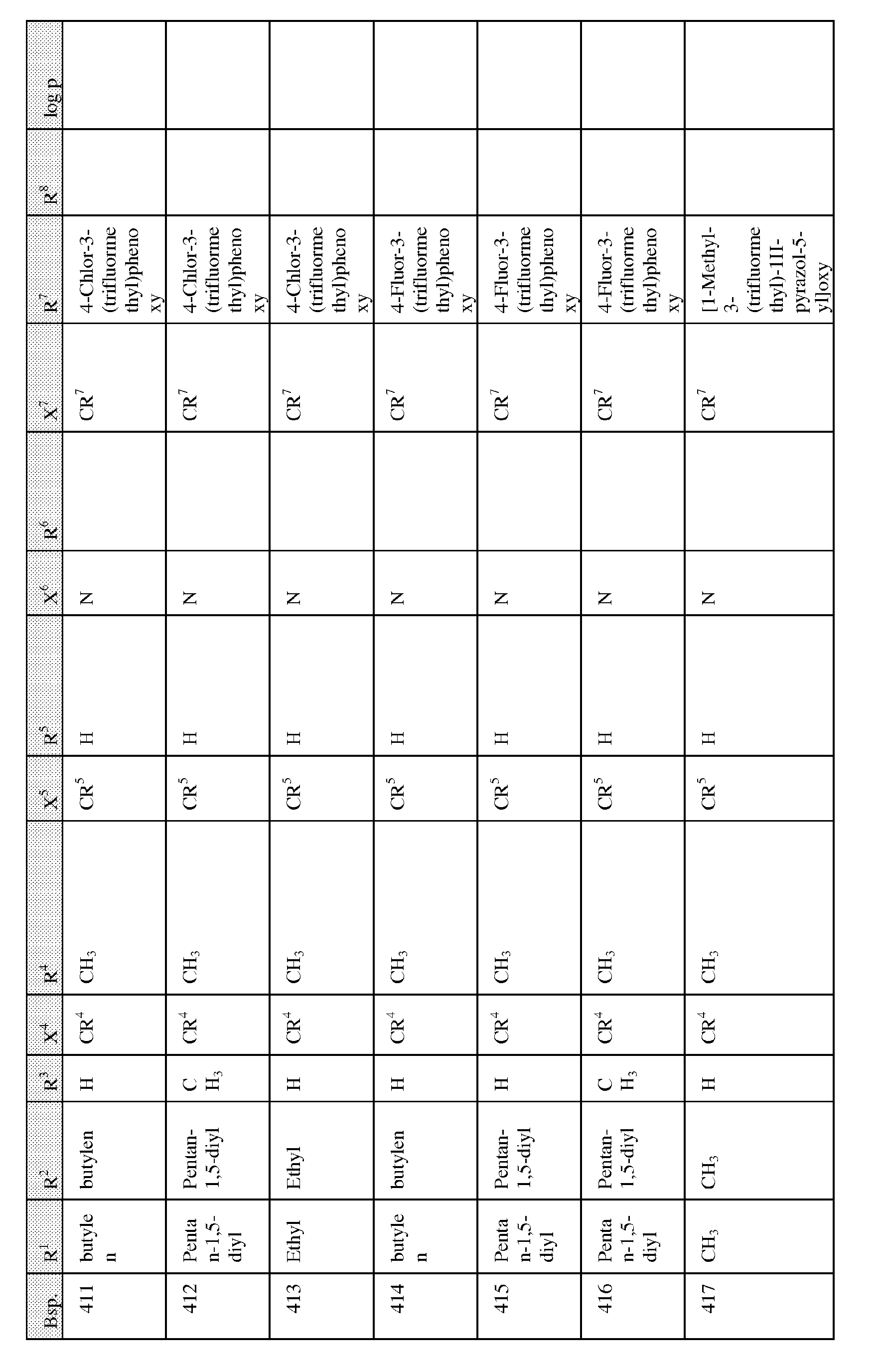

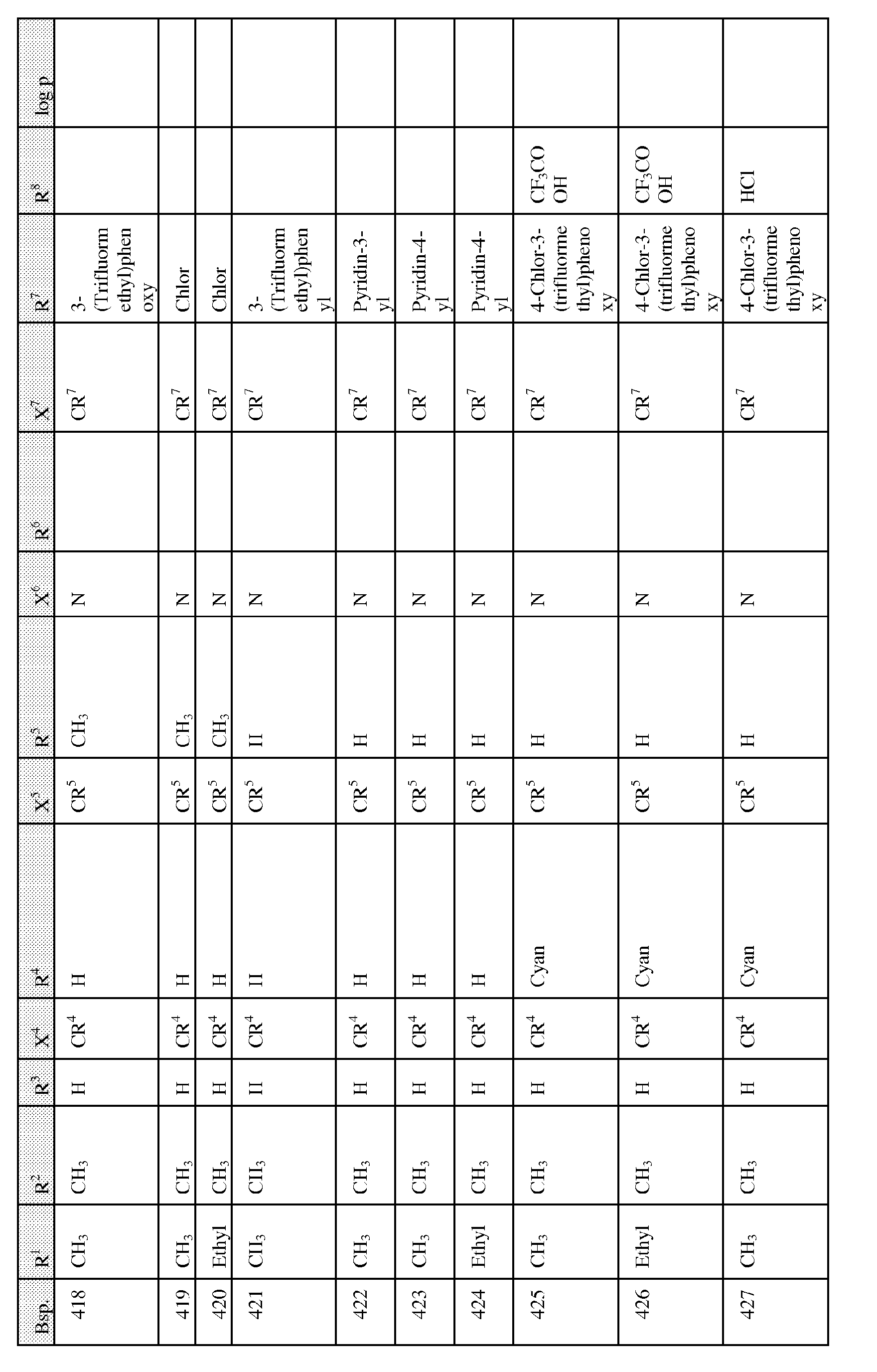

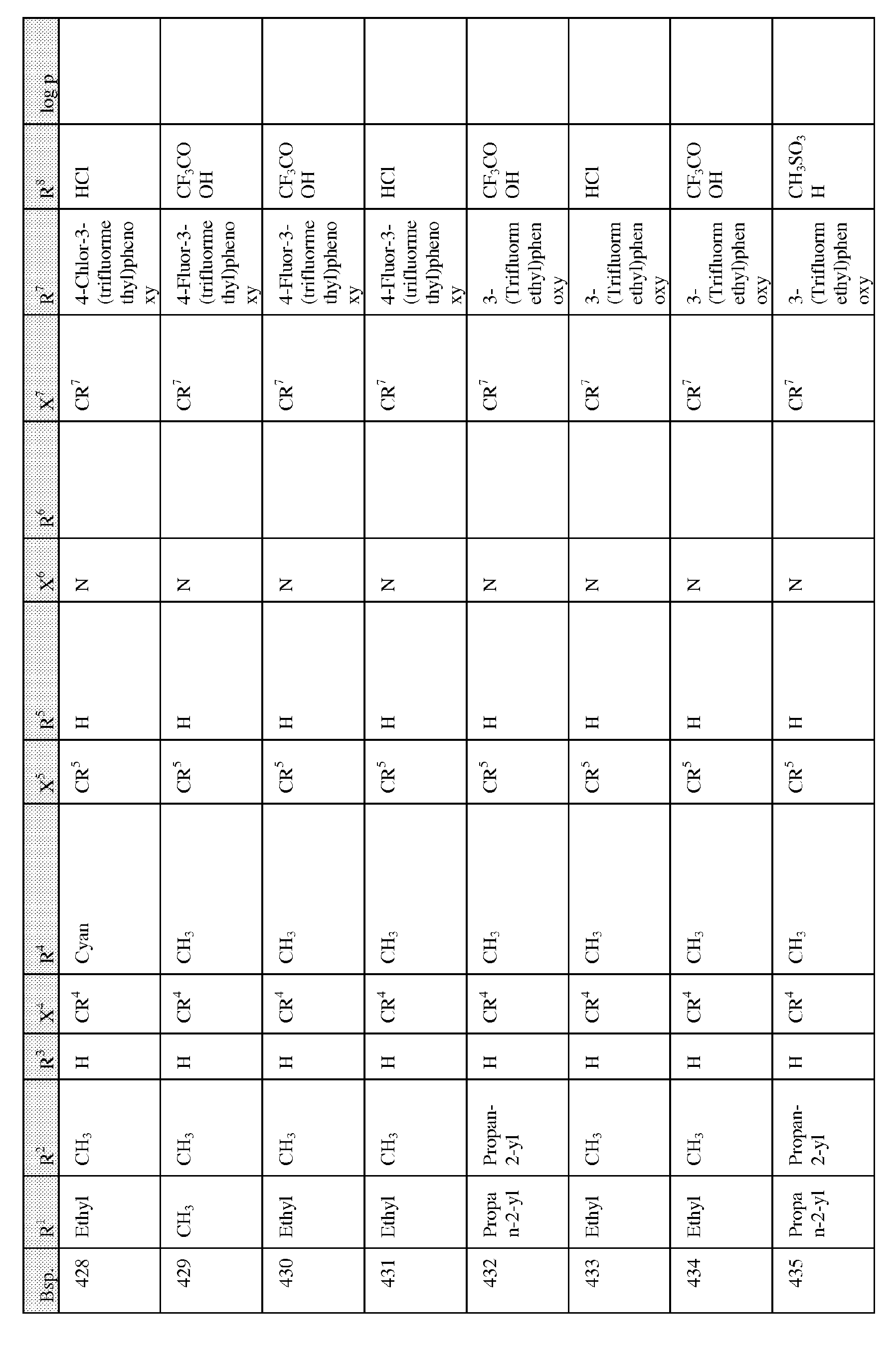

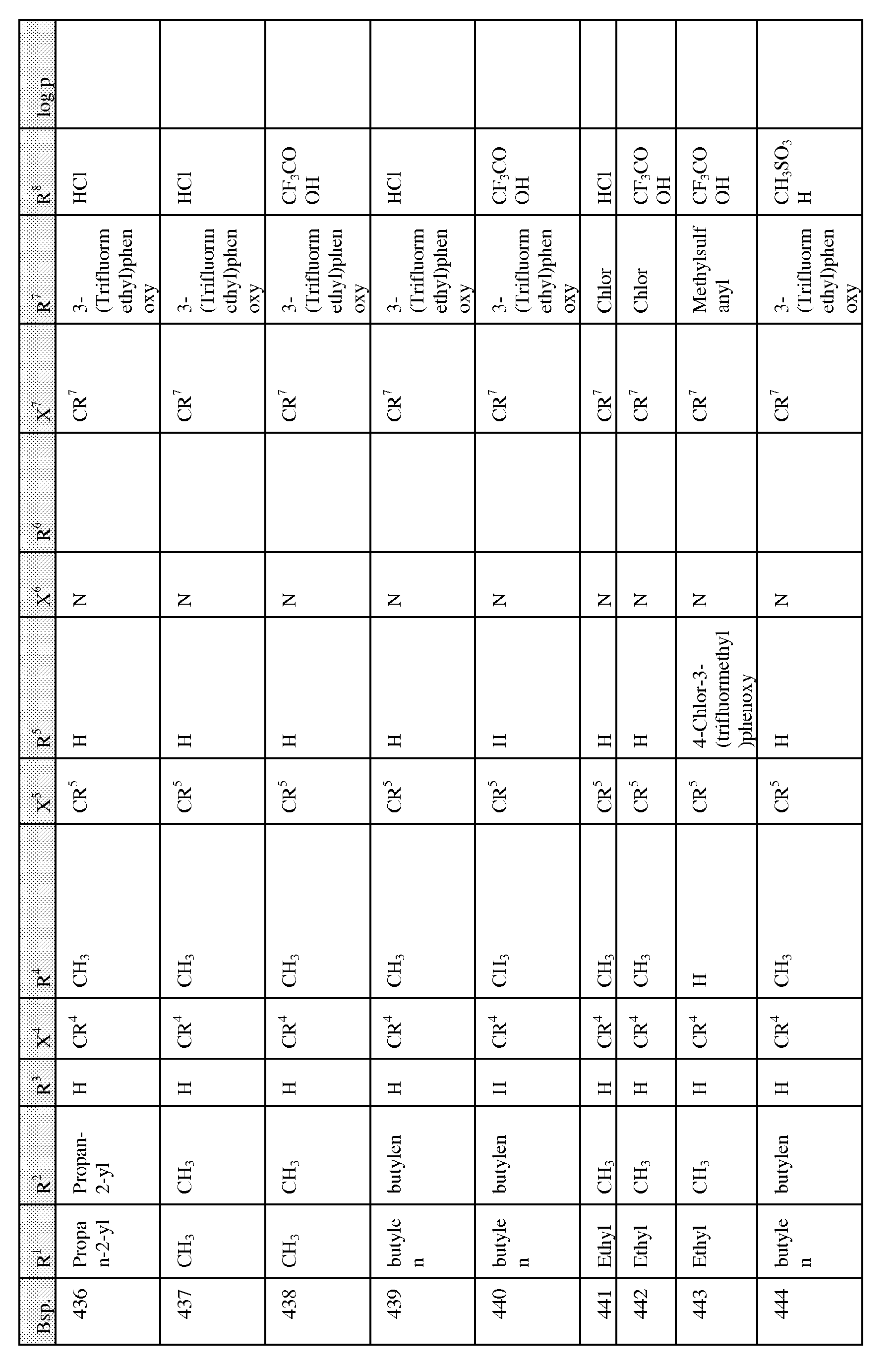

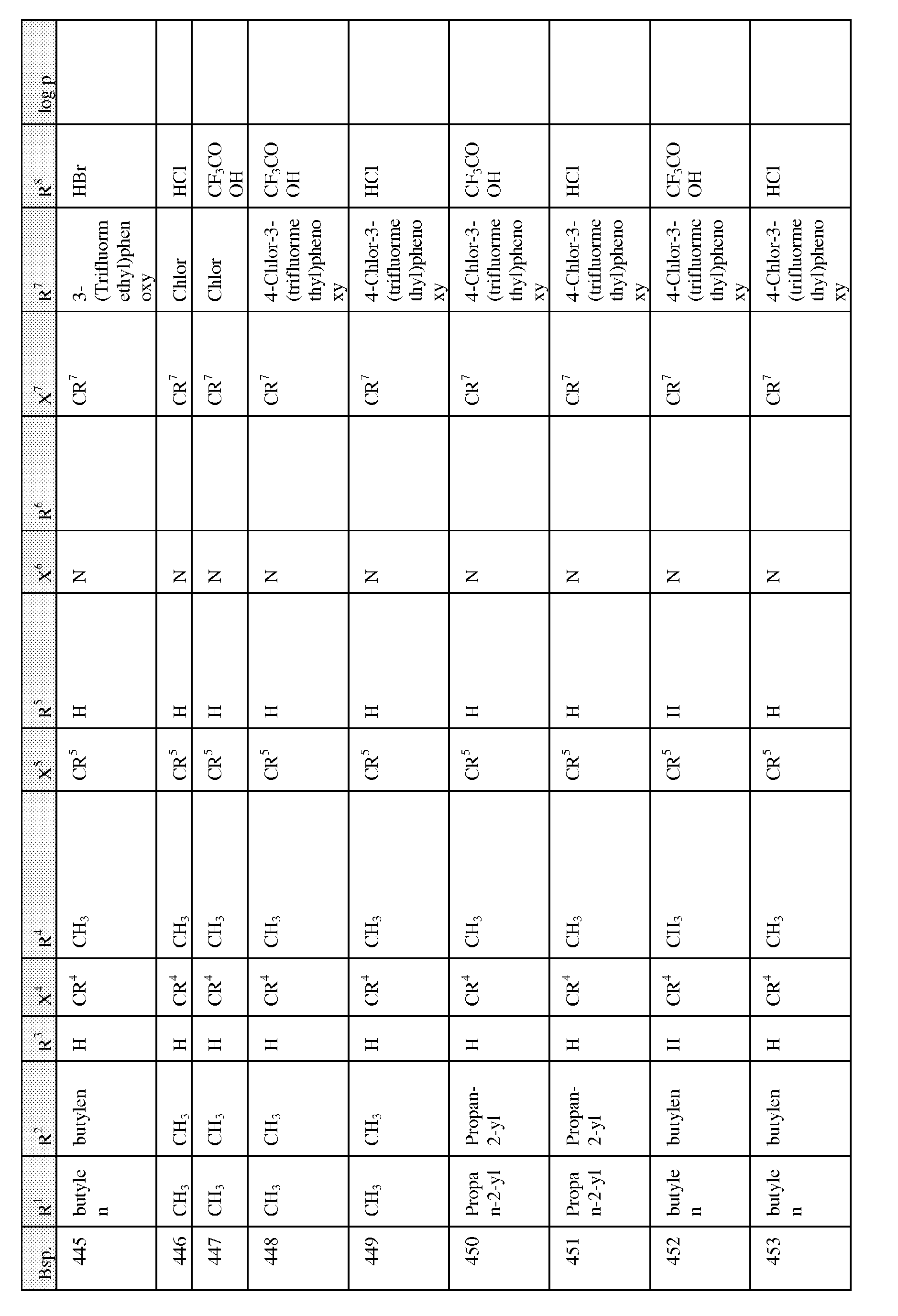

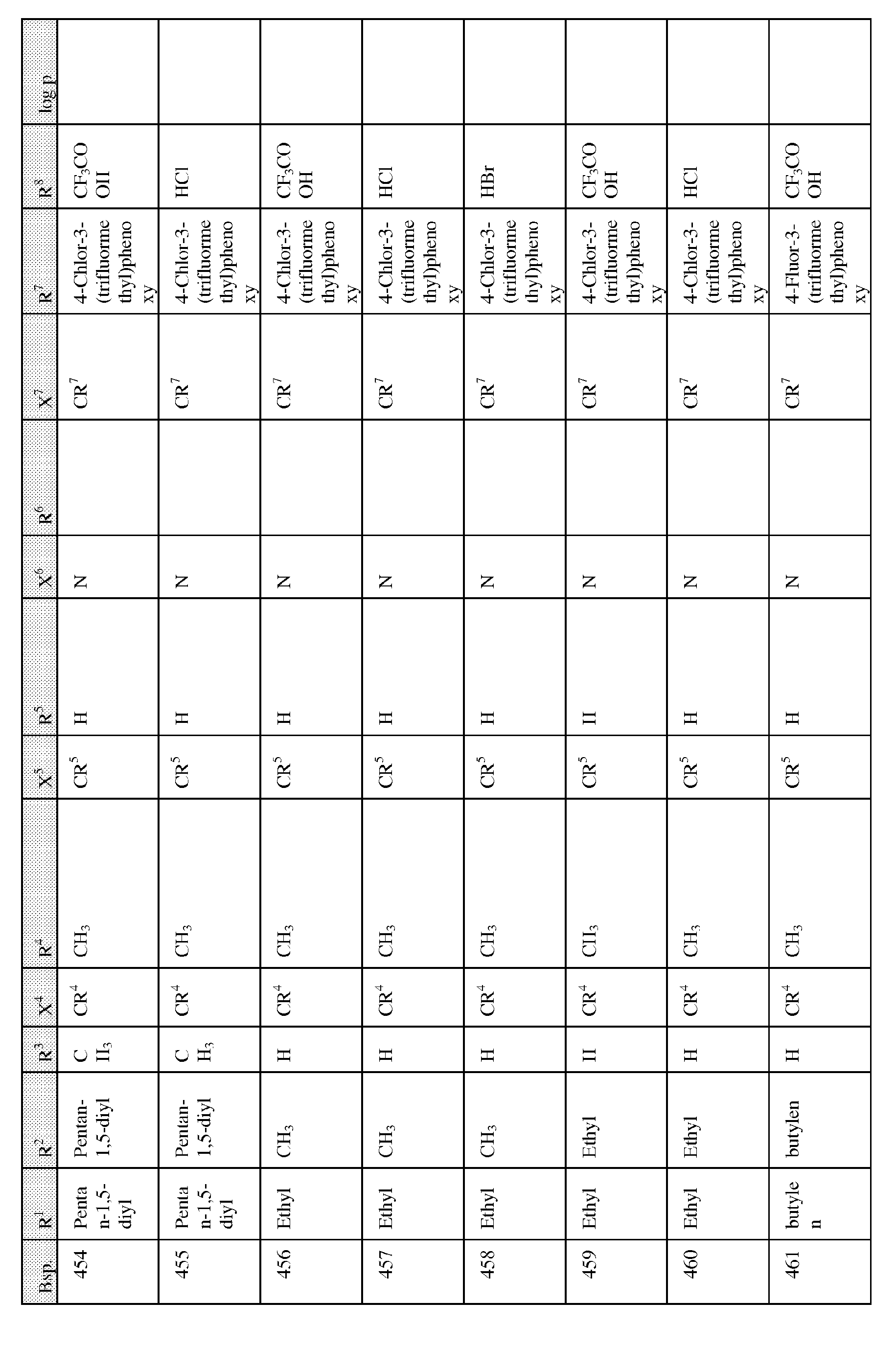

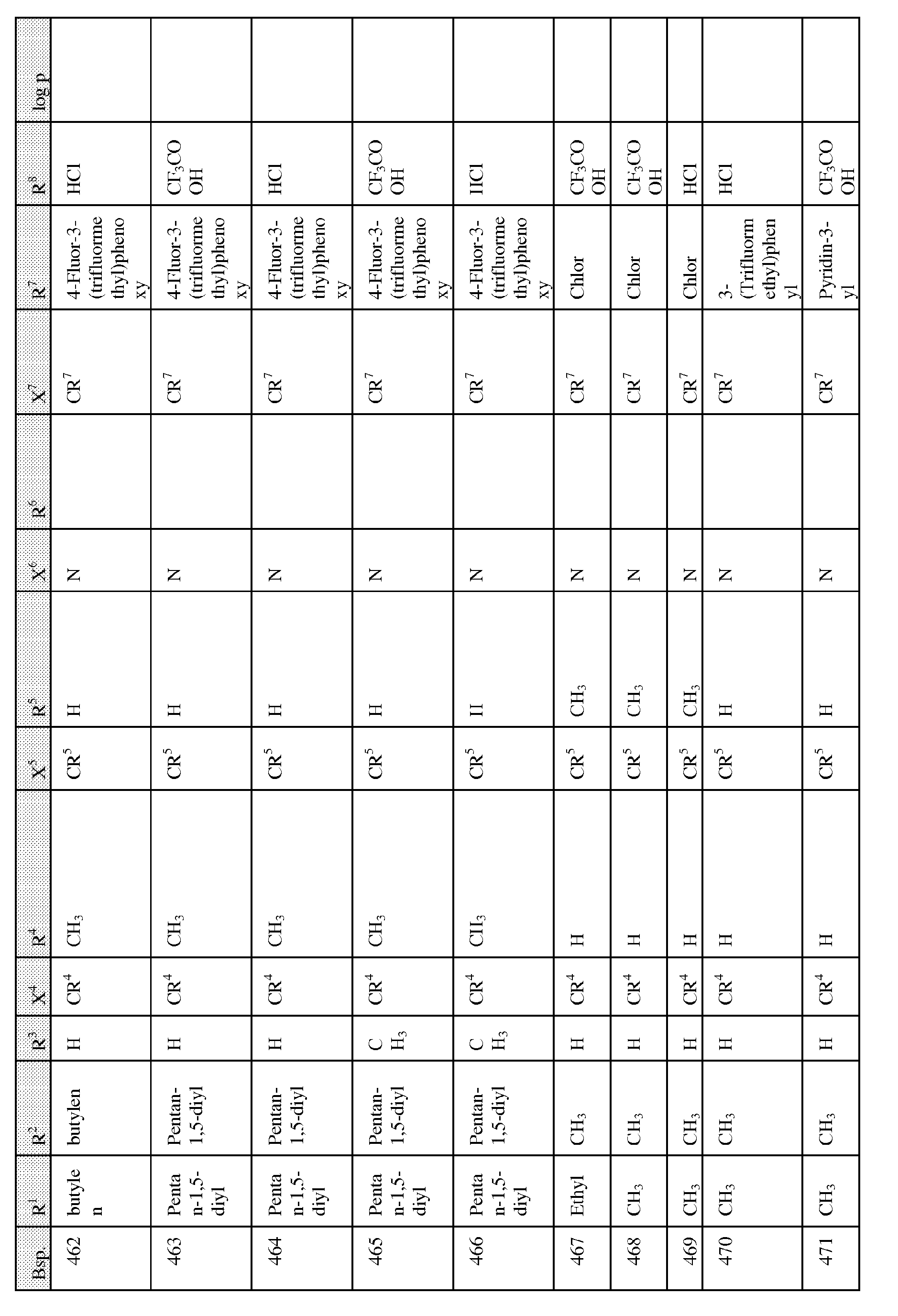

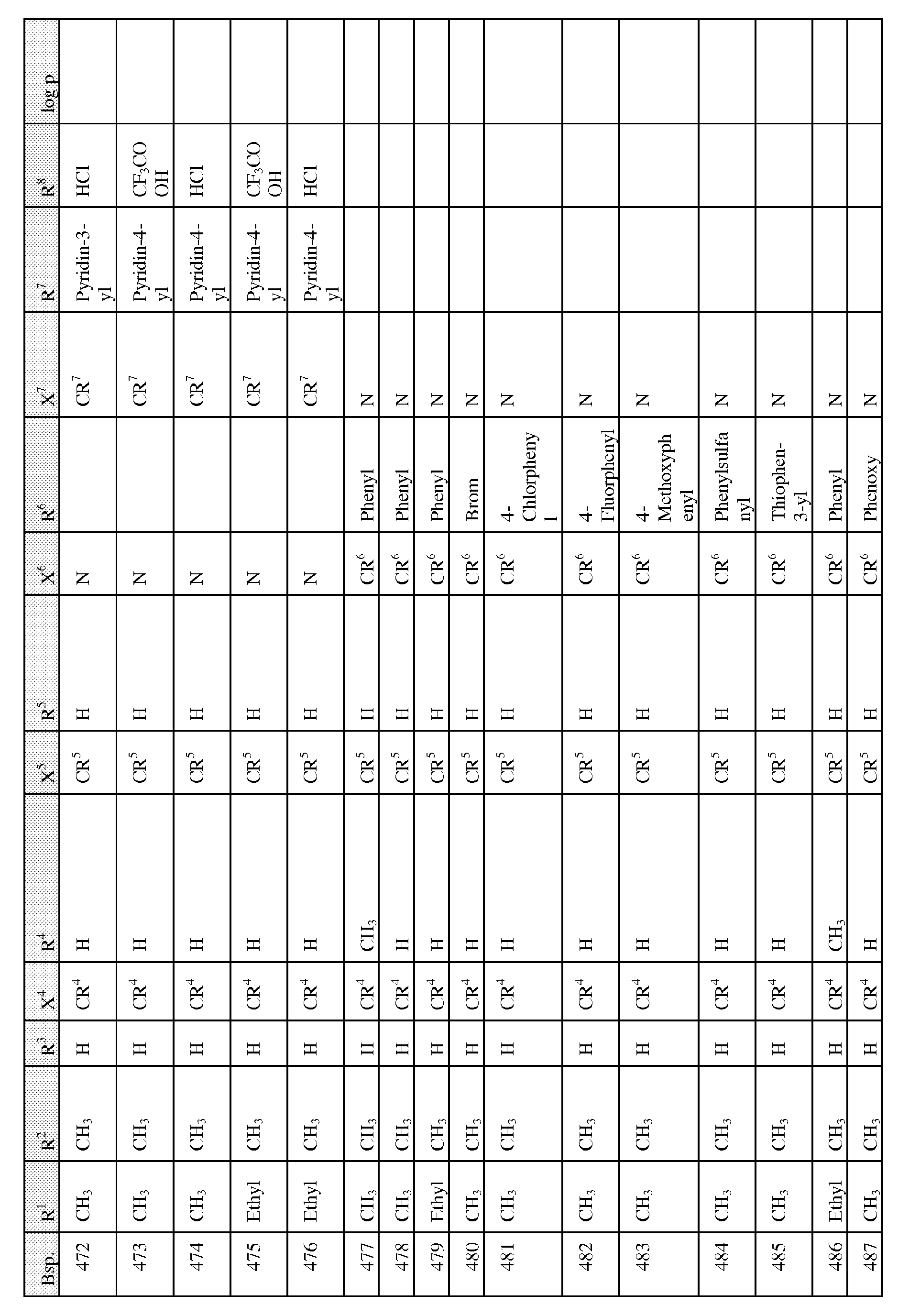

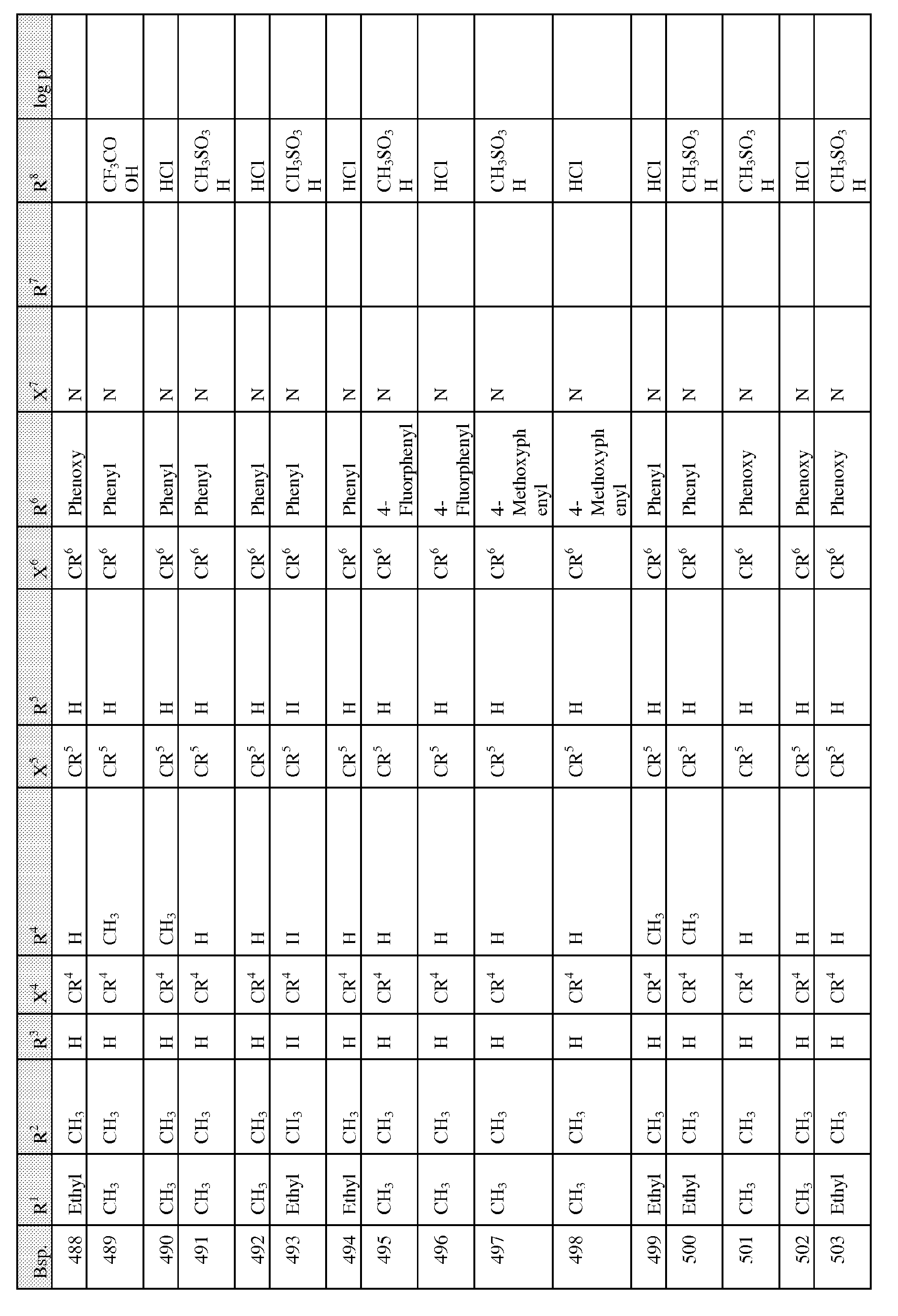

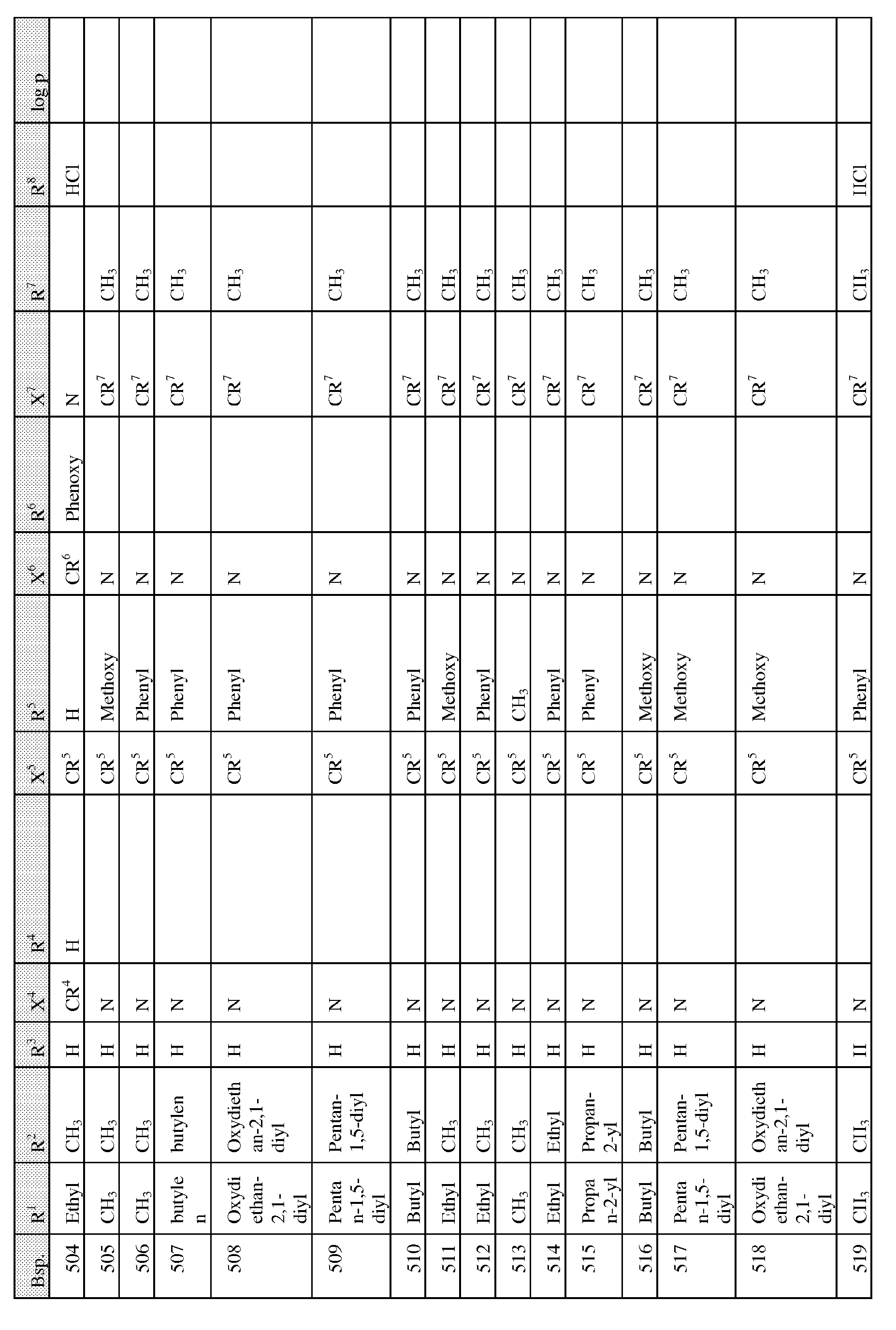

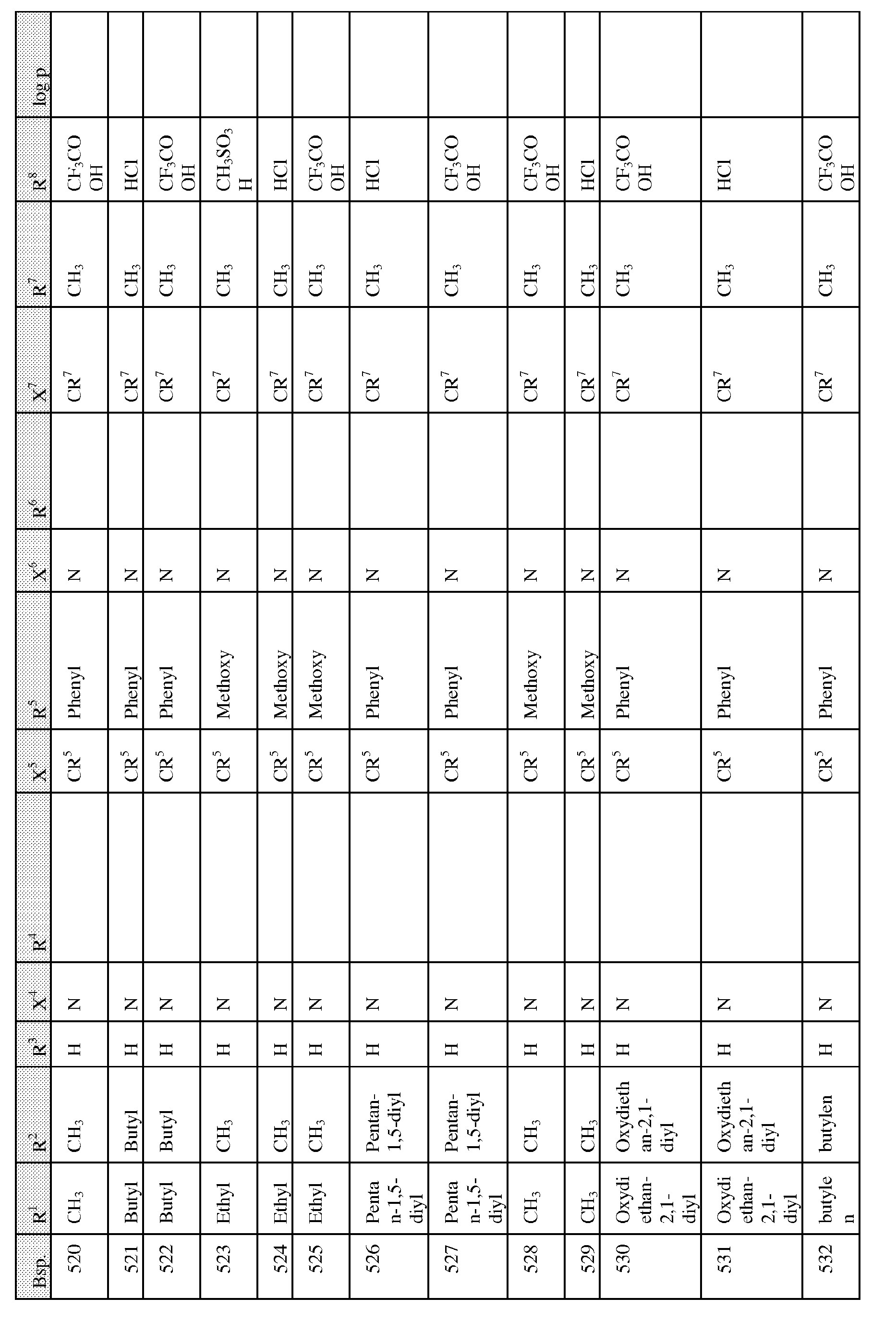

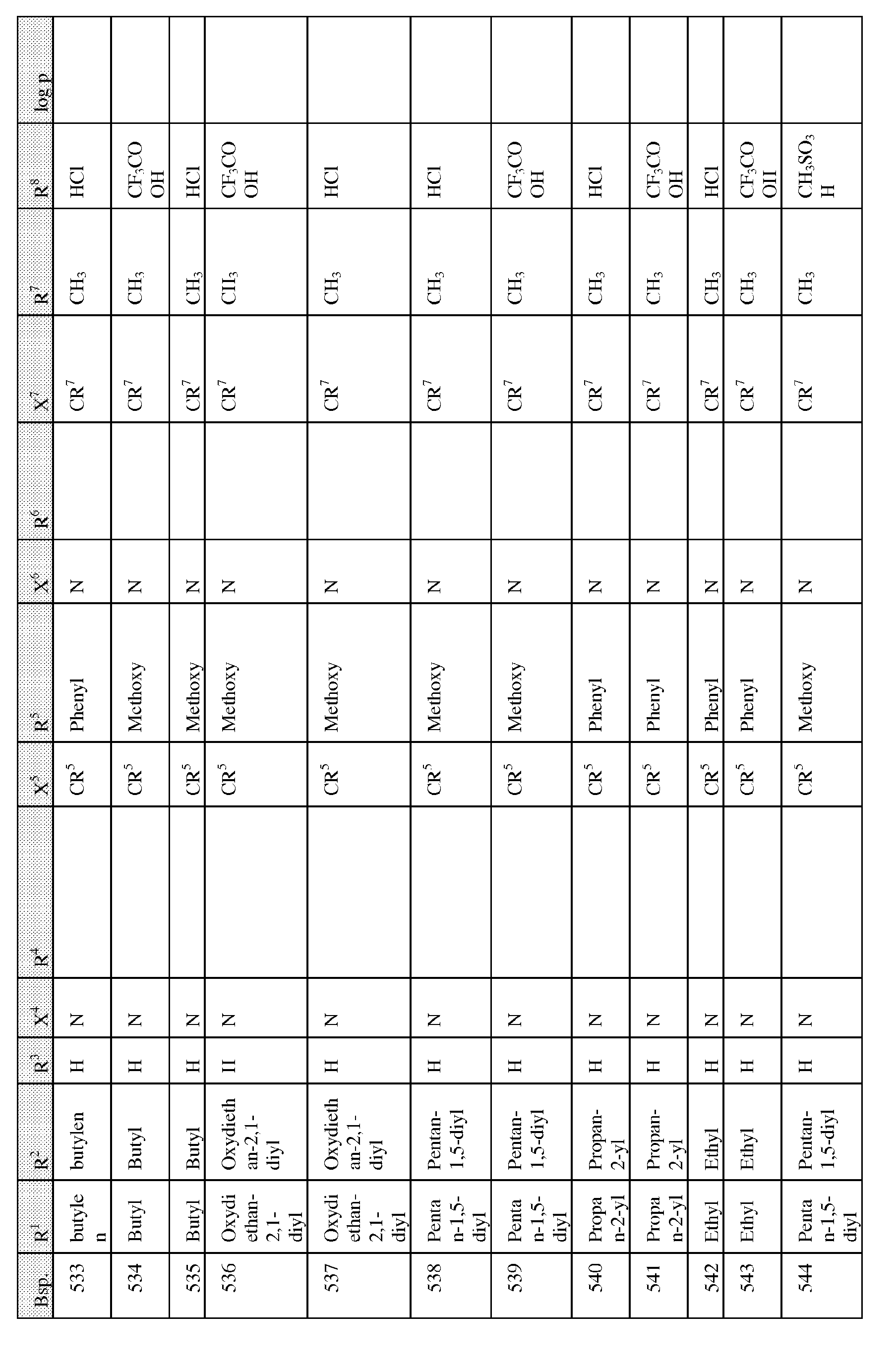

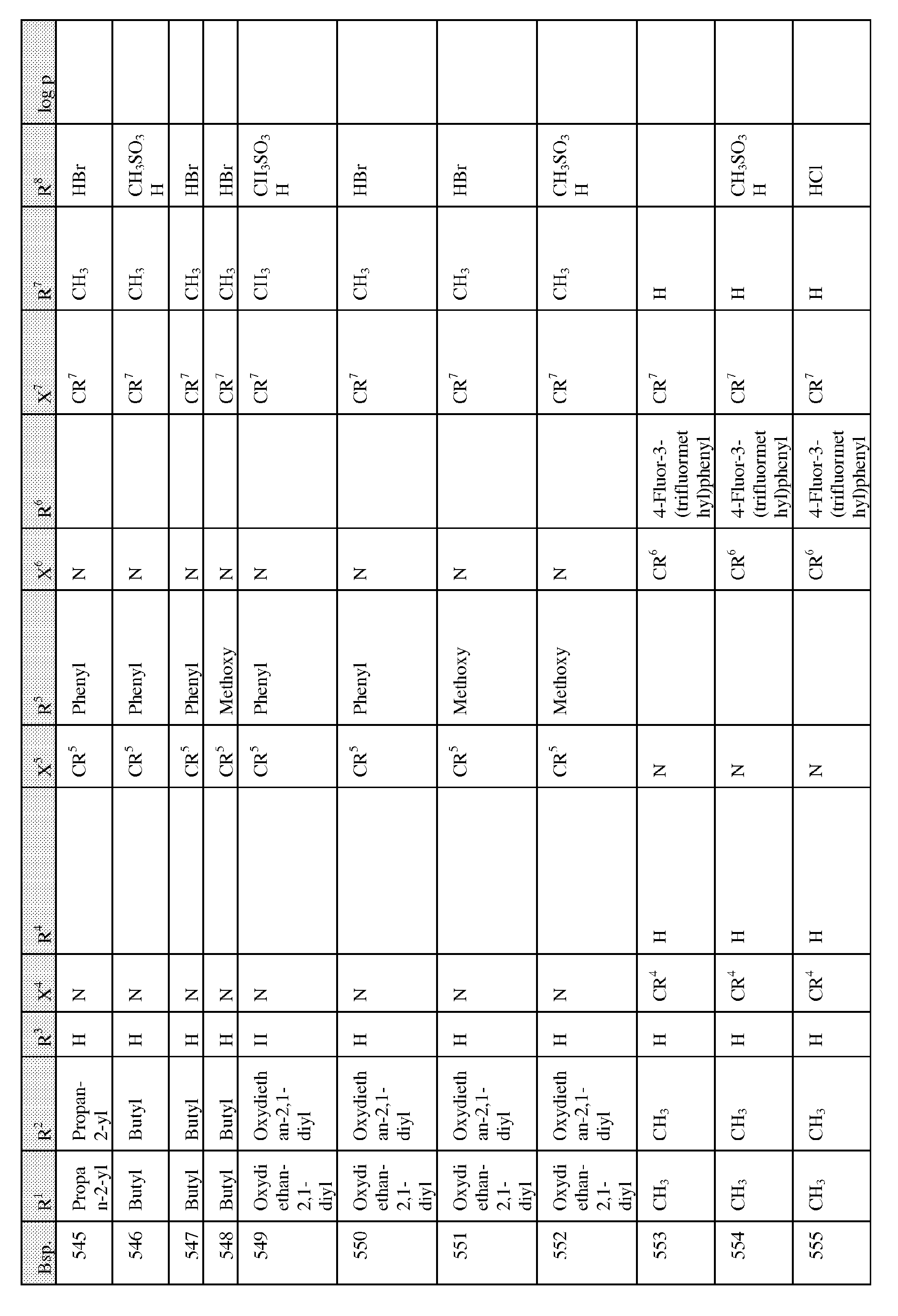

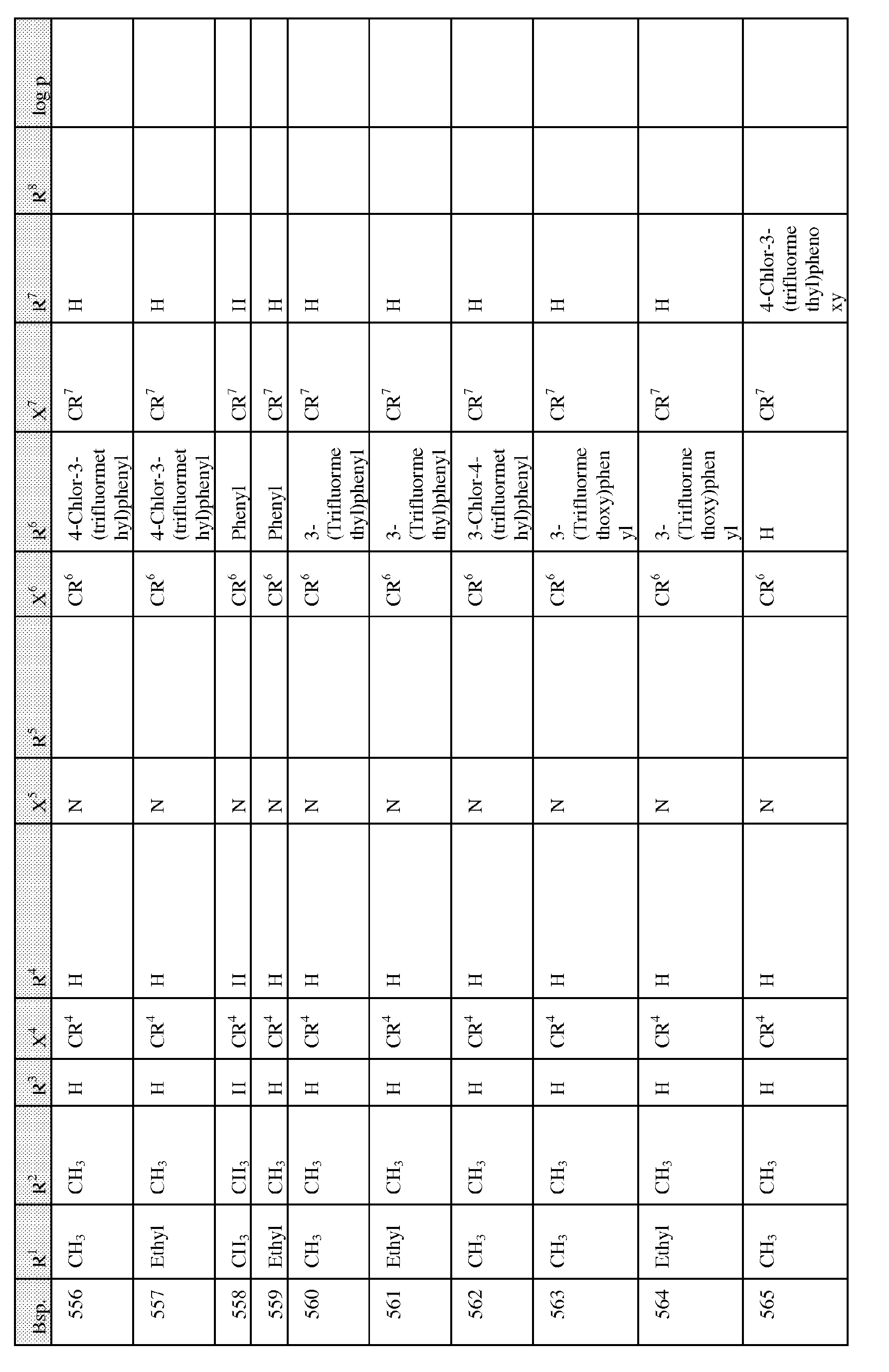

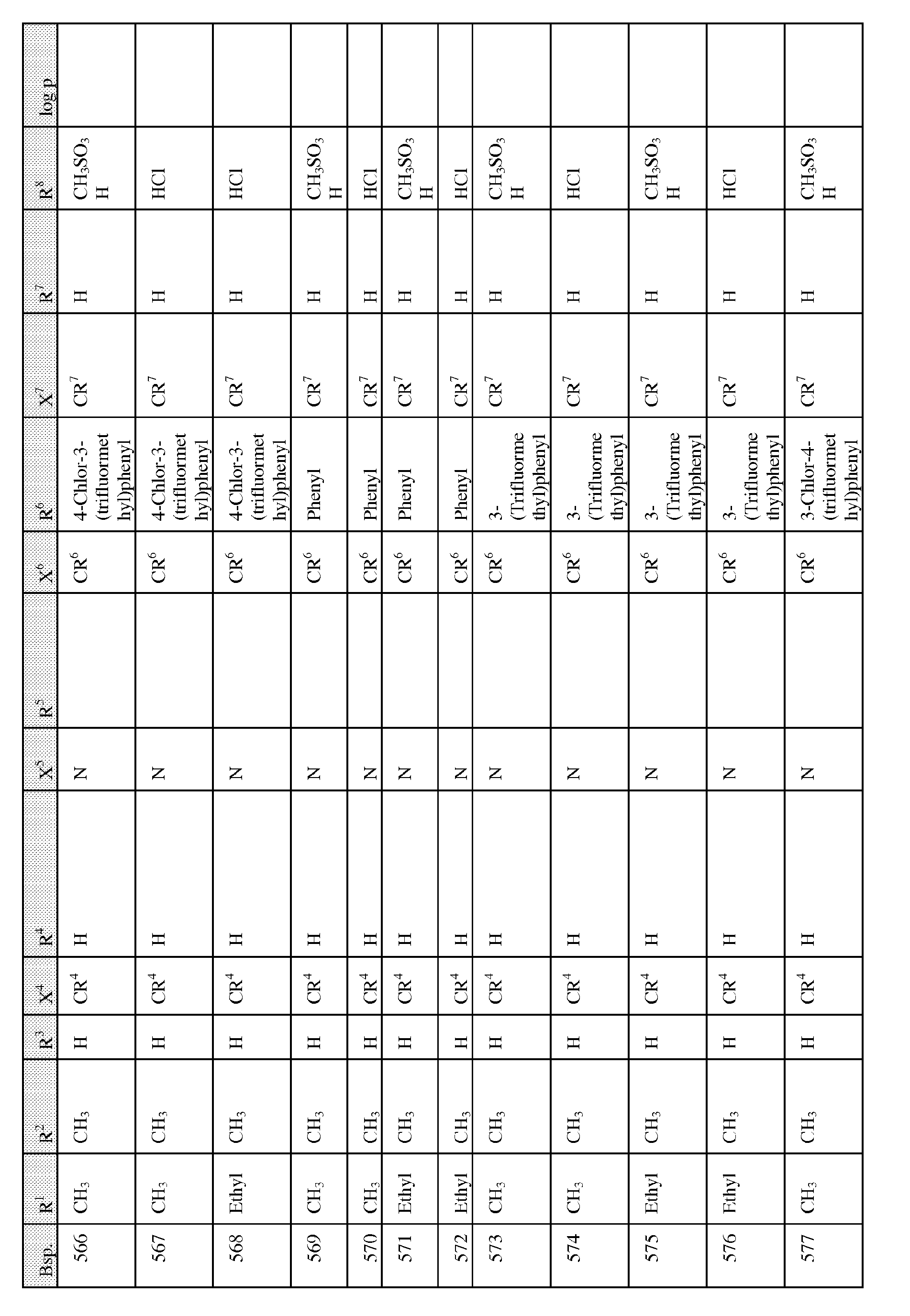

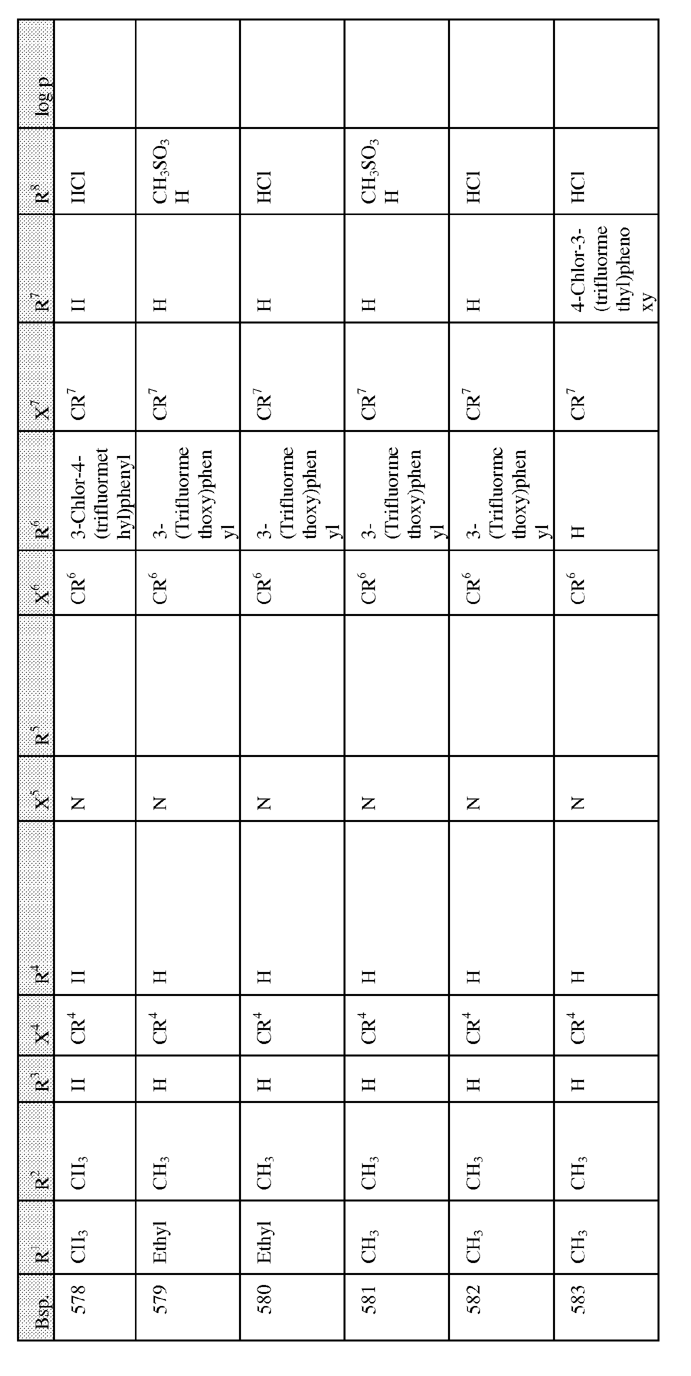

Die Messung der logP Werte erfolgte gemäß EEC Directive 79/831 Annex V.A8 durch HPLC (High Performance Liquid Chromatography) an reversed-phase Säulen (C 18), mit der nachfolgenden Methode:

### Biologische Beispiele

### Fungizide Wirkung

### Botrytis-Test (Gurke) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von ***Botrytis cinerea*** inokuliert und stehen dann 48h bei 100% rel. Feuchte und 22°C. Anschließend stehen die Pflanzen bei 96% rel. Luftfeuchtigkeit und einer Temperatur von 14°C.

5-6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 342, 359, 360, 425, 516, 517, 534, 535, 536, 537, 540, 543 aus Tabelle 1 bei einer Konzentration an Wirkstoff von 500ppm einen Wirkungsgrad von 70% oder mehr.

### Insektizide Wirkung

### Beispiel A

### Tetranychus-Test, OP-resistent (TETRUR Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung > 80 % bei einer Aufwandmenge von 500 g/ha :
Bsp Nr : 24, 33, 72, 73, 128.

### Beispiel B

### Phaedon-Test (PHAECO Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung > 80 % bei einer Aufwandmenge von 500 g/ha :
Bsp Nr : 182, 184, 257.

### Herbizide Wirkung

Die herbizide Wirkung der Verbindungen der allgemeinen Formel (I) wird in den nachfolgenden Tabellen näher erläutert.

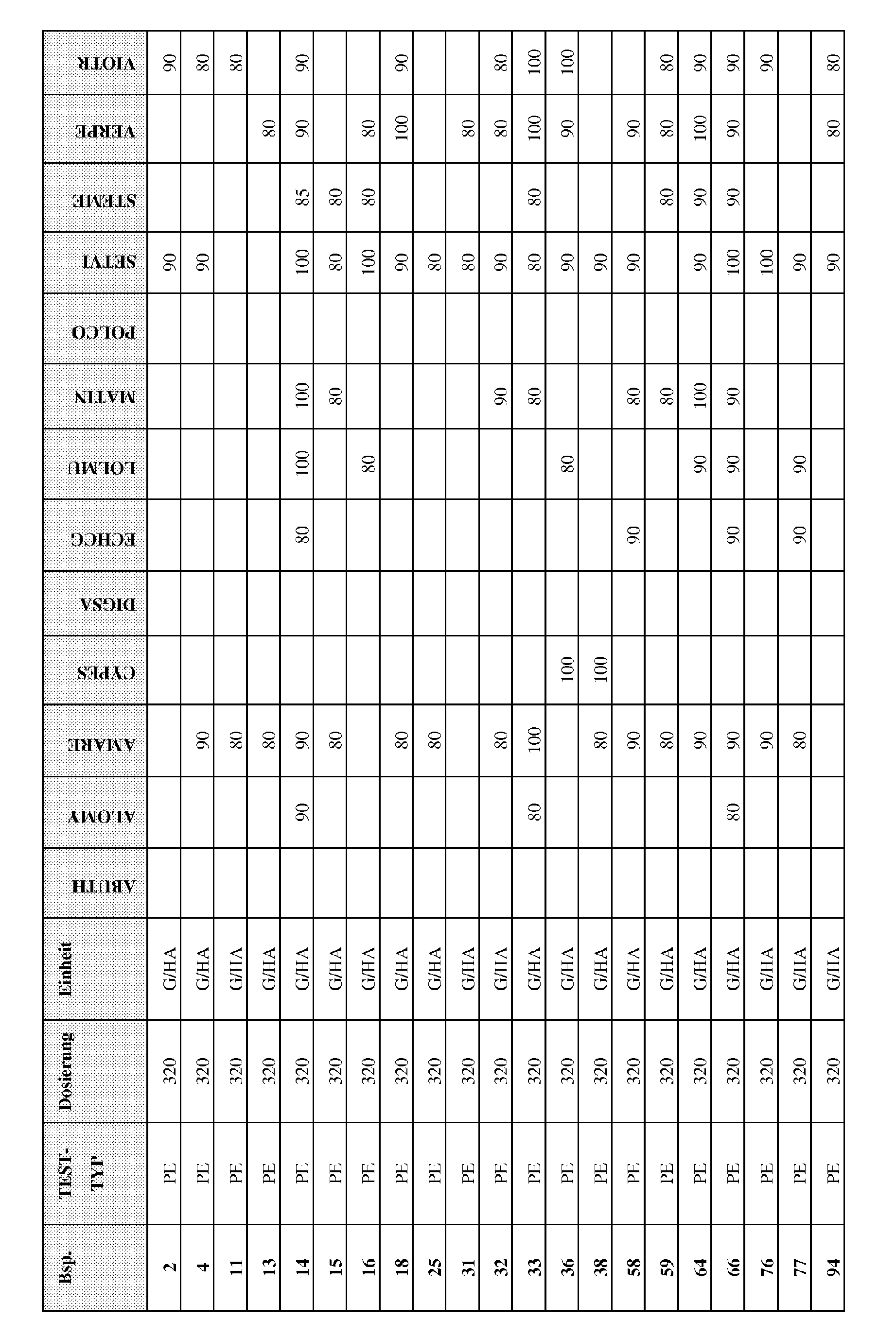

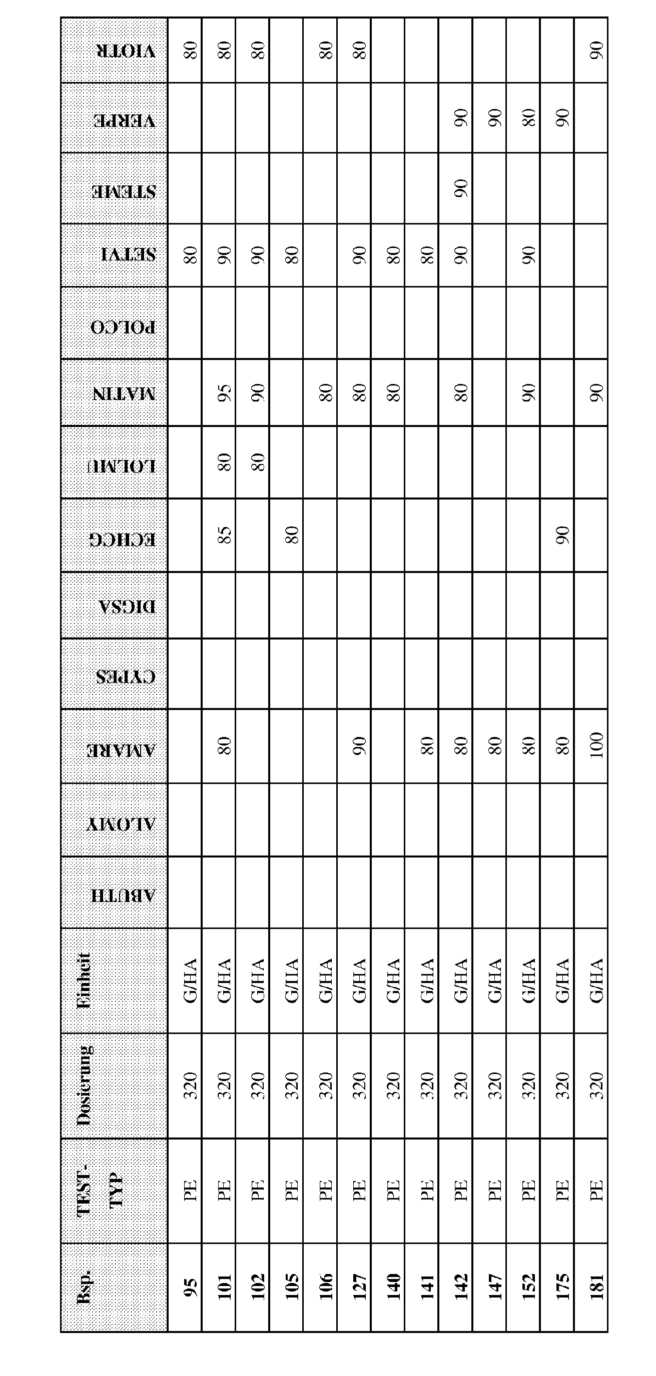

| **Bsp.** | **TEST-TYP** | **Dosierung** | **EINHEIT** | **ABUTH** | **AMARE** | **ECHCG** | **PHBPU** | **SETVI** | **VERPE** | **VIOTR** |
|---|---|---|---|---|---|---|---|---|---|---|
| 15 | PO | 80 | G/HA | | 90 | | | | 80 | 80 |
| 33 | PO | 80 | G/HA | | 80 | | | | 80 | 90 |
| 64 | PO | 80 | G/HA | | 80 | | | | | 80 |
| 66 | PO | 80 | G/HA | | | | 80 | | | 80 |
| 101 | PO | 80 | G/HA | | 80 | | 80 | | | |
| 140 | PO | 80 | G/HA | | 80 | | 80 | | 80 | |
| 181 | PO | 80 | G/HA | | 80 | | 90 | | 80 | |

## Patentansprüche

1. Hetarylamidine der allgemeinen Formel (I) 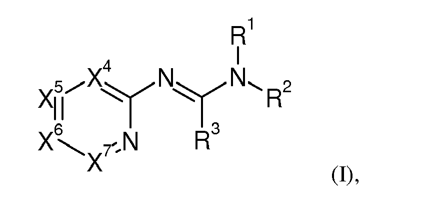 in welchen
R¹ und R², unabhängig voneinander, für gegebenenfalls substituierte Reste ausgewählt aus Alkyl, Alkenyl, Alkinyl, Carbocyclyl oder Heterocyclyl; Wasserstoff; Cyano; Acyl; -OR^{a} oder -SR^{a} stehen, wobei R^{a} für gegebenenfalls substituierte Reste, ausgewählt aus Alkyl, Alkenyl, Alkinyl, Carbocyclyl oder Heterocyclyl steht; oder
R¹ und R², zusammen mit den Atomen, an welche sie gebunden sind, einen fünf- bis siebengliedrigen Ring bilden, welcher substituiert sein kann und welcher gegebenenfalls 1 oder 2 Sauerstoffatome aufweist;
R³ für gegebenenfalls substituierte Reste, ausgewählt aus Alkyl, Alkenyl, Alkinyl, Alkoxycarbonyl, Carbocyclyl oder Heterocyclyl; OH; SH; SO₃H; Cyano; Wasserstoff; -OR^{b} oder -SR^{b} steht, wobei R^{b} für gegebenenfalls substituierte Reste, ausgewählt aus Alkyl, Alkenyl, Alkinyl, Carbocyclyl oder Heterocyclyl steht; oder
R² und R³, zusammen mit den Atomen, an welche sie gebunden sind, einen fünf- bis siebengliedrigen Ring bilden, welcher substituiert sein kann und welcher gegebenenfalls 1 oder 2 Sauerstoffatome aufweist;
X⁴ für C-R⁴ oder N steht;
X⁵ für C-R⁵ oder N steht;
X⁶ für C-R⁶ oder N steht;
X⁷ für C-R⁷ oder N steht;
R⁴, R⁵, R⁶ und R⁷, jeweils unabhängig voneinander für Wasserstoff; Halogen; Hydroxy; gegebenenfalls substituierte Reste, ausgewählt aus Alkyl, Alkenyl, Alkinyl, Alkoxy, Alkoxyalkyl, Amino, Carbocyclyl und Heterocyclyl; Azido; Nitro; Cyano; Acyl; Cyanato; -SF₅; -OR^{b} oder -S(O)ₙR^{b} stehen, wobei R^{b} für gegebenenfalls substituierte Reste, ausgewählt aus C₁-C₁₂- Alkyl, C₃-C₁₀-Carbocyclyl oder C₃-C₁₀-Heterocyclyl steht und n für eine ganzzahlige Zahl von 0 bis 2 steht; mit der Maßgabe, dass von den Resten X⁴, X⁵, X⁶ und X⁷ 0 bis 2 Reste gleich Stickstoff sind; sowie die N-Oxide und Salze der Verbindungen der allgemeinen Formel (I).

2. Hetarylamidine der allgemeinen Formel (I) nach Anspruch 1, **dadurch gekennzeichnet, dass** R¹ und R², jeweils unabhängig voneinander, für gegebenenfalls substituierte Reste, ausgewählt aus Alkyl, Alkenyl und Carbocyclyl; und Wasserstoff stehen oder R¹ und R², zusammen mit den Atomen, an welche sie gebunden sind, einen Ring bilden, welcher substituiert sein kann und welcher gegebenenfalls 1 oder 2 Sauerstoffatome aufweist.

3. Hetarylamidine der allgemeinen Formel (I) nach Anspruch 1 oder 2, **dadurch gekennzeichnet, dass** der Rest R³ für gegebenenfalls substituierte Reste ausgewählt aus Alkyl und Alkenyl; oder Wasserstoff steht, oder R² und R³, zusammen mit den Atomen, an welche sie gebunden sind, einen Ring bilden, welcher substituiert sein kann.

4. Hetarylamidine der allgemeinen Formel (I) nach einem der Ansprüche 1 bis 3, **dadurch gekennzeichnet, dass** X⁴ gleich Stickstoff, X⁵ gleich C-R⁵, X⁶ gleich C-R⁶ und X⁷ gleich C-R⁷ sind.

5. Hetarylamidine der allgemeinen Formel (I) nach einem der Ansprüche 1 bis 3, **dadurch gekennzeichnet, dass** X⁵ gleich Stickstoff, X⁴ gleich C-R⁴, X⁶ gleich C-R⁶ und X⁷ gleich C-R⁷ sind.

6. Hetarylamidine der allgemeinen Formel (I) nach einem der Ansprüche 1 bis 3, **dadurch gekennzeichnet, dass** X⁶ gleich Stickstoff, X⁴ gleich C-R⁴, X⁵ gleich C-R⁵ und X⁷ gleich C-R⁷ sind.

7. Hetarylamidine der allgemeinen Formel (I) nach einem der Ansprüche 1 bis 3, **dadurch gekennzeichnet, dass** X⁷ gleich Stickstoff, X⁴ gleich C-R⁴, X⁵ gleich C-R⁵ und X⁶ gleich C-R⁶ sind.

8. Hetarylamidine der allgemeinen Formel (I) nach einem der Ansprüche 1 bis 3, **dadurch gekennzeichnet, dass** X⁴ und X⁶ gleich Stickstoff, X⁵ gleich C-R⁵ und X⁷ gleich C-R⁷ sind.

9. Hetarylamidine der allgemeinen Formel (I) nach einem der Ansprüche 1 bis 3, **dadurch gekennzeichnet, dass** X⁴ gleich C-R⁴, X⁵ gleich C-R⁵, X⁶ gleich C-R⁶ und X⁷ gleich C-R⁷ sind.

10. Verfahren zur Herstellung von Hetarylamidine der allgemeinen Formel (I) gemäß einem der Ansprüche 1 bis 9, **gekennzeichnet durch** mindestens eines der folgenden Umsetzungen:
(1) Umsetzung ausgehend von Pyrimidinen der allgemeinen Formel (II-1) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI) gemäß dem nachfolgenden Reaktionsschema: 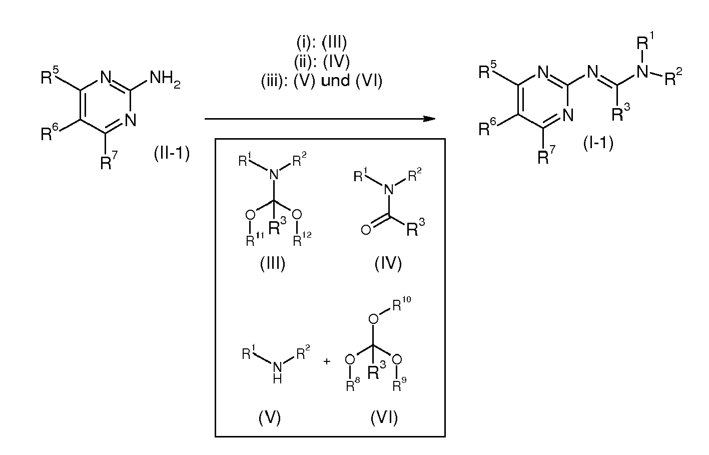
(2) Umsetzung ausgehend von Pyrazinen der allgemeinen Formel (II-2) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI) gemäß dem nachfolgenden Reaktionsschema: 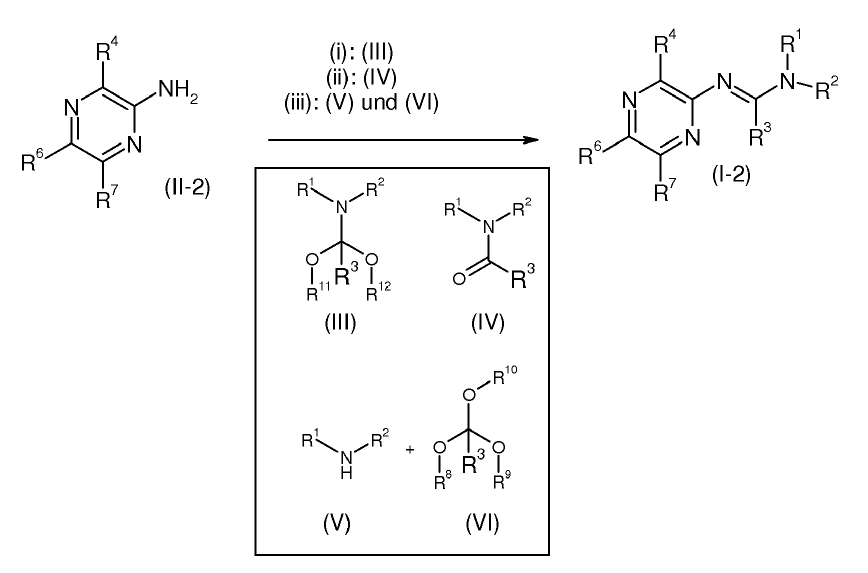
(3) Umsetzung ausgehend von Pyrimidinen der allgemeinen Formel (II-3) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI) gemäß dem nachfolgenden Reaktionsschema: 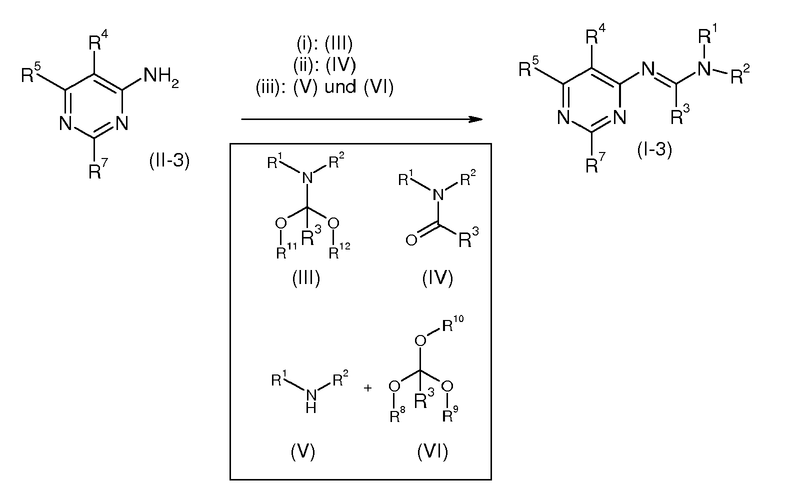
(4) Umsetzung ausgehend von Pyridazinen der allgemeinen Formel (II-4) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI) gemäß dem nachfolgenden Reaktionsschema: 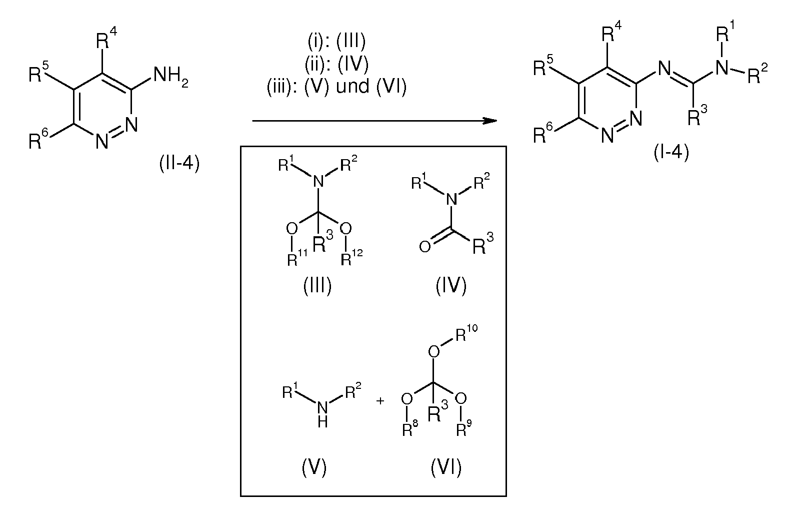
(5) Umsetzung ausgehend von Triazinen der allgemeinen Formel (II-5) nach unterschiedlichen alternativen Methoden unter Verwendung von
(i) Aminoacetalen der Formel (III) oder
(ii) Amiden der Formel (IV) oder
(iii) Aminen der Formel (V) in Gegenwart von Orthoestern der Formel (VI) gemäß dem nachfolgenden Reaktionsschema: 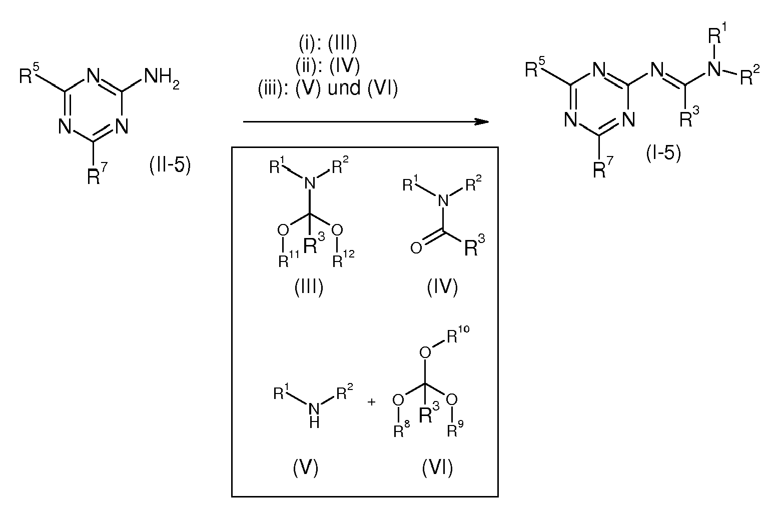
(6) Umsetzung von Verbindungen der allgemeinen Formel (VII) gemäß nachfolgendem Reaktionsschema: 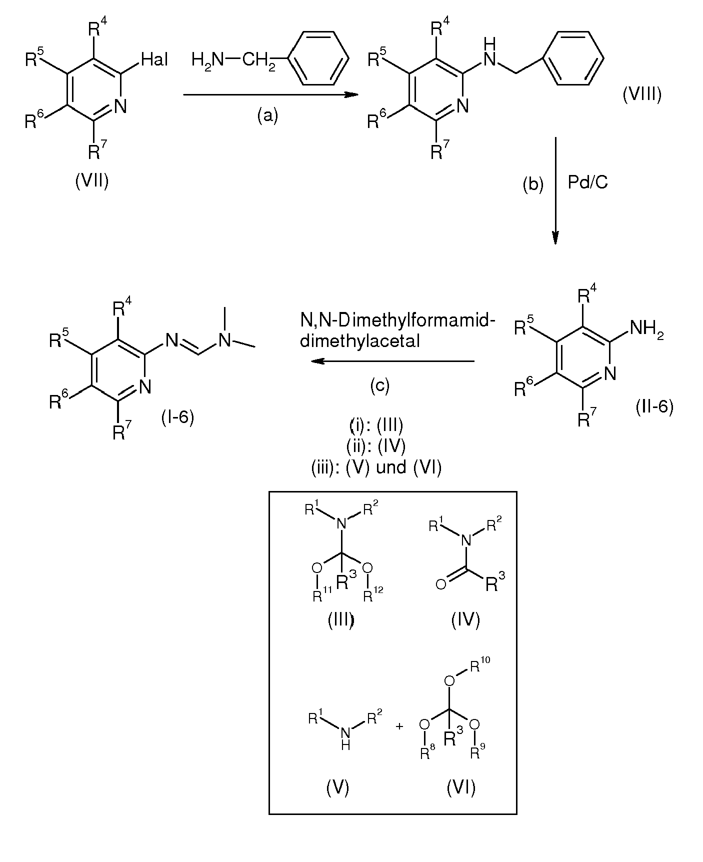 wobei in den vorstehenden Umsetzungen
R¹ bis R⁷ die Bedeutungen gemäß einem der Abnsprüche 1 bis 4 aufweisen;
R⁸ bis R¹⁰ unabhängig voneinander ausgewählt sind aus C₁₋₈-Alkyl-Gruppen, vorzugsweise aus C₂₋₆-Alkyl-Gruppen, besonders bevorzugt aus C₃₋₅-AlkyL¬- Gruppen und gemeinsam mit den O-Atomen, an die sie gebunden sind, einen fünf- oder sechsgliedrigen Ring bilden können; und
R¹¹ und R¹² ausgewählt sind aus C₁₋₈-Alkyl-Gruppen, vorzugsweise aus C₂₋₆-Alkyl- Gruppen, besonders bevorzugt aus C₃₋₅-Alkyl-Gruppen und gemeinsam mit den O-Atomen, an die sie gebunden sind, einen fünf- oder sechsgliedrigen Ring bilden können.

11. Mittel zum Bekämpfen unerwünschter Pilze, Pflanzen, Mikroorganismen und/oder Bakterien, umfassend wenigstens ein Hetarylamidin gemäß einem der Ansprüche 1 bis 9.

12. Verwendung eines Hetarylamidins gemäß irgendeinem der Ansprüche 1 bis 4 oder Mischungen dieser zum Bekämpfen unerwünschter Pilze, Pflanzen, Mikroorganismen und/oder Bakterien.

13. Verfahren zum Bekämpfen unerwünschter Pilze, Pflanzen, Mikroorganismen und/oder Bakterien, **dadurch gekennzeichnet, dass** ein Hetarylamidin gemäß einem der Ansprüche 1 bis 9 auf die Pilze, Pflanzen, Mikroorganismen, Bakterien und/oder in deren Lebensraum ausgebracht werden.

14. Saatgut, welches mit wenigstens einem Hetarylamidin gemäß einem der Ansprüche 1 bis 9 behandelt ist.
